# EUROPEAN PATENT SPECIFICATION

(11) **EP 1 723 241 B1**
(45) Date of publication and mention of the grant of the patent: **11.07.2012**
(21) Application number: 05715574.9
(22) Date of filing: 26.02.2005
(51) Int. Cl.: C12N 15/82, C12N 5/10, A01H 5/00

(54) **TRANSGENIC EXPRESSION CONSTRUCTS FOR VEGETATIVE PLANT TISSUE SPECIFIC EXPRESSION OF NUCLEIC ACIDS**
TRANSGENE EXPRESSIONSKONSTRUKTE ZUR VEGETATIVEN PFLANZENGEWEBE-SPEZIFISCHEN EXPRESSION VON NUKLEINSÄUREN
PRODUITS DE SYNTHESE D'EXPRESSION TRANSGENIQUE POUR EXPRESSION D'ACIDES NUCLEIQUES SPECIFIQUE D'UN TISSU DE PLANTE VEGETATIVE

(30) Priority: 01.03.2004 US 548911 P; 27.07.2004 US 591452 P
(43) Date of publication of application: 22.11.2006
(73) Proprietor: BASF Plant Science GmbH, 67056 Ludwigshafen (DE)
(72) Inventor: SONG, Hee-Sook, Raleigh, NC 27606 (US); ROCHE, Christina, E., Youngsville, NC 27596 (US); MORRA, Marc, Raleigh, NC 27607 (US); DAMMANN, Christian, Durham, NC 27705 (US); JENSEN, Timothy, C., Cary, NC 27513 (US); DOBSON, Alleson, Cary, NC 27511 (US)
(74) Representative: Popp, Andreas
(86) International application number: PCT/EP2005/002052
(87) International publication number: WO 2005/085450

(56) References cited:
- EP-A- 1 375 668
- AHN JI HOON ET AL: "A novel extensin gene encoding a hydroxyproline-rich glycoprotein requires sucrose for its wound-inducible expression in transgenic plants" PLANT CELL, vol. 8, no. 9, 1996, pages 1477-1490, XP002340753 ISSN: 1040-4651 -& DATABASE EMBL [Online] 27 January 1996 (1996-01-27), "Glycine max extensin (SbHRGP3) gene, complete cds." XP002340759 retrieved from EBI accession no. EM_PRO:GM44838 Database accession no. GM44838 cited in the application
- HERMANN S R ET AL: "The banana actin 1 promoter drives near-constitutive transgene expression in vegetative tissues of banana (Musa spp.)" PLANT CELL REPORTS, vol. 20, no. 6, September 2001 (2001-09), pages 525-530, XP002340754 ISSN: 0721-7714
- DATABASE EMBL [Online] 29 October 1997 (1997-10-29), "Pisum sativum ptxA gene" XP002340760 retrieved from EBI accession no. EM_PRO:PSPTXAG Database accession no. PSPTXAG cited in the application
- AHN JI HOON ET AL: "Expression of a soybean hydroxyproline-rich glycoprotein gene is correlated with maturation of roots" PLANT PHYSIOLOGY (ROCKVILLE), vol. 116, no. 2, February 1998 (1998-02), pages 671-679, XP002340755 ISSN: 0032-0889 cited in the application
- SADANANDOM ARIVANANTHAN ET AL: "Identification of a peptide methionine sulphoxide reductase gene in a oleosin promoter from Brassica napus" PLANT JOURNAL, vol. 10, no. 2, 1996, pages 235-242, XP002340756 ISSN: 0960-7412 cited in the application
- ITOH H. ET AL: "Cloning and functional analysis of two gibberelin 3beta-hydroxylase genes that are differently eypressed during the growth of rice", PNAS, vol. 98, no. 15 , pages 8909-8914,

### SEQUENCE LISTING

<110> BASF Plant Science GmbH
<120> Transgenic expression constructs for vegetative plant tissue specific expression of nucleic acids
   <130> PF55368 PCT / AE20040055
   <160> 19
   <170> PatentIn version 3.3
<210> 1
   <211> 863
   <212> DNA
   <213> Pisum sativum
<220>
   <221> promoter
   <222> (1)..(863)
   <223> promoter region of ptxA gene including 5'-untranslated region
<226>
   <221> misc_feature
   <222> (300)..(583)
   <223> potential core region of the promoter comprising clusters of promoter elements
<220>
   <221> TATA_signal
   <222> (549)..(554)
<220>
   <221> 5'UTR
   <222> (584)..(863)
<400> 1
   gcaatttttt gtgaagctga gggaggattg gattttacac ctattcaaaa gtcattcaaa     60
   gtttgtccct ccattcaagg atgaatgtag atttttcaag catcaaacac aagaatcact    120
   agcataacat gctttgaaac ccacacactt aaattaatgt taggaatatc aaatccaata    180
   taaaatcata gttgtcaatt acatactcaa tcaagtccct ttcttttacc caataaacat    240
   caacatattg cttcttccat taagcatata aacatcaaag tctaaaacta gcaaaatgtt    300
   gtttttagga tgacacattt catacatagt ttaaaagata cttgattcga ttacaaaaag    360
   aaattaccaa tagtttagca caaagtctaa agcataatta aagcatcaca tgtgcagatt    420
   tatgaaaaaa agattaagat tgcccctttc atcacgggtc gaataatagc actacttgtc    480
   actacatgtt aaaaaaatgt cctctagtac atcaaacttt ttccattgat tccccttatc   540
   catgaaaaaa ataaacaaat tcttaagaca caaaaaaatg gccccacatc cttttttctg   600
   gcctagtttg tttgaattca ttctaactct tgaatatgta acgaggccca ctaaaaatca    660
   atcaatgatt taacataaaa aatgaatagt ttaattccaa tttgctgcaa catggtccgt    720
   gaatatgact cacgagaaag atatatcaaa atatcaaaat ttcatagttt ttttcaccat    780
   ataaacctca tcactcattc tattttttta agtgcaaagc ttcatagtag tgagcacaca     840
   cattacacta aaatcttcga aac                                            863
<210> 2
   <211> 1380
   <212> DNA
   <213> Glycine max
<220>
   <221> promoter
   <222> (1)..(1380)
   <223> promoter region of SbHRGP3 gene including 5' untranslated region
<220>
   <221> misc_feature
   <222> (800)..(1179)
   <223> potential core region of the promoter comprising clusters of promoter elements
<220>
   <221> TATA_signal
   <222> (1147)..(1152)
<220>
   <221> 5'UTR
   <222> (1180)..(1380)
   <223> potential 5' UTR
<400> 2
   tagaaagctt ttcaacaatc atgcccatgt caagtgtaaa acaggtttac ctctcttaaa     60
   taaccgtatt aaaatgctga atgatgtata tatgtgggtt caaattacat aatttgtaag    120
   tatgttacac attgtataaa tatgttttag agaaaaatgt aaacttatat gtctaaagtt    180
   ataaaagaaa catgtccaac acatttcagt taagatttaa atagtataaa ttaaaaatta    240
   tcgatgatga caaaaaattg taaatataat tcattttaaa aaaagttaag aaattgaaaa    300
   aggaaatatc gagaaaaaaa tatgtcgatt atatatatgt gtgagctgag tgaatatata    360
   tgtatatttt atttttgact gaatatatgt gtgtatagac aataatgcgc agaatgccga    420
   tcgatgaatt gtttactgca tttccaaata tgtgtgcata agcgttccac atgtcaccca    480
   tgttgtaatt agtttcttcc ctggatgaat tactaagaaa cagattgatt gatagtacta    540
   tattaaatta tgtagcttta catgtcagga aaatgtagtt gcagtattat gtaatgtaat    600
   taataggaag tcacagacaa tttgaagaca atttctttag cttacctatc tcatgccaca    660
   attatgtact tacgacagta aaatgtttaa aagcaaaaaa aagaaagaag aagaagaagt    720
   aataaatgga attatataga atgtactctt tgtcttcatc tgccctataa ttcctgcagc    780
   agccaaagca taatagcatg caatatgcac atattcgttt taggctttta gcctccacga    840
   tctgttaatg gaaagtgaaa agtaagagat atgaagttca ttatggcagc catggtccca    900
   gggaagcact agaagatatg aaatgacata aaaggtcacc atgcataatg ctttaaatgc    960
   ttgctataga atcaaaaaat gaagagatgt gacaaattgt tacatctaat acgcaataat   1020
   ttgacaaaga cgactatgcg tttatatatt tattttaatt agttggcgtc tcttattata   1080
   aagaaaataa gggcagtgtc aacatttcca ggcaactagt tagttatttt attttcttgt   1140
   ttataattat ttccatatag ctagctgtct ctatctaatc caaatccgct ttccacaacc   1200
   aacttggtcg cattggtcca aaaaactcaa tatcaatatt ttcgaaatag ttttagcatt   1260
   gtttaggaag agaattgtaa gagataaaat ctaagtactc cacctaccaa gataaaatag   1320
   ttggataaat gggtaaaaaa agttgtataa agggcaacac tacctctcct aatggcagta   1380
<210> 3
   <211> 26
   <212> DNA
   <213> Artificial
<220>
   <223> Oligonucleotide primer ptxA5'
<400> 3
   ggcgcgcccg caattttttg tgaagc 26
<210> 4
   <211> 25
   <212> DNA
   <213> Artificial
<220>
   <223> Oligonucleotide.primer ptxA3'
<400> 4
   tctagataag tttcgaagat tttag 25
<210> 5
   <211> 29
   <212> DNA
   <213> Artificial
<220>
   <223> Oligonucleotide primer SbHRGP3-5'
<400> 5
   tctagataga agcttttcaa caatcatgc 29
<210> 6
   <211> 24
   <212> DNA
   <213> Artificial
<220>
   <223> Oligonucleotide primer SbHRGP3-3'
<400> 6
   agatcttact gccattagga gagg 24
<210> 7
   <211> 1381
   <212> DNA
   <213> Glycine max
<220>
   <221> promoter
   <222> (1)..(1368)
<220>
   <221> misc_feature
   <222> (801)..(1178)
   <223> potential core region of promoter
<220>
   <221> TATA_signal
   <222> (1146)..(1151)
<220>
   <221> 5'UTR
   <222> (1369)..(1381)
<400> 7
   aagcttttca acaatcatgc ccatgtcaag tgtaaaacag gtttacctct cttaaataac     60
   cgtattaaaa tgctgaatga tgtatatatg tgggttcaaa ttacataatt tgtaagtatg    120
   ttacacattg tataaatatg ttttagagaa aaatgtaaac ttatatgtct aaagttataa    180
   aagaaacatg tccaacacat ttcagttaag atttaaatag tataattaaa aattatcgat    240
   gatgacaaaa aattgtaaat ataattcatt ttaaaaaaag ttaagaaatt gaaaaaggaa    300
   atatcgagaa aaaaatatgt cgattatata tatgtgtgag ctgagtgaat atatatgtat    360
   attttatttt tgactgaata tatgtgtgta tagacaataa tgcgcagaat gccgatcgat    420
   gaattgttta ctgcatttcc aaatatgtgt gcataagcgt tccacatgtc acccatgttg    480
   taattagttt cttccctgga tgaattacta agaaacagat tgattgatag tactatatta    540
   aattatgtag ctttacatgt caggaaaatg tagttgcagt attatgtaat gtaattaata    600
   ggaagtcaca gacaatttga agacaatttc tttagcttac ctatctcatg ccacaattat    660
   gtacttacga cagtaaaatg tttaaaagca aaagcaaaaa aaagaaagaa gaagaagaag    720
   taataaatgg aattatatag aatgtactct ttgtcttcat ctgccctata attcctgcag    780
   cagccaaagc ataatagcat gcaatatgca catattcgtt ttaggctttt agctccacga    840
   tctgttaatg gaaagtgaaa agtaagagat atgaagttca ttatggcagc catggtccca    900
   gggaagcact agaagatatg aaatgactaa aaggtcacca tgcataatgc tttaaatgct    960
   tgctatagaa tcaaaaaatg aagagatgtg acaaattgtt acatctaata cgcaataatt   1020
   tgacaaagac gactatgcgt ttatatattt attttaatta gttggcgtct cttattataa   1080
   agaaaataag ggcagtgtca acatttccag gcaactagtt agttatttta ttttcttgtt   1140
   tataattatt tccatatagc tagctgtctc tatctaatcc aaatccgcgt tccacaacca   1200
   acttggtcca aaaaactcaa tatcaatatt ttcaaaatag ttttagcatt gtttaggaag   1260
   agaattgtaa gagataaaat ctaagtactc cacctaccaa gataaaatag ttggataaat   1320
   gggtaaaaaa gttgtataaa gggcaacact acctctccta atggcagtac caaaacccaa   1380
   g                                                                   1381
<210> 8
   <211> 1388
   <212> DNA
   <213> Glycine max
<220>
   <221> promoter
   <222> (1)..(1175)
   <223> potential promoter region
<220>
   <221> misc_feature
   <222> (796)..(1175)
   <223> potential core region of promoter
<220>
   <221> TATA_signal
   <222> (1143)..(1148)
<220>
   <221> 5'UTR
   <222> (1176)..(1388)
<400> 8
   aagcttttca acaatcatgc ccatgtcaag tgtaaaacag gtttacctct cttaaataac     60
   cgtattaaaa tgctgaatga tgtatatatg tgggttcaaa ttacataatt tgtaagtatg    120
   ttacacattg tataaatatg ttttagagaa aaatgtaaac ttatatgtct aaagttataa    180
   aagaaacatg tccaacacat ttcagttaag atttaaatag tataaattaa aaattatcga    240
   tgatgacaaa aaattgtaaa tataattcat tttaaaaaaa gttaagaaat tgaaaaagga    300
   aatatcgaga aaaaaatatg tcgattatat atatgtgtga gctgagtgaa tatatatgta    360
   tattttattt ttgactgaat atatgtgtgt atagacaata atgcgcagaa tgccgatcga    420
   tgaattgttt actgcatttc caaatatgtg tgcataagcg ttccacatgt cacccatgtt    480
   gtaattagtt tcttccctgg atgaattact aagaaacaga ttgattgata gtactatatt    540
   aaattatgta gctttacatg tcaggaaaat gtagttgcag tattatgtaa tgtaattaat    600
   aggaagtcac agacaatttg aagacaattt ctttagctta cctatctcat gccacaatta    660
   tgtacttacg acagtaaaat gtttaaaagc aaaaaaaaga aagaagaaga agaagtaata    720
   aatggaatta tatagaatgt actctttgtc ttcatctgcc ctataattcc tgcagcagcc    780
   aaagcataat agcatgcaat atgcacatat tcgttttagg cttttagcct ccacgatctg    840
   ttaatggaaa gtgaaaagta agagatatga agttcattat ggcagccatg gtcccaggga    900
   agcactagaa gatatgaaat gacataaaag gtcaccatgc ataatgcttt aaatgcttgc    960
   tatagaatca aaaaatgaag agatgtgaca aattgttaca tctaatacgc aataatttga   1020
   caaagacgac tatgcgttta tatatttatt ttaattagtt ggcgtctctt attataaaga   1080
   aaataagggc agtgtcaaca tttccaggca actagttagt tattttattt tcttgtttat   1140
   aattatttcc atatagctag ctgtctctat ctaatccaaa tccgctttcc acaaccaact   1200
   tggtcgcatt ggtccaaaaa actcaatatc aatattttcg aaatagtttt agcattgttt   1260
   aggaagagaa ttgtaagaga taaaatctaa gtactccacc taccaagata aaatagttgg   1320
   ataaatgggt aaaaaaagtt gtataaaggg caacactacc tctcctaatg gcagtaccaa   1380
   aacccaag                                                             1388
<210> 9
   <211> 1373
   <212> DNA
   <213> Glycine max
<220>
   <221> promoter
   <222> (1)..(1172)
   <223> potential promoter region
<220>
   <221> misc_feature
   <222> (793)..(1172)
   <223> potential core region of promoter
<220>
   <221> TATA_signal
   <222> (1140)..(1145)
<220>
   <221> 5'UTR
   <222> (1173)..(1373)
<400> 9
   cttttcaaca atcatgccca tgtcaagtgt aaaacaggtt tacctctctt aaataaccgt 60
   attaaaatgc tgaatgatgt atatatgtgg gttcaaatta cataatttgt aagtatgtta 120
   cacattgtat aaatatgttt tagagaaaaa tgtaaactta tatgtctaaa gttataaaag 180
   aaacatgtcc aacacatttc agttaagatt taaatagtat aaattaaaaa ttatcgatga 240
   tgacaaaaaa ttgtaaatat aattcatttt aaaaaaagtt aagaaattga aaaaggaaat 300
   atcgagaaaa aaatatgtcg attatatata tgtgtgagct gagtgaatat atatgtatat 360
   tttatttttg actgaatata tgtgtgtata gacaataatg cgcagaatgc cgatcgatga 420
   attgtttact gcatttccaa atatgtgtgc ataagcgttc cacatgtcac ccatgttgta 480
   attagtttct tccctggatg aattactaag aaacagattg attgatagta ctatattaaa 540
   ttatgtagct ttacatgtca ggaaaatgta gttgcagtat tatgtaatgt aattaatagg 600
   aagtcacaga caatttgaag acaatttctt tagcttacct atctcatgcc acaattatgt 660
   acttacgaca gtaaaatgtt taaaagcaaa aaaaagaaag aagaagaaga agtaataaat 720
   ggaattatat agaatgtact ctttgtcttc atctgcccta taattcctgc agcagccaaa 780
   gcataatagc atgcaatatg cacatattcg ttttaggctt ttagcctcca cgatctgtta 840
   atggaaagtg aaaagtaaga gatatgaagt tcattatggc agccatggtc ccagggaagc 900
   actagaagat atgaaatgac ataaaaggtc accatgcata atgctttaaa tgcttgctat 960
   agaatcaaaa aatgaagaga tgtgacaaat tgttacatct aatacgcaat aatttgacaa 1020
   agacgactat gcgtttatat atttatttta attagttggc gtctcttatt ataaagaaaa 1080
   taagggcagt gtcaacattt ccaggcaact agttagttat tttattttct tgtttataat 1140
   tatttccata tagctagctg tctctatcta atccaaatcc gctttccaca accaacttgg 1200
   tcgcattggt ccaaaaaact caatatcaat attttcgaaa tagttttagc attgtttagg 1260
   aagagaattg taagagataa aatctaagta ctccacctac caagataaaa tagttggata 1320
   aatgggtaaa aaaagttgta taaagggcaa cactacctct cctaatggca gta 1373
<210> 10
   <211> 1924
   <212> DNA
   <213> Artificial
<220>
   <223> Artificial construct of ptxA promoter and ubiquitin intron
<220>
   <221> promoter
   <222> (1)..(583)
   <223> potential promoter region
<220>
   <221> misc_feature
   <222> (300)..(583)
   <223> potential core region of promoter
<220>
   <221> TATA_signal
   <222> (549)..(554)
<220>
   <221> 5'UTR
   <222> (584)..(828)
<220>
   <221> misc_feature
   <222> (829)..(874)
   <223> multiple cloning site
<220>
   <221> Intron
   <222> (875)..(1924)
   <223> Zea mais ubiquitin intron
<400> 10
   gcaatttttt gtgaagctga gggaggattg gattttacac ctattcaaaa gtcattcaaa     60
   gtttgtccct ccattcaagg atgaatgtag atttttcaag catcaaacac aagaatcact    120
   agcataacat gctttgaaac ccacacactt aaattaatgt taggaatatc aaatccaata    180
   taaaatcata gttgtcaatt acatactcaa tcaagtccct ttcttttacc caataaacat    240
   caacatattg cttcttccat taagcatata aacatcaaag tctaaaacta gcaaaatgtt    300
   gtttttagga tgacacattt catacatagt ttaaaagata cttgattcga ttacaaaaag    360
   aaattaccaa tagtttagca caaagtctaa agcataatta aagcatcaca tgtgcagatt    420
   tatgaaaaaa agattaagat tgcccctttc atcacgggtc gaataatagc actacttgtc    480
   actacatgtt aaaaaaatgt cctctagtac atcaaacttt ttccattgat tccccttatc    540
   catgaaaaaa ataaacaaat tcttaagaca caaaaaaatg gccccacatc cttttttctg    600
   gcctagtttg tttgaattca ttctaactct tgaatatgta acgaggccca ctaaaaatca    660
   atcaatgatt taacataaaa aatgaatagt ttaattccaa tttgctgcaa catggtccgt    720
   gaatatgact cacgagaaag atatatcaaa atatcaaaat ttcatagttt ttttcaccat    780
   ataaacctca tcactcattc tattttttta agtgcaaagc ttcatagtta attaaggcgc    840
   gccaagcttg catgcctgca ggtcgactct agaggatctc ccccaaatcc acccgtcggc    900
   acctccgctt caaggtacgc cgctcgtcct cccccccccc ccctctctac cttctctaga    960
   tcggcgttcc ggtccatggt tagggcccgg tagttctact tctgttcatg tttgtgttag   1020
   atccgtgttt gtgttagatc cgtgctgcta gcgttcgtac acggatgcga cctgtacgtc   1080
   agacacgttc tgattgctaa cttgccagtg tttctctttg gggaatcctg ggatggctct   1140
   agccgttccg cagacgggat cgatttcatg attttttttg tttcgttgca tagggtttgg   1200
   tttgcccttt tcctttattt caatatatgc cgtgcacttg tttgtcgggt catcttttca   1260
   tgcttttttt tgtcttggtt gtgatgatgt ggtctggttg ggcggtcgtt ctagatcgga   1320
   gtagaattct gtttcaaact acctggtgga tttattaatt ttggatctgt atgtgtgtgc   1380
   catacatatt catagttacg aattgaagat gatggatgga aatatcgatc taggataggt   1440
   atacatgttg atgcgggttt tactgatgca tatacagaga tgctttttgt tcgcttggtt   1500
   gtgatgatgt ggtgtggttg ggcggtcgtt cattcgttct agatcggagt agaatactgt   1560
   ttcaaactac ctggtgtatt tattaatttt ggaactgtat gtgtgtgtca tacatcttca   1620
   tagttacgag tttaagatgg atggaaatat cgatctagga taggtataca tgttgatgtg   1680
   ggttttactg atgcatatac atgatggcat atgcagcatc tattcatatg ctctaacctt   1740
   gagtacctat ctattataat aaacaagtat gttttataat tattttgatc ttgatatact   1800
   tggatgatgg catatgcagc agctatatgt ggattttttt agccctgcct tcatacgcta   1860
   tttatttgct tggtactgtt tcttttgtcg atgctcaccc tgttgtttgg tgttacttct   1920
   gcag                                                                1924
<210> 11
   <211> 23
   <212> DNA
   <213> Artificial
<220>
   <223> oligonucleotide primer ptxA3'-2
<400> 11
   tctagataaa ctatgaagct ttg 23
<210> 12
   <211> 22
   <212> DNA
   <213> Artificial
<220>
   <223> oligonucleotide primer
<400> 12
   ccgcttcgaa accaatgcct aa 22
<210> 13
   <211> 24
   <212> DNA
   <213> Artificial
<220>
   <223> oligonucleotide primer
<400> 13
   tggtcgtcat gaagatgcgg actt 24
<210> 14
   <211> 20
   <212> DNA
   <213> Artificial
<220>
   <223> Oligonucleotide primer ptxaF1
<400> 14
   gggccaagga catagtagaa 20
<210> 15
   <211> 20
   <212> DNA
   <213> Artificial
<220>
   <223> Oligonucleotide primer ptxaR1
<400> 15
   tgaagttaca aacgctgaca 20
<210> 16
   <211> 20
   <212> DNA
   <213> Artificial
<220>
   <223> Oligonucleotide primer ptxaR1
<400> 16
   agagcatcac acgcaatcaa 20
<210> 17
   <211> 20
   <212 > DNA
   <213> Artificial
<220>
   <223> Oligonucleotide primer SbHRGP3-F1
<400> 17
   catgtgcgcg tacttttgta 20
<210> 18
   <211> 20
   <212> DNA
   <213> Artificial
<220>
   <223> Oligonucleotide primer SbHRGP3-F1
<400> 18
   atgaagaata taagccaata 20
<210> 19
   <211> 20
   <212> DNA
   <213> Artificial
<220>
   <223> Oligonucleotide primer SbHRGP3-R1
<400> 19
   agtgccatac aactgtctaa 20

## Description

### FIELD OF THE INVENTION

The invention relates to transgenic expression constructs and vectors comprising plant promoters with a non-seed tissue, preferably vegetative plant tissue specific expression profile, and the use of these transgenic expression constructs or vectors for the transgenic expression of nucleic acid sequences in plants. The promoters of the invention demonstrate strong expression levels in most vegetative organs and tissues at different developmental stages (including but not limited to leafs, stem and roots), but low levels of expression in flowers (including the reproductive organs) and very low expression levels in seeds. The invention furthermore relates to transgenic plants and plant cells transformed with these expression constructs or vectors, to cultures, parts or propagation material derived therefrom, and to the use of same for the preparation of foodstuffs, animal feeds, seed, pharmaceuticals or fine chemicals, to improve plant biomass, yield, or provide desirable phenotypes. Strong expression controlled by these promoters in young seedlings and cultured cells provide an appropriate tool to express selectable marker genes for plant transformation.

### BACKGROUND OF THE INVENTION

The aim of plant biotechnology is the generation of plants with advantageous novel properties, such as pest and disease resistance, resistance to environmental stress (*e.g*., water-logging, drought, heat, cold, light-intensity, day-length, chemicals, *etc*.), improved qualities (*e.g*., high yield of fruit, extended shelf-life, uniform fruit shape and color, higher sugar content, higher vitamins C and A content, lower acidity, *etc*.), or for the production of certain chemicals or pharmaceuticals (Dunwell 2000) . Furthermore resistance against abiotic stress (drought, salt) and/or biotic stress (insects, fungal, nematode infections) can be increased. Crop yield enhancement and yield stability can be achieved by developing genetically engineered plants with desired phenotypes (Alia 1999; Sakamoto 1998). Appropriate promoters play an important role in regulating genes of interest to obtain the desired phenotypes.

A basic prerequisite for the recombinant expression of specific genes in plants is the provision of plant-specific promoters. A variety of plant promoters are known. Known examples are constitutive promoters such as the nopaline synthase promoter from *Agrobacterium*, the promoter of the cauliflower mosaic virus (CaMV) 35S transcript (Odell 1985), the OCS (octopine synthase) promoter from *Agrobacterium*, the ubiquitin promoter (Callis 1990), the promoters of the vacuolar ATPase subunits or the promoter of proline-rich protein from wheat (WO 91/13991). The disadvantage of these promotors is that they are constitutively active in virtually all of the plant's tissues. A targeted expression of genes in specific plant parts or at specific developmental stages is not possible with these promoters.

Promoters with specificities for the anthers, ovaries, flowers, leaves, stems, roots and seeds have been described. The stringency of the specificity and the expression activity of these promoters differ greatly. Promoters which must be mentioned are those which ensure a leaf-specific expression, such as the potato cytosolic FBPase promoter (WO 97/05900), the Rubisco (ribulose-1,5-bisphosphate carboxylase) SSU (small subunit) promoter, or the potato ST-LSI promoter (Stockhaus 1989) .

Examples of other promoters are promoters with specificity for tubers, storage roots or roots, such as, for example, the class I patatin promoter (B33), the potato cathepsin D inhibitor promoter, the starch synthase (GBSS1) promoter or the sporamin promoter, fruit-specific promoters such as, for example, the tomato fruit-specific promoter (EP-A1 409 625), fruit-maturation-specific promoters such as, for example, the tomato fruit-maturation specific promoter (WO 94/21794), flower-specific promoters such as, for example, the phytoene synthase promoter (WO 92116635) or the promoter of the P1-rr gene (WO 98/22593).

A promoter, which is regulated in a development-dependent fashion is described (Baerson 1993).

Promoters are described with tissue specificity for the mesophyll and the palisade cells in leaves (Broglie 1984), the dividing shoot and the root meristem (Atanassova 1992), pollen (Guerrero 1990), seed endosperm (Stalberg 1993), root epidermis (Suzuki 1993), and for the root meristem, root vascular tissue and root knots (Bogusz 1990).

Other known promoters are those, which govern expression in seeds and plant embryos. Examples of seed-specific promoters are the phaseolin promoter (US 5,504,200, Bustos 1989), the promoter of 2S albumin gene (Joseffson 1987), the legumin promoter (Shirsat 1989, the USP (unknown seed protein) promoter (Bäumlein 1991), the promoter of the napin gene (Stalberg 1996), the promoter of the sucrose binding protein (WO 00/26388) or the LeB4 promoter (Bäumlein 1991). These promoters govern a seed-specific expression of storage proteins.

Described is the promoter of the salt-inducible MsPRP2 gene from alfalfa (Bastola 1998; WO 99/53016). This promoter is described to be highly root specific.

Seeds are the most relevant agronomical product, which is heavily used for feed and food purposes. However, expression of transgenes in seeds is in most cases neither necessary nor beneficial. For example, traits like herbicide resistance, resistance against insects, fungi, or nematode, cold or drought resistance do not need to be expressed in seeds, since expression is only required in roots or green tissues. Expression in seeds can have one or more of the following disadvantages:
1. Unnecessary expression of traits in seeds may lead to lower germination rates or at least unnecessary consumption of transcription / translation capacity resulting in yield loss or negatively affecting composition of the seed.
2. Unnecessary expression of traits in seeds may raise higher hurdles in de-regulation proceedings (since a more substantial amount of the transgenic product is comprised in the feed or food materials).
3. Unnecessary expression of traits in seeds may negatively affect consumer acceptance.

Flowers-comprise the plants reproductive organs (carpels and stamens). Expression in these tissues is for some traits also regarded as disadvantageous. For example, expression of the Bt protein (conferring resistance against corn root borer and other plant parasites) under a strong constitutive promoter resulted in expression in pollen and was discussed to have a toxic effect on beneficial pollen transferring insects like the monarch butterflies.

It is, however, an unsolved demand in the plant biotech field to establish reliable expression systems, which express traits only in the vegetative plant tissues but not (or much less) in seeds and flowers (or their reproductive organs). As described above, there are numerous tissue specific promoters known in the art. However, in cases they have no or a low seed and/or flower expression capacity, they are highly specific for other tissues (like *e.g*., leaves or roots), but do not allow for a broad expression profile in all vegetative plant tissues.

It is therefore an objective of the present invention, to provide promoter sequences which demonstrate a constitutive expression activity in all (or substantially all) non-seed tissues, preferably vegetative plant tissues and/or organs, but have only a low (preferably none) expression activity in seeds and preferably also in flowers.

This objective is achieved by the promoter sequences provided within this invention.

A first subject matter of the invention therefore relates to a transgenic expression constructs for predominant expression of a nucleic acid sequence of interest in substantially all vegetative plant tissues and/or organs but where expression is comparatively low or non existing in seeds and flowers comprising a promoter sequence selected from the group consisting of
a) the promoter of the *Pisum sativum* ptxA gene as described by SEQ ID NO. 1
b) a nucleotide sequence which has essentially the same promoter activity as the promoter sequence described by SEQ ID NO:1, and has an identity of at least 80% over a sequence of at least 700 consecutive base pairs to the sequence as described by SEQ ID NO:1.
wherein said promoter sequence is operably linked to a nucleic acid sequence of interest to be transgenically expressed, and wherein said promoter sequence is heterologous with respect to said nucleic acid sequence of interest.

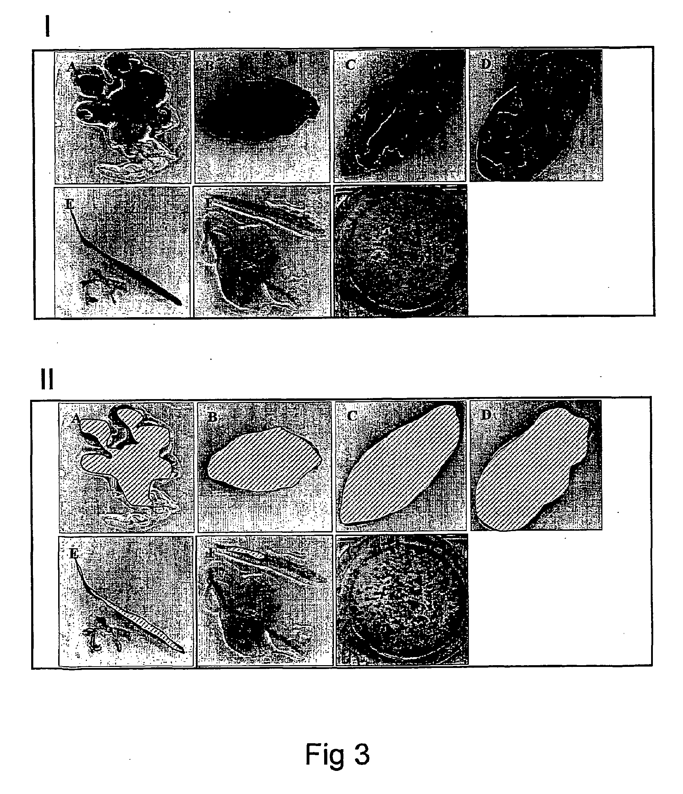

The promoter sequences of the ptxA gene demonstrate highly uniform, homogenous expression activity in virtually all vegetative organs and/or tissues of various species including dicotyledonous and monocotyledonous plants. In seeds, there is no expression activity detectable by GUS staining (see Example 7 and Fig. 3, 4 and 5) and low expression activity detectable by the more sensitive method of RT-PCR (-see Example 16 and Table 2). This is an advantage since very little, if any transgenic protein will be expressed in the seed (which is used for food and feed purpose). For numerous agronomically valuable traits (*e.g*., stress resistance, improved water use, resistance against fungi or insects, *etc*.) no or low expression in seeds is required. Therefore, avoidance of this unnecessary expression may facilitate regulatory approval and/or consumer acceptance.

Furthermore, the promoter activity in the vegetative plant tissues and organs at the vegetative stages is relatively stronger than at the reproductive stages. In consequence the promoter activity is most active in the young vulnerable plantlet, but becomes lower in the mature plant. This is of an additional advantage, especially for genes which confer resistance against biotic or abiotic stress factors (*e.g*., cold, drought, insect damage, *etc*.) since young, developing plants are considered much more vulnerable against said stress factors than mature plants. The promoter activity of the promoters of the invention is especially high in non-differentiated or de-differentiated tissues or cells like, *e.g*., callus culture. This is very useful for utilizing the promoter in combination with selection marker in transformation protocols.

The invention furthermore relates to a method for transgenic predominant expression of a nucleic acid sequence of interest in substantially all vegetative plant tissues and/or organs, but where the expression is comparatively low or non existing in seeds and flowers comprising:
i. introduction of a transgenic expression construct into a plant cell or a plant, said transgenic expression construct comprising a promoter sequence selected from the group consisting of
   a) a nucleotide sequence as described above having essentially the same promoter activity as the promoter sequence described by SEQ ID NO:1,
wherein said promoter sequence or a nucleotide sequence as described above having essentially the same promoter activity as the promoter sequence described by SEQ ID NO:1, is operably linked to a nucleic acid sequence of interest to be transgenically expressed, and wherein said promoter sequence or a nucleotide sequence as described above having essentially the same promoter activity as the promoter sequence described by SEQ ID NO:1, is heterologous with respect to said nucleic acid sequence of interest, under conditions such that said nucleic acid sequence of interest is expressed in said plant cell and/or predominantly expressed in the vegetative plant tissue and/or organs of said transgenic plant.

In a preferred embodiment, the method further comprises ii) identifying or selecting the transgenic plant cell comprising said transgenic expression construct. In another preferred embodiment, the method further comprises iii) regenerating transgenic plant tissue from the transgenic plant cell. In an alternative preferred embodiment, the methods further comprises iv) regenerating a transgenic plant from the transgenic plant cell.

A preferred functional equivalent fragment of the ptxA promoter comprises a sequence from about base pair 300 to about base pair 583 of the sequence described by SEQ ID NO: 1. Another preferred functional equivalent homolog of the ptxA promoter comprises a sequence from about base pair 300 to about base pair 828 of the sequence described by SEQ ID NO: 1.

Also disclosed is a promoter sequence utilized in the inventive transgenic expression constructs or methods of expression, selected from the group of sequences consisting of:
a) the promoter of the *Glycine max* extensin (SbHRGP3) gene as described by SEQ ID NO: 2, or its complement,
b) a functional equivalent fragment of at least 50 consecutive base pairs of the promoter sequence described by SEQ ID NO: 2, or its complement, having essentially the same promoter activity as the promoter sequence described by SEQ ID NO: 2,
c) a functional equivalent homolog of the promoter sequence described by SEQ ID NO: 2 which has essentially the same promoter activity as the promoter sequence described by SEQ ID NO: 2, and has
   i) a homology of at least 60% over a sequence of at least 100 consecutive base pairs to the sequence as described by SEQ ID NO: 2 and/or
   ii) hybridizes under high stringency conditions with a fragment of at least 50 consecutive base pairs of the nucleic acid molecule described by SEQ ID NO: 2.

An equivalent fragment of the SbHRGP3 promoter comprises a sequence from about base pair 800 to about base pair 1179 of the sequence described by SEQ ID NO: 2.

Other preferred functional equivalent homologs of the SbHRGP3 promoter comprise a sequence selected from the group described by SEQ ID NO: 7, 8 and 9.

The transgenic expression construct of the invention may comprise further genetic control sequences linked operably to the nucleic acid sequence of interest to be expressed is to, and/or additional functional elements.

The nucleic acid sequence of interest transgenically expressed from the transgenic expression construct of the invention may results in expression of a protein encoded by said nucleic acid sequence (by transcription and subsequent translation), and/or expression of sense, antisense or double-stranded RNA encoded by said nucleic acid sequence of interest.

In another embodiment, nucleotide sequence encoding the transgenic expression construct of the invention is double-stranded. In yet another embodiment, the nucleotide sequence encoding the transgenic expression construct of the invention is single-stranded.

In yet another alternative embodiment, the transgenic expression construct of the invention is contained in a vector or in a plant cell or a plant. In a preferred embodiment, the plant cell is derived from a dicotyledonous or monocotyledonous plant. In a yet more preferred embodiment, the monocotyledonous plant is selected from the group consisting of sugarcane, maize, sorghum, pineapple, rice, barley, oat, wheat, rye, yam, onion, banana, coconut and date. In a yet more preferred embodiment, the dicotyledonous plant is selected from the group consisting of hop, rapeseed, tobacco, tomato, tagetes (marigold), soybean, pea, common bean, and papaya.

Further embodiments of the invention relate to the use of a transgenic organism of the invention or of cell cultures, parts of transgenic propagation material derived therefrom for the production of foodstuffs, animal feeds, seed, pharmaceuticals or fine chemicals.

Another embodiment of the invention related to a method for production of a foodstuff, animal feed, seed, pharmaceutical or fine chemical employing a transgenic organism of the invention or of cell cultures, parts of transgenic propagation material derived therefrom.

### BRIEF DESCRIPTION OF THE DRAWINGS

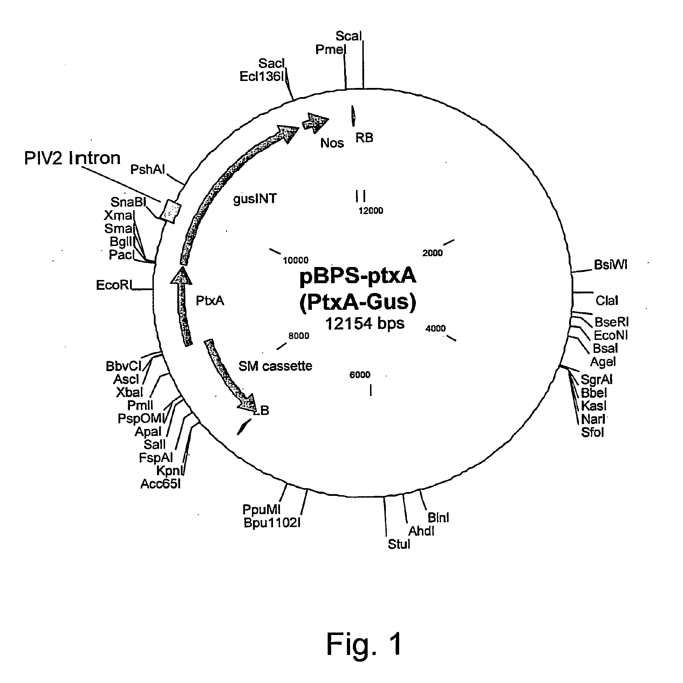

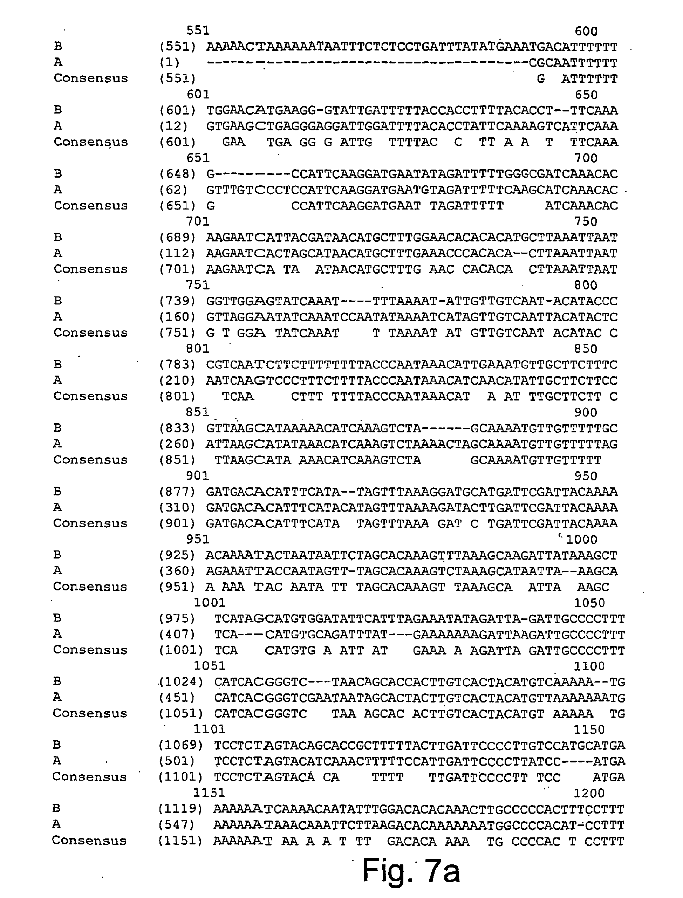

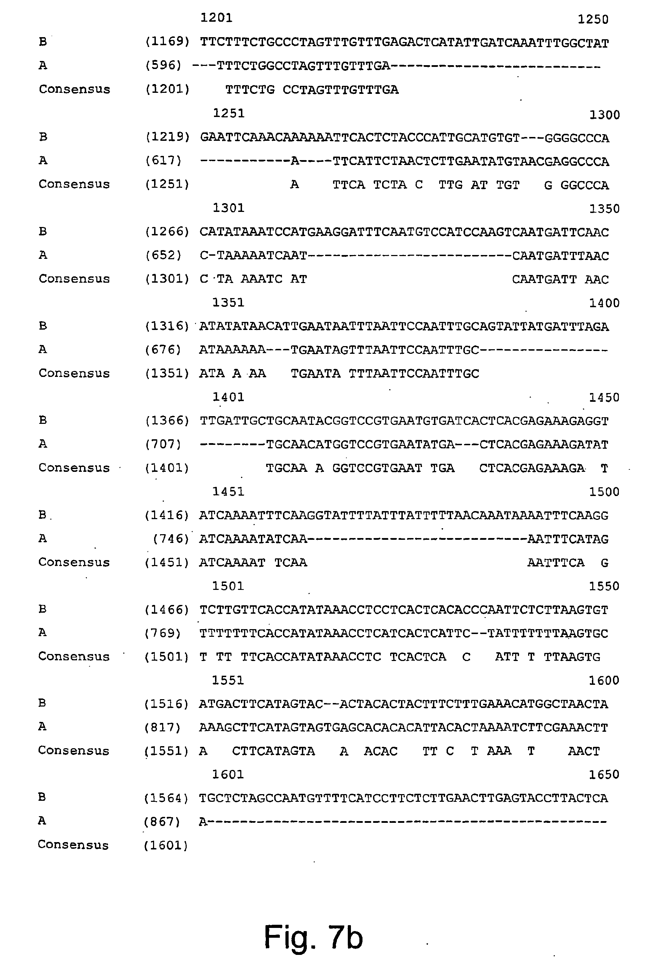

- Fig. 1: Map of ptxA::GUS::NOS chimeric construct (pBPS-ptxA). The plasmid comprises an expression construct containing a ptxA promoter (ptxA) operably linked to a β-glucuronidase gene (gusINT), and 3' untranslated region and termination derived from the nopaline synthase gene (NOS). The "SM" cassette is representing a selectable marker expression cassette.

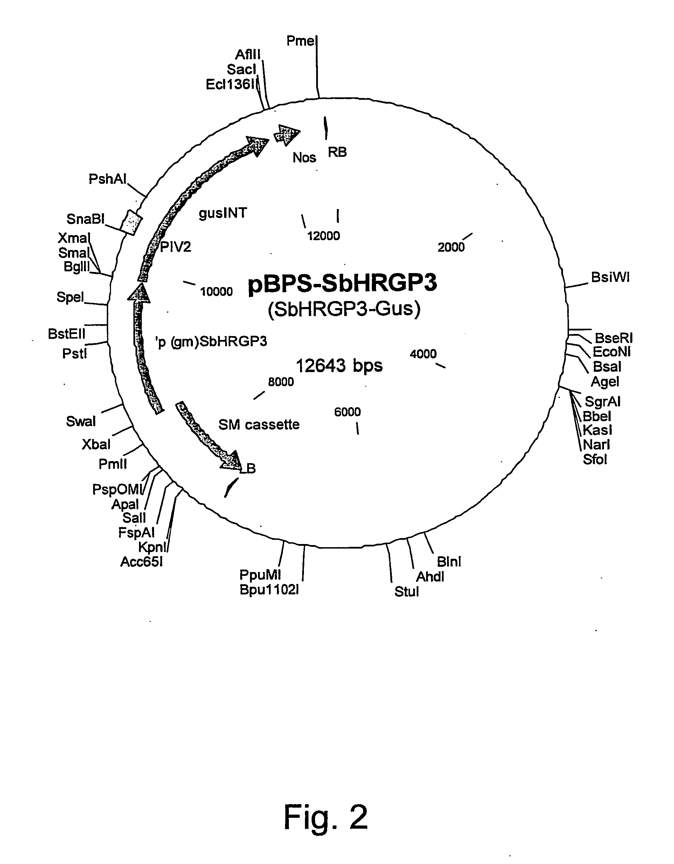

- Fig. 2: Map of SbHRGP3::GUS::NOS chimeric construct (pBPS-SbHRGP3). The plasmid comprises an expression construct containing SbHRGP3 promoter ('p(gm)SbHRGP3) operably linked to a β-glucuronidase gene (gusINT), and 3' untranslated region and termination derived from the nopaline synthase gene (NOS). The "SM" cassette is representing a selectable marker expression cassette.
- Fig. 3: GUS expression controlled by pea ptxA promoter in *Arabidopsis.* The upper panel (I) represents the original photos with the GUS staining, while the lower panel (II) indicates areas distinctly stained blue by overlaid shaded areas.
(A) seedlings (14 Days After Germination),
(B) rosette leaf (25 DAG),
(C) leaf from mature plants (35 DAG),
(D) leaf from old plants (>40 DAG),
(E) flowers and siliques from high expression line,
(F) flowers and siliques from low expression line, and
(G) crushed dried seeds in X-Gluc solution.
Pictures represent reproducible expression patterns from 30 T₁ lines and 10 T₂ lines with low copy.
- Fig. 4: GUS expression controlled by pea ptxA promoter in Canola. The upper panel (I) represents the original photos with the GUS staining, while the lower panel (II) indicates areas distinctly stained blue by overlaid shaded areas.
(A) seedlings (3-4 Days After Germination),
(B) shoot with leaves from young plants (first 2-3 true leaves)
(C) leaf from mature plants (4-6 weeks after germination),
(D) leaf from old plants at late flowering stage,
(E) flower,
(F) style after pollination,
(G) mature seeds
- Fig. 5: GUS expression controlled by SbHRGP3 promoter in *Arabidopsis.* The upper panel (I) represents the original photos with the GUS staining, while the lower panel (II) indicates areas distinctly stained blue by overlaid shaded areas.
(A) seedlings (14 Days After Germination),
(B) rosette leaf (25 DAG),
(C) leaf from old plants (>40 DAG),
(D) flowers and siliques. Only very slight expression could be detected in reproductive organs.
Pictures represent reproducible expression patterns from 30 T₁ lines and 10 T₂ lines with low copy.

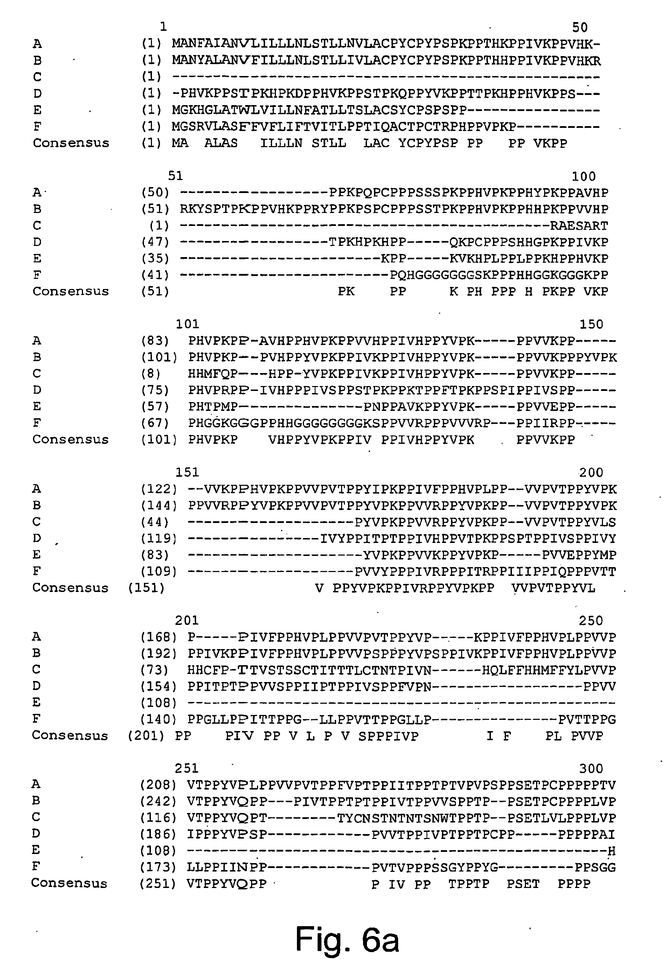

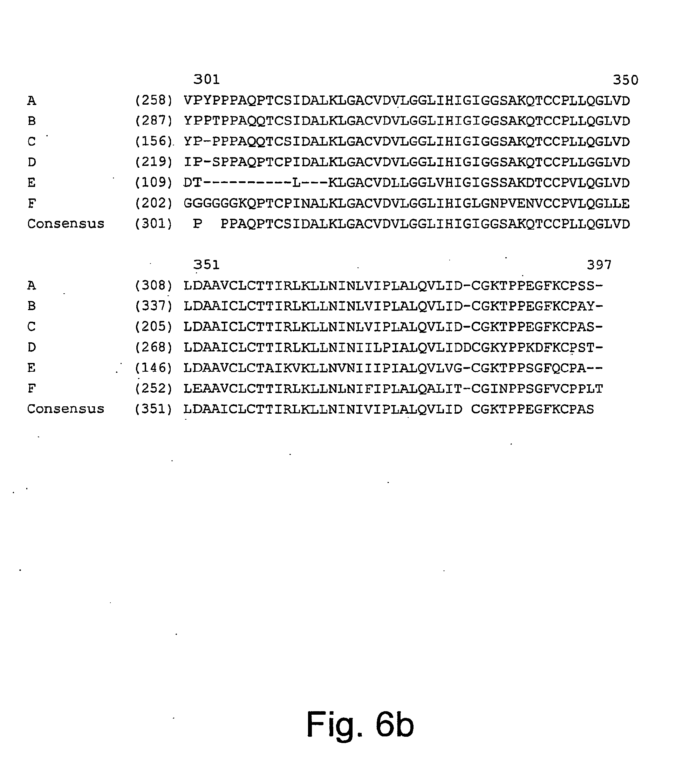

- Fig. 6a+b: Protein alignment of the ptxA protein with the MSPRP2 protein from *Medicago sativa* and other similar proteins.
A: ptxA protein, GenBank Acc.-No.: X67427
B: *Medicago sativa* proline-rich cell wall protein GenBank Acc.-No.: AF028841
C: *Lycopersicum esculentum* proline rich protein GenBank Acc.-No.: X57076
D: *Vitis vinifera proline-rich* protein 1 (PRP1) GenBank Acc.-No.: AY046416
E: *Arabidopsis thaliana* protease inhibitor/seed storage/lipid transfer protein (LTP) GenBank Acc.-No.: NM104929
- Fig. 7a+b:: Alignment of the promoter regions of ptxA gene (A) and the MSPRP2 gene from *Medicago sativa* (B).

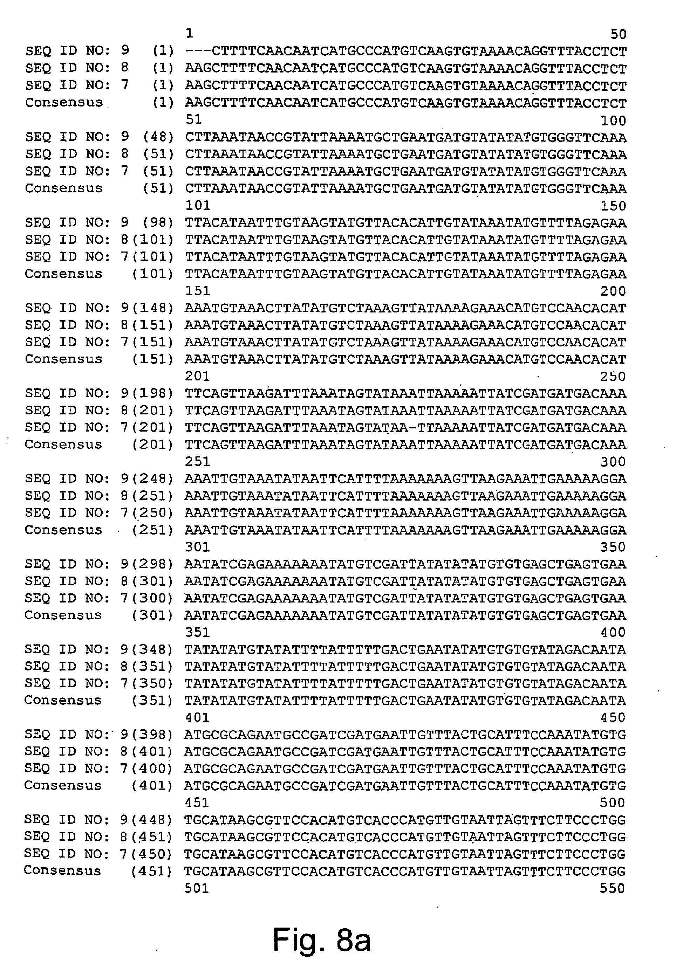

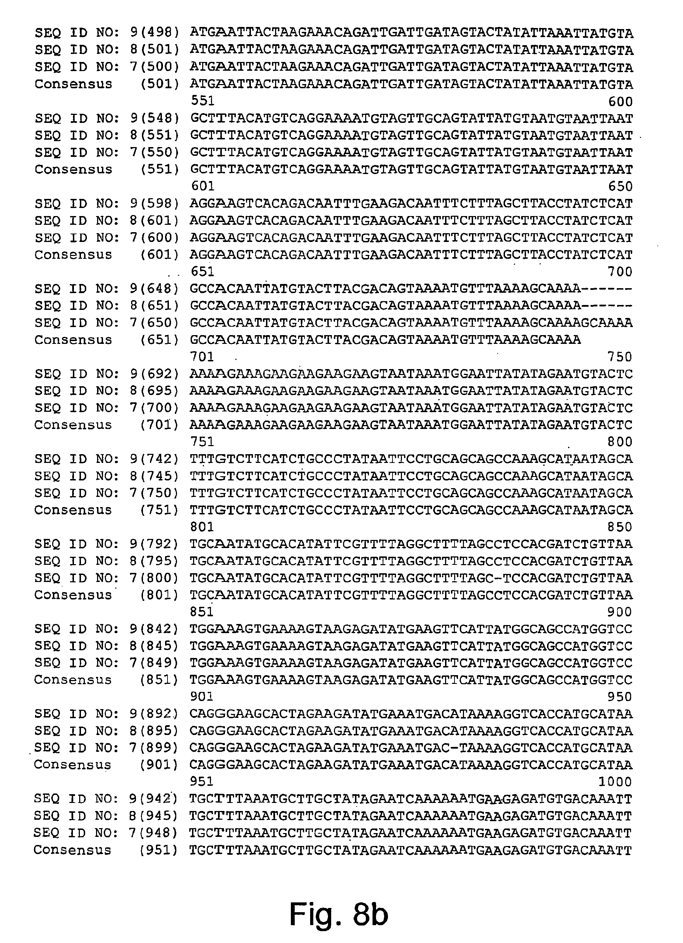

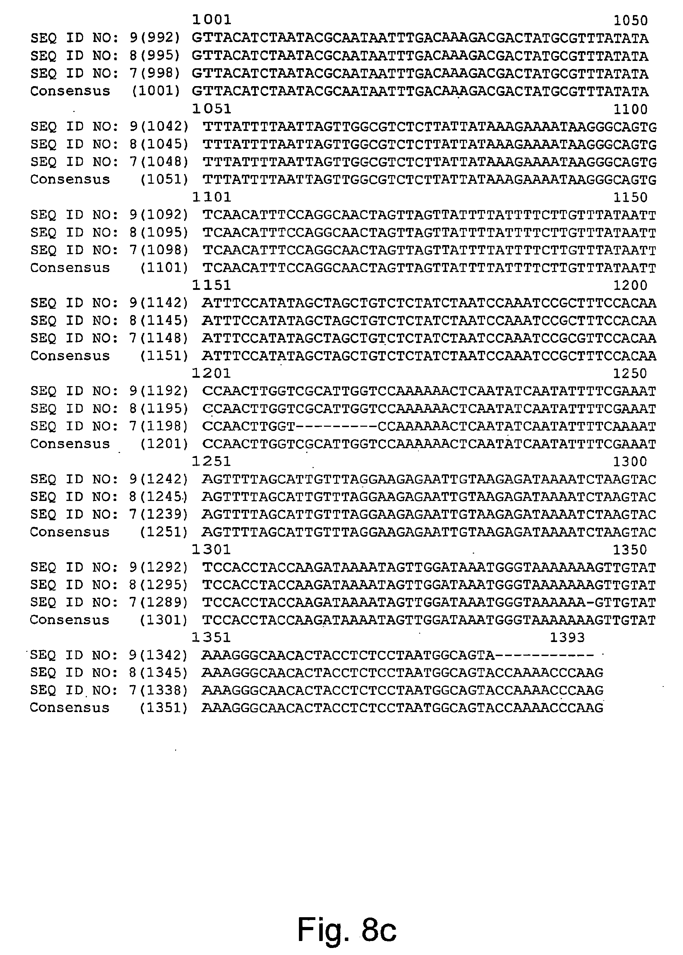

- Fig. 8a-c:: Alignment of the SbHRGP3 promoter variations.

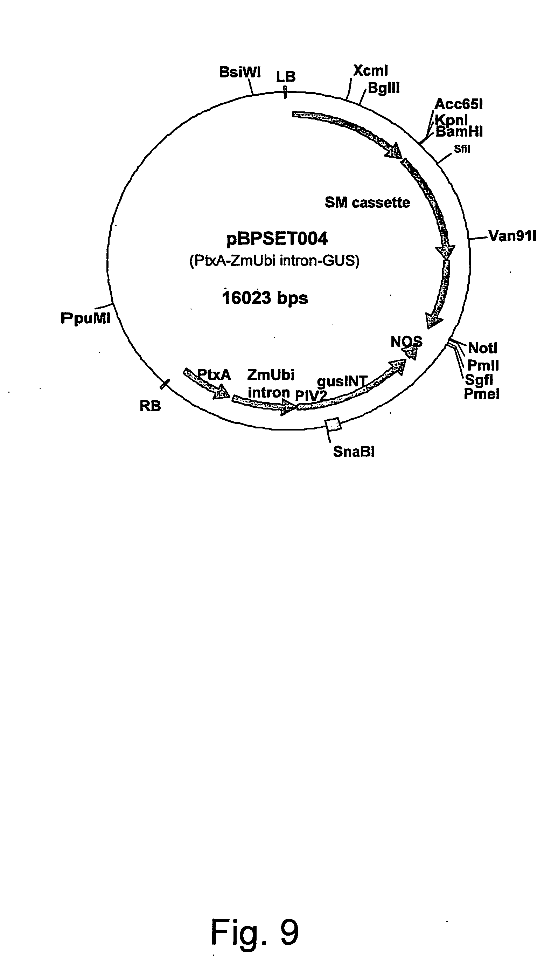

- Fig. 9: Map of ptxA promoter::ZmUbiquitin intron::GUS::NOS chimeric construct (pBPSET004: ptxA-ZmUbi intron-GUS). The plasmid comprises an expression construct containing a ptxA promoter (ptxA) operably linked to maize Ubiquitin intron (ZmUbi intron), β-glucuronidase gene (gusINT), and 3' untranslated region and termination derived from the nopaline synthase gene (NOS). SM cassette stands for a selectable marker cassette.

### GENERAL DEFINITIONS

To facilitate understanding of the invention, a number of terms are defined below. It is to be understood that this invention is not limited to the particular methodology, protocols, cell lines, plant species or genera, constructs, and reagents described as such. It is also to be understood that the terminology used herein is for the purpose of describing particular embodiments only, and is not intended to limit the scope of the present invention which will be limited only by the appended claims. It must be noted that as used herein and in the appended claims, the singular forms "a," "and," and "the" include plural reference unless the context clearly dictates otherwise. Thus, for example, reference to "a vector" is a reference to one or more vectors and includes equivalents thereof known to those skilled in the art, and so forth.

The term "about" is used herein to mean approximately, roughly, around, or in the region of. When the term "about" is used in conjunction with a numerical range, it modifies that range by extending the boundaries above and below the numerical values set forth. In general, the term "about" is used herein to modify a numerical value above and below the stated value by a variance of 20 percent up or down (higher or lower).

As used herein, the word "or" means any one member of a particular list and also includes any combination of members of that list.

The term "nucleic acid" refers to deoxyribonucleotides or ribonucleotides and polymers or hybrids thereof in either single-or double-stranded, sense or antisense form.

Unless otherwise indicated, a particular nucleic acid sequence also implicitly encompasses conservatively modified variants thereof (*e.g.*, degenerate codon substitutions) and complementary sequences, as well as the sequence explicitly indicated. The term "nucleic acid" is used interchangeably herein with "gene", "cDNA, "mRNA", "oligonucleotide," and "polynucleotide".

The phrase "nucleic acid sequence" as used herein refers to a consecutive list of abbreviations, letters, characters or words, which represent nucleotides. In one embodiment, a nucleic acid can be a "probe" which is a relatively short nucleic acid, usually less than 100 nucleotides in length. Often a nucleic acid probe is from about 50 nucleotides in length to about 10 nucleotides in length. A "target region" of a nucleic acid is a portion of a nucleic acid that is identified to be of interest. A "coding region" of a nucleic acid is the portion of the nucleic acid, which is transcribed and translated in a sequence-specific manner to produce into a particular polypeptide or protein when placed under the control of appropriate regulatory sequences. The coding region is said to encode such a polypeptide or protein.

The term "antisense" is understood to mean a nucleic acid having a sequence complementary to a target sequence, for example a messenger RNA (mRNA) sequence the blocking of whose expression is sought to be initiated by hybridization with the target sequence.

The term "sense" is understood to mean a nucleic acid having a sequence which is homologous or identical to a target sequence, for example a sequence which binds to a protein transcription factor and which is involved in the expression of a given gene. According to a preferred embodiment, the nucleic acid comprises a gene of interest and elements allowing the expression of the said gene of interest.

The term "gene" refers to a coding region operably joined to appropriate regulatory sequences capable of regulating the expression of the polypeptide in some manner. A gene includes untranslated regulatory regions of DNA (*e.g*., promoters, enhancers, repressers, *etc*.) preceding (upstream) and following (downstream) the coding region (open reading frame, ORF) as well as, where applicable, intervening sequences (*i.e.*, introns) between individual coding regions (*i.e*., exons).

As used herein the term "coding region" when used in reference to a structural gene refers to the nucleotide sequences which encode the amino acids found in the nascent polypeptide as a result of translation of a mRNA molecule. The coding region is bounded, in eukaryotes, on the 5'-side by the nucleotide triplet "ATG" which encodes the initiator methionine and on the 3'-side by one of the three triplets, which specify stop cordons (*i.e*., TAA, TAG, TGA). In addition to containing introns, genomic forms of a gene may also include sequences located on both the 5'- and 3'-end of the sequences, which are present on the RNA transcript. These sequences are referred to as "flanking" sequences or regions (these flanking sequences are located 5' or 3' to the non-translated sequences present on the mRNA transcript). The 5'-flanking region may contain regulatory sequences such as promoters and enhancers, which control or influence the transcription of the gene. The 3'-flanking region may contain sequences, which direct the termination of transcription, posttranscriptional cleavage and polyadenylation.

The terms "polypeptide", "peptide", "oligopeptide", "polypeptide", "gene product", "expression product" and "protein" are used interchangeably herein to refer to a polymer or oligomer of consecutive amino acid residues.

Preferably, the term "isolated" when used in relation to a nucleic acid, as in "an isolated nucleic acid sequence" refers to a nucleic acid sequence that is identified and separated from at least one contaminant nucleic acid with which it is ordinarily associated in its natural source. Isolated nucleic acid is nucleic acid present in a form or setting that is different from that in which it is found in nature. In contrast, non-isolated nucleic acids are nucleic acids such as DNA and RNA which are found in the state they exist in nature. For example, a given DNA sequence (*e.g*., a gene) is found on the host cell chromosome in proximity to neighboring genes; RNA sequences, such as a specific mRNA sequence encoding a specific protein, are found in the cell as a mixture with numerous other mRNAs, which encode a multitude of proteins. However, an isolated nucleic acid sequence comprising SEQ ID NO:1 includes, by way of example, such nucleic acid sequences in cells which ordinarily contain SEQ ID NO:1 where the nucleic acid sequence is in a chromosomal or extrachromosomal location different from that of natural cells, or is otherwise flanked by a different nucleic acid sequence than that found in nature. The isolated nucleic acid sequence may be present in single-stranded or double-stranded form. When an isolated nucleic acid sequence is to be utilized to express a protein, the nucleic acid sequence will contain at a minimum at least a portion of the sense or coding strand (*i.e*., the nucleic acid sequence may be single-stranded). Alternatively, it may contain both the sense and anti-sense strands (*i.e*., the nucleic acid sequence may be double-stranded).

As used herein, the term "purified" refers to molecules, either nucleic or amino acid sequences that are removed from their natural environment, isolated or separated. An "isolated nucleic acid sequence" is therefore a purified nucleic acid sequence. "Substantially purified" molecules are at least 60% free, preferably at least 75% free, and more preferably at least 90% free from other components with which they are naturally associated.

As used herein, the terms "complementary" or "complementarity" are used in reference to nucleotide sequences related by the base-pairing rules. For example, the sequence 5'-AGT-3' is complementary to the sequence 5'-ACT-3'. Complementarity can be "partial" or "total." "Partial" complementarity is where one or more nucleic acid bases is not matched according to the base pairing rules. "Total" or "complete" complementarity between nucleic acids is where each and every nucleic acid base is matched with another base under the base pairing rules. The degree of complementarity between nucleic acid strands has significant effects on the efficiency and strength of hybridization between nucleic acid strands.

A "complement" of a nucleic acid sequence as used herein refers to a nucleotide sequence whose nucleic acids show total complementarity to the nucleic acids of the nucleic acid sequence.

The term "wild-type", "natural" or of "natural origin" means with respect to an organism, polypeptide, or nucleic acid sequence, that said organism is naturally occurring or available in at least one naturally occurring organism which is not changed, mutated, or otherwise manipulated by man.

The term "transgenic" or "recombinant" when used in reference to a cell refers to a cell which contains a transgene, or whose genome has been altered by the introduction of a transgene. The term "transgenic" when used in reference to a tissue or to a plant refers to a tissue or plant, respectively, which comprises one or more cells that contain a transgene, or whose genome has been altered by the introduction of a transgene. Transgenic cells, tissues and plants may be produced by several methods including the introduction of a "transgene" comprising nucleic acid (usually DNA) into a target cell or integration of the transgene into a chromosome of a target cell by way of human intervention, such as by the methods described herein.

The term "transgene" as used herein refers to any nucleic acid sequence, which is introduced into the genome of a cell by experimental manipulations. A transgene may be an "endogenous DNA sequence," or a "heterologous DNA sequence" (*i.e*., "foreign DNA"). The term "endogenous DNA sequence" refers to a nucleotide sequence, which is naturally found in the cell into which it is introduced so long as it does not contain some modification (*e.g.*, a point mutation, the presence of a selectable marker gene, *etc*.) relative to the naturally-occurring sequence. The term "heterologous DNA sequence" refers to a nucleotide sequence, which is ligated to, or is manipulated to become ligated to, a nucleic acid sequence to which it is not ligated in nature, or to which it is ligated at a different location in nature. Heterologous DNA is not endogenous to the cell into which it is introduced, but has been obtained from another cell. Heterologous DNA also includes an endogenous DNA sequence, which contains some modification. Generally, although not necessarily, heterologous DNA encodes RNA and proteins that are not normally produced by the cell into which it is expressed. Examples of heterologous DNA include reporter genes, transcriptional and translational regulatory sequences, selectable marker proteins (*e.g*., proteins which confer drug resistance), *etc.* Preferably, the term "transgenic" or "recombinant" with respect to a regulatory sequence (*e.g*., a promoter of the invention) means that said regulatory sequence is covalently joined and adjacent to a nucleic acid to which it is not adjacent in its natural environment.

The term "foreign gene" refers to any nucleic acid (*e.g*., gene sequence) which is introduced into the genome of a cell by experimental manipulations and may include gene sequences found in that cell so long as the introduced gene contains some modification (*e.g*., a point mutation, the presence of a selectable marker gene, *etc*.) relative to the naturally-occurring gene.

Preferably, the term "transgene" or "transgenic" with respect to, for example, a nucleic acid sequence (or an organism, expression construct or vector comprising said nucleic acid sequence) refers to all those constructs originating by experimental manipulations in which either
a) said nucleic acid sequence, or
b) a genetic control sequence linked operably to said nucleic acid sequence (a), for example a promoter, or
c) (a) and (b)
is not located in its natural genetic environment or has been modified by experimental manipulations, an example of a modification being a substitution, addition, deletion, inversion or insertion of one or more nucleotide residues. Natural genetic environment refers to the natural chromosomal locus in the organism of origin, or to the presence in a genomic library. In the case of a genomic library, the natural genetic environment of the nucleic acid sequence is preferably retained, at least in part. The environment flanks the nucleic acid sequence at least at one side and has a sequence of at least 50 bp, preferably at least-500 bp, especially preferably at least 1,000 bp, very especially preferably at least 5,000 bp, in length. A naturally occurring expression construct - for example the naturally occurring combination of a promoter with the corresponding gene - becomes a transgenic expression construct when it is modified by non-natural, synthetic "artificial" methods such as, for example, mutagenization. Such methods have been described (US 5,565,350; WO 00/15815).

"Recombinant" polypeptides or proteins refer to polypeptides or proteins produced by recombinant DNA techniques, *i.e*., produced from cells transformed by an exogenous recombinant DNA construct encoding the desired polypeptide or protein. Recombinant nucleic acids and polypeptide may also comprise molecules, which as such does not exist in nature but are modified, changed, mutated or otherwise manipulated by man.

The terms "heterologous nucleic acid sequence" or "heterologous DNA" are used interchangeably to refer to a nucleotide sequence, which is ligated to a nucleic acid sequence to which it is not ligated in nature, or to which it is ligated at a different location in nature. Heterologous DNA is not endogenous to the cell into which it is introduced, but has been obtained from another cell. Generally, although not necessarily, such heterologous DNA encodes RNA and proteins that are not normally produced by the cell into which it is expressed.

The "efficiency of transformation" or "frequency of transformation" as used herein can be measured by the number of transformed cells (or transgenic organisms grown from individual transformed cells) that are recovered under standard experimental conditions (*i.e*. standardized or normalized with respect to amount of cells contacted with foreign DNA, amount of delivered DNA, type and conditions of DNA delivery, general culture conditions *etc*.) For example, when isolated zygotes are used as starting material for transformation, the frequency of transformation can be expressed as the number of transgenic plant lines obtained per 100 isolated zygotes transformed.

The term "cell" refers to a single cell. The term "cells" refers to a population of cells. The population may be a pure population comprising one cell type. Likewise, the population may comprise more than one cell type. In the present invention, there is no limit on the number of cell types that a cell population may comprise. The cells may be synchronize or not synchronized, preferably the cells are synchronized.

The term "plant" as used herein refers to a plurality of plant cells, which are largely differentiated into a structure that is present at any stage of a plant's development. Such structures include one or more plant organs including, but are not limited to, fruit, shoot, stem, leaf, flower petal, *etc*.

The term "organ" with respect to a plant (or "plant organ") means parts of a plant and may include (but shall not limited to) for example roots, fruits, shoots, stem, leaves, anthers, sepals, petals, pollen, seeds, *etc*.

The term "tissue" with respect to a plant (or "plant tissue") means arrangement of multiple plant cells including differentiated and undifferentiated tissues of plants. Plant tissues may constitute part of a plant organ (*e.g*., the epidermis of a plant leaf) but may also constitute tumor tissues and various types of cells in culture (*e.g.,* single cells, protoplasts, embryos, calli, protocorm-like bodies, *etc*.). Plant tissue may be *in planta*, in organ culture, tissue culture, or cell culture.

The term "chromosomal DNA" or "chromosomal DNA-sequence" is to be understood as the genomic DNA of the cellular nucleus independent from the cell cycle status. Chromosomal DNA might therefore be organized in chromosomes or chromatids, they might be condensed or uncoiled. An insertion into the chromosomal DNA can be demonstrated and analyzed by various methods known in the art like *e.g*., polymerase chain reaction (PCR) analysis, Southern blot analysis, fluorescence *in situ* hybridization (FISH), and *in situ* PCR.

The term "structural gene" as used herein is intended to mean a DNA sequence that is transcribed into mRNA, which is then translated into a sequence of amino acids characteristic of a specific polypeptide.

The term "nucleotide sequence of interest" refers to any nucleotide sequence, the manipulation of which may be deemed desirable for any reason (*e.g*., confer improved qualities), by one of ordinary skill in the art. Such nucleotide sequences include, but are not limited to, coding sequences of structural genes (*e.g*., reporter genes, selection marker genes, oncogenes, drug resistance genes, growth factors, *etc*.), and non-coding regulatory sequences which do not encode an mRNA or protein product, (*e.g*., promoter sequence, polyadenylation sequence, termination sequence, enhancer sequence, *etc*.).

The term "expression" refers to the biosynthesis of a gene product. For example, in the case of a structural gene; expression involves transcription of the structural gene into mRNA and - optionally - the subsequent translation of mRNA into one or more polypeptides.

The term "transformation" as used herein refers to the introduction of genetic material (*e.g*., a transgene) into a cell. Transformation of a cell may be stable or transient. The term "transient transformation" or "transiently transformed" refers to the introduction of one or more transgenes into a cell in the absence of integration of the transgene into the host cell's genome. Transient transformation may be detected by, for example, enzyme-linked immunosorbent assay (ELISA), which detects the presence of a polypeptide encoded by one or more of the transgenes. Alternatively, transient transformation may be detected by detecting the activity of the protein (*e.g*., β-glucuronidase) encoded by the transgene (*e.g*., the *uidA* gene) as demonstrated herein [*e.g.*, histochemical assay of GUS enzyme activity by staining with X-gluc which gives a blue precipitate in the presence of the GUS enzyme; and a chemiluminescent assay of GUS enzyme activity using the GUS-Light kit (Tropix)]. The term "transient transformant" refers to a cell which has transiently incorporated one or more transgenes. In contrast, the term "stable transformation" or "stably transformed" refers to the introduction and integration of one or more transgenes into the genome of a cell, preferably resulting in chromosomal integration and stable heritability through meiosis. Stable transformation of a cell may be detected by Southern blot hybridization of genomic DNA of the cell with nucleic acid sequences, which are capable of binding to one or more of the transgenes. Alternatively, stable transformation of a cell may also be detected by the polymerase chain reaction of genomic DNA of the cell to amplify transgene sequences. The term "stable transformant" refers to a cell, which has stably integrated one or more transgenes into the genomic DNA. Thus, a stable transformant is distinguished from a transient transformant in that, whereas genomic DNA from the stable transformant contains one or more transgenes, genomic DNA from the transient transformant does not contain a transgene. Transformation also includes introduction of genetic material into plant cells in the form of plant viral vectors involving epichromosomal replication and gene expression, which may exhibit variable properties with respect to meiotic stability.

The terms "infecting" and "infection" with a bacterium refer to co-incubation of a target biological sample, (*e.g*., cell, tissue, *etc*.) with the bacterium under conditions such that nucleic acid sequences contained within the bacterium are introduced into one or more cells of the target biological sample.

The term "*Agrobacterium*" refers to a soil-borne, Gram-negative, rod-shaped phytopathogenic bacterium, which causes crown gall. The term "*Agrobacterium*" includes, but is not limited to, the strains *Agrobacterium tumefaciens,* (which typically causes crown gall in infected plants), and *Agrobacterium rhizogenes* (which causes hairy root disease in infected host plants). Infection of a plant cell with *Agrobacterium* generally results in the production of opines (*e.g*., nopaline, agropine, octopine *etc*.) by the infected cell. Thus, *Agrobacterium* strains which cause production of nopaline (*e.g*., strain LBA4301, C58, A208) are referred to as "nopaline-type" *Agrobacteria; Agrobacterium* strains which cause production of octopine (*e.g*., strain LBA4404, Ach5, B6) are referred to as "octopine-type" *Agrobacteria*; and *Agrobacterium* strains which cause production of agropine (*e.g*., strain EHA105, EHA101, A281) are referred to as "agropine-type" *Agrobacteria.*

The terms "bombarding, "bombardment," and "biolistic bombardment" refer to the process of accelerating particles towards a target biological sample (*e.g*., cell, tissue, *etc*.) to effect wounding of the cell membrane of a cell in the target biological sample and/or entry of the particles into the target biological sample. Methods for biolistic bombardment are known in the art (*e.g*., US 5,584,807), and are commercially available (*e.g*., the helium gas-driven microprojectile accelerator (PDS-1000/He) (BioRad).

The term "microwounding" when made in reference to plant tissue refers to the introduction of microscopic wounds in that tissue. Microwounding may be achieved by, for example, particle bombardment as described herein.

The term "expression construct" or "expression construct" as used herein is intended to mean the combination of any nucleic acid sequence to be expressed in operable linkage with a promoter sequence and - optionally - additional elements (like *e.g*., terminator and/or polyadenylation sequences) which facilitate expression of said nucleic acid sequence.

The term "promoter," "promoter element," or "promoter sequence" as used herein, refers to a DNA sequence which when ligated to a nucleotide sequence of interest is capable of controlling the transcription of the nucleotide sequence of interest into mRNA. A promoter is typically, though not necessarily, located 5' (*i.e*., upstream) of a nucleotide sequence of interest (*e.g*., proximal to the transcriptional start site of a structural gene) whose transcription into mRNA it controls, and provides a site for specific binding by RNA polymerase and other transcription factors for initiation of transcription.

Promoters may be tissue specific or cell specific. The term "tissue specific" as it applies to a promoter refers to a promoter that is capable of directing selective expression of a nucleotide sequence of interest to a specific type of tissue (*e.g*., petals) in the relative absence of expression of the same nucleotide sequence of interest in a different type of tissue (*e.g*., roots). Tissue specificity of a promoter may be evaluated by, for example, operably linking a reporter gene to the promoter sequence to generate a reporter construct, introducing the reporter construct into the genome of a plant such that the reporter construct is integrated into every tissue of the resulting transgenic plant, and detecting the expression of the reporter gene (*e.g*., detecting mRNA, protein, or the activity of a protein encoded by the reporter gene) in different tissues of the transgenic plant. The detection of a greater level of expression of the reporter gene in one or more tissues relative to the level of expression of the reporter gene in other tissues shows that the promoter is specific for the tissues in which greater levels of expression are detected. The term "cell type specific" as applied to a promoter refers to a promoter which is capable of directing selective expression of a nucleotide sequence of interest in a specific type of cell in the relative absence of expression of the same nucleotide sequence of interest in a different type of cell within the same tissue. The term "cell type specific" when applied to a promoter also means a promoter capable of promoting selective expression of a nucleotide sequence of interest in a region within a single tissue. Cell type specificity of a promoter may be assessed using methods well known in the art, *e.g*., GUS activity staining (as described for example in Example 7) or immunohistochemical staining. Briefly, tissue sections are embedded in paraffin, and paraffin sections are reacted with a primary antibody, which is specific for the polypeptide product encoded by the nucleotide sequence of interest whose expression is controlled by the promoter. A labeled (*e.g*., peroxidase conjugated) secondary antibody, which is specific for the primary antibody is allowed to bind to the sectioned tissue and specific binding detected (*e.g*., with avidin/biotin) by microscopy.

Promoters may be constitutive or regulatable. The term "constitutive" when made in reference to a promoter means that the promoter is capable of directing transcription of an operably linked nucleic acid sequence in the absence of a stimulus (*e.g*., heat shock, chemicals, light, *etc*.). Typically, constitutive promoters are capable of directing expression of a transgene in substantially any cell and any tissue. In contrast, a "regulatable" promoter is one which is capable of directing a level of transcription of an operably linked nuclei acid sequence in the presence of a stimulus (*e.g*., heat shock, chemicals, light, *etc*.) which is different from the level of transcription of the operably linked nucleic acid sequence in the absence of the stimulus.

The term "operable linkage" or "operably linked" is to be understood as meaning, for example, the sequential arrangement of a regulatory element (*e.g*. a promoter) with a nucleic acid sequence to be expressed and, if appropriate, further regulatory elements (such as *e.g*., a terminator) in such a way that each of the regulatory elements can fulfill its intended function to allow, modify, facilitate or otherwise influence expression of said nucleic acid sequence. The expression may result depending on the arrangement of the nucleic acid sequences in relation to sense or antisense RNA. To this end, direct linkage in the chemical sense is not necessarily required. Genetic control sequences such as, for example, enhancer sequences, can also exert their function on the target sequence from positions, which are further away, or indeed from other DNA molecules. Preferred arrangements are those in which the nucleic acid sequence to be expressed recombinantly is positioned behind the sequence acting as promoter, so that the two sequences are linked covalently to each other. The distance between the promoter sequence and the nucleic acid sequence to be expressed recombinantly is preferably less than 200 base pairs, especially preferably less than 100 base pairs, very especially preferably less than 50 base pairs. Operable linkage, and an expression construct, can be generated by means of customary recombination and cloning techniques as described (*e.g*., in Maniatis 1989; Silhavy 1984; Ausubel 1987; Gelvin 1990). However, further sequences, which, for example, act as a linker with specific cleavage sites for restriction enzymes, or as a signal peptide, may also be positioned between the two sequences. The insertion of sequences may also lead to the expression of fusion proteins. Preferably, the expression construct, consisting of a linkage of promoter and nucleic acid sequence to be expressed, can exist in a vector-integrated form and be inserted into a plant genome, for example by transformation.

The terms "homology" or "identity" when used in relation to nucleic acids refers to a degree of complementarity. Homology or identity between two nucleic acids is understood as meaning the identity of the nucleic acid sequence over in each case the entire length of the sequence, which is calculated by comparison with the aid of the program algorithm GAP (Wisconsin Package Version 10.0, University of Wisconsin, Genetics Computer Group (GCG), Madison, USA) with the parameters being set as follows:

| | |
|---|---|
| Gap Weight: 12 | Length Weight: 4 |
| Average Match: 2,912 | Average Mismatch:-2,003 |

For example, a sequence with at least 95% homology (or identity) to the sequence SEQ ID NO. 1 at the nucleic acid level is understood as meaning the sequence, which, upon comparison with the sequence SEQ ID NO. 1 by the above program algorithm with the above parameter set, has at least 95% homology. There may be partial homology (*i.e*., partial identity of less then 100%) or complete homology (*i.e*., complete identity of 100%).

Alternatively, a partially complementary sequence is understood to be one that at least partially inhibits a completely complementary sequence from hybridizing to a target nucleic acid and is referred to using the functional term "substantially homologous." The inhibition of hybridization of the completely complementary sequence to the target sequence may be examined using a hybridization assay (Southern or Northern blot, solution hybridization and the like) under conditions of low stringency. A substantially homologous sequence or probe (*i.e*., an oligonucleotide which is capable of hybridizing to another oligonucleotide of interest) will compete for and inhibit the binding (*i.e*., the hybridization) of a completely homologous sequence to a target under conditions of low stringency. This is not to say that conditions of low stringency are such that non-specific binding is permitted; low stringency conditions require that the binding of two sequences to one another be a specific (*i.e*., selective) interaction. The absence of non-specific binding may be tested by the use of a second target which lacks even a partial degree of complementarity (*e.g*., less than about 30% identity); in the absence of non-specific binding the probe will not hybridize to the second non-complementary target.

When used in reference to a double-stranded nucleic acid sequence such as a cDNA or genomic clone, the term "substantially homologous" refers to any probe which can hybridize to either or both strands of the double-stranded nucleic acid sequence under conditions of low stringency as described infra.

When used in reference to a single-stranded nucleic acid sequence, the term "substantially homologous" refers to any probe which can hybridize to the single-stranded nucleic acid sequence under conditions of low stringency as described infra.

The term "hybridization" as used herein includes "any process by which a strand of nucleic acid joins with a complementary strand through base pairing." (Coombs 1994). Hybridization and the strength of hybridization (*i.e*., the strength of the association between the nucleic acids) is impacted by such factors as the degree of complementarity between the nucleic acids, stringency of the conditions involved, the Tm of the formed hybrid, and the G:C ratio within the nucleic acids.

As used herein, the term "Tm" is used in reference to the "melting temperature." The melting temperature is the temperature at which a population of double-stranded nucleic acid molecules becomes half dissociated into single strands. The equation for calculating the Tm of nucleic acids is well known in the art. As indicated by standard references, a simple estimate of the Tm value may be calculated by the equation: Tm=81.5+0.41 (% G+C), when a nucleic acid is in aqueous solution at 1 M NaCl [see *e.g*., Anderson and Young, Quantitative Filter Hybridization, in Nucleic Acid Hybridization (1985)]. Other references include more sophisticated computations, which take structural as well as sequence characteristics into account for the calculation of Tm.

Low stringency conditions when used in reference to nucleic acid hybridization comprise conditions equivalent to binding or hybridization at 68°C. in a solution consisting of 5x SSPE (43.8 g/L NaCl, 6.9 g/L NaH₂PO₄.H₂O and 1.85 g/L EDTA, pH adjusted to 7.4 with NaOH), 1% SDS, 5x Denhardt's reagent [50x Denhardt's contains the following per 500 mL: 5 g Ficoll (Type 400, Pharmacia), 5 g BSA (Fraction V; Sigma)] and 100 µg/mL denatured salmon sperm DNA followed by washing in a solution comprising 0.2x SSPE, and 0.1% SDS at room temperature when a DNA probe of about 100 to about 1000 nucleotides in length is employed. High stringency conditions when used in reference to nucleic acid hybridization comprise conditions equivalent to binding or hybridization at 68° C. in a solution consisting of 5x SSPE, 1% SDS, 5x Denhardt's reagent and 100 µg/mL denatured salmon sperm DNA followed by washing in a solution comprising 0.1x SSPE, and 0.1% SDS at 68° C. when a probe of about 100 to about 1000 nucleotides in length is employed.

The term "equivalent" when made in reference to a hybridization condition as it relates to a hybridization condition of interest means that the hybridization condition and the hybridization condition of interest result in hybridization of nucleic acid sequences (which have the same range of percent (%) homology. For example, if a hybridization condition of interest results in hybridization of a first nucleic acid sequence with other nucleic acid sequences that have from 80% to 90% homology to the first nucleic acid sequence, then another hybridization condition is said to be equivalent to the hybridization condition of interest if this other hybridization condition also results in hybridization of the first nucleic acid sequence with the other nucleic acid sequences that have from 80% to 90% homology to the first nucleic acid sequence.

When used in reference to nucleic acid hybridization the art knows well that numerous equivalent conditions may be employed to comprise either low or high stringency conditions; factors such as the length and nature (DNA, RNA, base composition) of the probe and nature of the target (DNA, RNA, base composition, present in solution or immobilized, *etc*.) and the concentration of the salts and other components (*e.g*., the presence or absence of formamide, dextran sulfate, polyethylene glycol) are considered and the hybridization solution may be varied to generate conditions of either low or high stringency hybridization different from, but equivalent to, the above-listed conditions. Those skilled in the art know that whereas higher stringencies may be preferred to reduce or eliminate non-specific binding between the nucleotide sequence of SEQ ID NOs:1 or 2 and other nucleic acid sequences, lower stringencies may be preferred to detect a larger number of nucleic acid sequences having different homologies to the nucleotide sequence of SEQ ID NOs:1 and 2.

### DETAILED DESCRIPTION OF THE INVENTION

A first subject matter of the invention therefore relates to a transgenic expression constructs for predominant expression of a nucleic acid sequence of interest in substantially all vegetative plant tissues comprising a promoter sequence selected from the group consisting of
a) the promoter of the *Pisum sativum* ptxA gene as described by SEQ ID NO:1 and
b) a nucleotide sequence which has essentially the same promoter activity as the promoter sequence described by SEQ ID NO:1, and has an identity of at least 80% over a sequence of at least 700 consecutive base pairs to the sequence as described by SEQ ID NO: 1
wherein said promoter sequence is operably linked to a nucleic acid sequence of interest to be transgenically expressed, and wherein said promoter sequence is heterologous with respect to said nucleic acid sequence of interest.

The sequence of the ptxA gene from *Pisum sativum* is disclosed (GenBank Acc.-No.: X67427). However, the promoter was so far not isolated and combined with other (heterologous) sequences to realized transgenic expression. The promoter region of the ptxA gene has approximately 50% nucleotide sequence identity to the promoter region of *Medicago sativa* proline-rich protein (MsPRP2) gene. In some regions the sequence identity raises even higher, up to 87% over 100 consecutive base pairs. On protein level the similarity between the ptxA protein and the MsPRP protein is also very high (see Fig. 6 a and b). The ptxA gene encodes for a protein of 352 amino acids. The sequences include 60-80% amino acid identity in 83 residues to the proline-rich proteins from *Medicago truncatula, Lycopersicon esculentum, Solanum brevidens, Vitis vinifera* or *Zea mays.* In the same residues, the sequence alignment shows approximately 50% amino acid identity to the probable cell wall-plasma membrane linker protein (PRP) from *Arabidopsis thaliana,* 48% identity to the protease inhibitor/seed storage/lipid transfer protein (LTP) family from *Arabidopsis thaliana,* 95% identity to salt-inducible protein RF2 from *Medi*cago *sativa,* and 53 % identity to root-specific protein RCc3 from *Oryza sativa.* However, the MsPRP2 promoter is described to be both root-specific and salt-inducible (Bastola 1998; WO 99/53016). Both reported expression characteristics for the MsPRP2 promoter are significantly different from the expression patterns that were observed for the ptxA promoter of this invention. Its vegetative plant tissue/organ specific, stress-independent expression profile is surprising and unexpected with respect to the MsPRP2 promoter expression profile.

Ahn *et a*/*.* (1998) reported that the expression of soybean hydroxyproline-rich glycoprotein (SbHRGP3) gene is required for root maturation to terminate root elongation. The SbHRGP3 gene encodes for a protein of 432 amino acids representing hydroxyproline-rich glycoprotein with 50-80% amino acid sequence identity to HRGP or extensin protein from *Phaseolus vulgaris, Pisum sativum, Solanum tuberosum.* Combination of wounding and sucrose enhanced expression of this gene in roots. In leaves, both wounding and sucrose were required for the expression of *SbHRGP3.* The sequence of the SbHRGP3 gene is disclosed (GenBank Acc.-No.: U44838). However, the promoter was so far not combined with other (heterologous) sequences to realized transgenic expression. It is surprising, that a heterologous combination totally changes the expression profile of the SbHRGP3 promoter described in the art. Both the root specific and the stress-inducible expression pattern described for the native gene cannot be observed in the transgenic expression construct of this invention. This is surprising. The change in the expression profile may be explained by the absence of regulatory elements (mediating the tissue specificity and stress responsiveness of the native gene) not present in the promoter region (but *e.g*., in introns of the native gene) and/or by the absence of regulatory proteins in the heterologous plant species

The promoter sequences of the ptxA or SbHRGP3 gene demonstrate a highly uniform, homogenous expression activity in virtually all vegetative plant tissues of various species including dicotyledonous and monocotyledonous plants. In seeds and flowers, there is no expression activity detectable by GUS staining (see Example 7, and Fig. 3, 4, and 5) and low expression activity detectable with the more sensitive method of RT-PCR (data not shown). Only in plant lines comprising multiple copies of a transgenic ptxA-promoter / GUS expression construct some expression can be detected in part of the flowers and the siliques (seedpods). It is an advantage that no or very little transgenic protein will be expressed in the seed (which is used for food and feed purpose) and flowers (which is preferred from an environmental point of view). For numerous agronomically valuable traits (*e.g*., stress resistance, improved water use, resistance against fungi or insects, *etc*.) no expression in seeds and flowers is required. Therefore, avoidance of this unnecessary expression may facilitate regulatory approval and/or consumer acceptance.

Furthermore, data from the β-glucuronidase (GUS) expression assay suggest, that the promoter activity in the vegetative plant tissues and organs at the vegetative stages is relatively stronger than at the reproductive stages. In consequence the promoter activity is most active in the young vulnerable plantlet, but becomes lower in the mature plant. This is of an additional advantage, especially for genes, which confer resistance against biotic or abiotic stress factors (*e.g*., cold, drought, insect damage, *etc*.) since young, developing plants are considered much more vulnerable against said stress factors then mature plants. The promoter activity of the promoters of the invention is specially high in non-differentiated or de-differentiated tissue or cells like, *e.g*., callus culture. This is very useful for utilizing the promoter in combination with selection marker in transformation protocols.

The invention furthermore relates to a method for transgenic predominant expression of a nucleic acid sequence of interest in substantially all vegetative plant tissues and/or organs, but where the expression is comparatively low or non existing in seeds and flowers comprising:
i. introduction of a transgenic expression construct into a plant cell or a plant, said transgenic expression construct comprising a promoter sequence selected from the group consisting of
   a) a nucleotide sequences as described above having essentially the same promoter activity as the promoter sequence described by SEQ ID NO:1,
wherein said promoter sequence or a nucleotide sequences as described above having essentially the same promoter activity as the promoter sequence described by SEQ ID NO:1, is operably linked to a nucleic acid sequence of interest to be transgenically expressed, and wherein said promoter sequence or a nucleotide sequences as described above having essentially the same promoter activity as the promoter sequence described by SEQ ID NO:1, is heterologous with respect to said nucleic acid sequence of interest, under conditions such that said nucleic acid sequence of interest is expressed in said plant cell and/or predominantly expressed in the vegetative plant tissue and/or organs of said transgenic plant.

In a preferred embodiment, the method further comprises ii) identifying or selecting the transgenic plant cell comprising said transgenic expression construct. In another preferred embodiment, the method further comprises iii) regenerating transgenic plant tissue from the transgenic plant cell. In an alternative preferred embodiment, the methods further comprises iv) regenerating a transgenic plant from the transgenic plant cell.

A preferred functional equivalent fragment of the ptxA promoter comprises a sequence from about base pair 300 to about base pair 583 of the sequence described by SEQ ID NO: 1.

The transgenic expression construct of the invention may comprise further genetic control sequences linked operably to the nucleic acid sequence of interest to be expressed is to, and/or additional functional elements.

The nucleic acid sequence of interest transgenically expressed from the transgenic expression construct of the invention may results in expression of a protein encoded by said nucleic acid sequence (by transcription and subsequent translation), and/or expression of sense, antisense or double-stranded RNA encoded by said nucleic acid sequence of interest.

In another embodiment, nucleotide sequence encoding the transgenic expression construct of the invention is double-stranded. In yet another embodiment, the nucleotide sequence encoding the transgenic expression construct of the invention is single-stranded.

In yet another alternative embodiment, the transgenic expression construct of the invention is contained in a vector or in a plant cell or a plant. In a preferred embodiment, the plant cell is derived from a dicotyledonous or monocotyledonous plant. In a yet more preferred embodiment, the monocotyledonous plant is selected from the group consisting of sugarcane, maize, sorghum, pineapple, rice, barley, oat, wheat, rye, yam, onion, banana, coconut and date. In a yet more preferred embodiment, the dicotyledonous plant is selected from the group consisting of hop, rapeseed, tobacco, tomato, tagetes (marigold), soybean, pea, common bean, and papaya.

Further embodiments of the invention relate to the use of a transgenic organism of the invention or of cell cultures, parts of transgenic propagation material derived therefrom for the production of foodstuffs, animal feeds, seed, pharmaceuticals or fine chemicals.
Another embodiment of the invention related to a method for production of a foodstuff, animal feed, seed, pharmaceutical or fine chemical employing a transgenic organism of the invention or of cell cultures, parts of transgenic propagation material derived therefrom.

Beside the promoter sequence as described by SEQ ID NO 1 additional sequences are subject of the present invention.

Another embodiment of the invention relates to function equivalent homologs of the ptxA promoter, preferably functional equivalent homologs of the promoter sequences as described by SEQ ID NO 1. A "functional equivalent homolog" of SEQ ID NOs:1 is defined as a nucleotide sequence having less than 100% homology with SEQ ID NOs: 1 but having at least 80% identity over a sequence of at least 700 consecutive base pairs to the sequence as described by SEQ ID NO:1 and which has promoter activity having the essential characteristics (vegetative plant tissue specific expression) of the promoter activity of SEQ ID NOs:1. Functional equivalent homologs of SEQ ID NOs:1, and of functional equivalent fragments (portions) thereof, include, but are not limited to, nucleotide sequences having deletions, insertions or substitutions of different nucleotides or nucleotide analogs as compared to SEQ ID NOs:1

Functional equivalent homologs with regard to the ptxA promoter as defined above means, in particular, natural or artificial mutations of the ptxA promoter sequence described in SEQ ID NO: 1 or 2 or of the deletion variants derived or its homologs from other plant genera and plant species which continue to exhibit essentially the same promoter activity.

A promoter activity - with respect to the ptxA promoter - is termed essentially the same when the transcription of any nucleic acid sequence expressed under the control of a specific promoter in a plant takes predominantly place in substantially all vegetative plant tissues and/or organs but is comparatively low or non existing in seeds and flowers.

The term "vegetative plant tissue" or "vegetative organs" as used herein in intended to comprise all organs and tissues of a plant beside seeds and flowers (the reproductive organs leading to development of seeds).

The term "seed" as used herein means seeds in all developmental stages, preferably a mature seed. A mature seed is understood to comprise seeds, which have reached physiological maturity in all stages between the late stages of seed development to dried seed after harvest. Preferably the term seed means seed in the condition where it is normally stored and marketed for feed and food purpose. Such seed may be characterized by its water content. Depending on the target plant, the water content in the whole seeds can range from about 5% (w/w) (for *e.g*. dried *Arabidopsis* seeds) to about 30% (for *e.g*. whole maize seeds on a fresh weight basis) (Villela 1998).

The term "flower" as used herein means the reproductive organ of an flowering (angio-sperm) plant being that part of a plant destined to produce seed, and hence including one or both of the sexual organs; an organ or combination of the organs of reproduction, whether enclosed by a circle of foliar parts or not. A complete flower consists of two essential parts, the stamens and the carpels, and two floral envelopes, the corolla and callyx. In mosses the flowers consist of a few special leaves surrounding or subtending organs called archegonia.

The term "substantially all vegetative plant tissues or organs" means that the accumulated biomass of organs (or tissues), for which an expression under control of a promoter of the invention can be detected, adds up for more than 50%, preferably more than 80%, more preferably more than 90% of the total biomass of the vegetative organs (or tissues) (which is the total biomass of the plantlet or plant minus the biomass of the seed and flowers). Possible are scenarios where one or more vegetative organ (or tissues) do not demonstrate detectable expression. Preferably, expression in the vegetative organs occurs at least in stems, leaves, or roots and in undifferentiated cells (like, *e.g*., callus). In a preferred embodiment that term "substantially all vegetative plant tissues or organs" means a promoter which has no detectable expression (as for example judged by employing a promoter/GUS expression cassette) in seed tissue but has detectable expression in at least one tissue selected from the group of leaves, stem, and roots. More preferably said promoter has expression in leafs, stem and roots (but not in seeds).

The term "comparatively low" with respect to expression in the seed and/or flower tissues or organs, means that the expression rate realized by the transgenic expression construct (as measured by any of the methods given below, or exemplified in the Examples: preferably by a quantitative β-glucuronidase assay) and normalized to units of β-glucuronidase per gram of biomass in seed and/or flower tissue is less the 10% of the corresponding value in total vegetative plant tissues, preferably less then 5%.

In a preferred embodiment of the invention, a promoter activity is considered essentially the same, especially, when the expression rate of a promoter decreases during development (*i.e.* the promoter activity in the tissues and organs at the vegetative stages is relatively stronger than that at the reproductive stages).

In the even more preferred embodiment of the invention, a promoter activity is considered essentially the same, especially, when the expression rate of a promoter is especially high in non-differentiated or de-differentiated tissue or cells like, *e.g*., callus culture.

The expression level of a functional equivalent homolog promoter may be lower or higher when compared with a reference value obtained by a promoter as described by SEQ ID NO: 1 in a specific tissue (although the expression pattern remains essentially the same). Preferred sequences are those whose expression level, measured on the basis of the transcribed mRNA or the protein which is translated as a consequence, differs quantitatively by not more than 50%, preferably 25%, especially preferably 10%, from a reference value obtained with the promoter described by SEQ ID NO: 1 under otherwise unchanged conditions.

Functional equivalent homologs also comprise those promoter sequences whose function, compared with the ptxA promoter as shown in SEQ ID NO: 1 is reduced or increased. In this context, the promoter activity is at least 50% higher, preferably at least 100% higher, especially preferably at least 300% higher, very especially preferably at least 500% higher than a reference value obtained with the ptxA promoter as shown in SEQ ID NO: 1 under otherwise unchanged conditions. Preferably, the activity falls short of that of the ptxA or SbHRGP3 promoter as shown in SEQ ID NO: 1 by not more than 80%, preferably not more than 50%, especially preferably not more than 20%, very especially preferably not more than 10%.

The term "promoter activity" when made in reference to a nucleic acid sequence refers to the ability of the nucleic acid sequence to initiate transcription of an operably linked nucleotide sequence into mRNA. The terms "operably linked," "in operable combination," and "in operable order" as used herein refer to the linkage of nucleic acid sequences in a manner such that a nucleic acid molecule is capable of directing the transcription of nucleic acid sequence of interest and/or the synthesis of a polypeptide sequence of interest. Promoter activity may be determined using methods Known in the art. For example, a candidate nucleotide sequence whose promoter activity is to be determined is ligated in-frame to a nucleic acid sequence of interest (*e.g*., a reporter gene sequence, a selectable marker gene sequence) to generate a reporter vector, introducing the reporter vector into plant tissue using methods described herein, and detecting the expression of the reporter gene (*e.g*., detecting the presence of encoded mRNA or encoded protein, or the activity of a protein encoded by the reporter gene). The reporter gene may express visible markers. Reporter gene systems which express visible markers include β-glucuronidase and its substrate (X-Gluc), luciferase and its substrate (luciferin), and β-galactosidase and its substrate (X-Gal) which are widely used not only to identify transformants, but also to quantify the amount of transient or stable protein expression attributable to a specific vector system (Rhodes 1995). In a preferred embodiment, the reporter gene is a GUS gene. The selectable marker gene may confer antibiotic or herbicide resistance. Examples of reporter genes include, but are not limited to, the *dhfr* gene, which confers resistance to methotrexate (Wigler 1980); *npt,* which confers resistance to the aminoglycosides neomycin and G-418 (Colbere-Garapin 1981) and *als* or *pat*, which confer resistance to chlorsulfuron and phosphinotricin acetyl transferase, respectively. Detecting the presence of encoded mRNA or encoded protein, or the activity of a protein encoded by the reporter gene or the selectable marker gene indicates that the candidate nucleotide sequence has promoter activity.

The term "otherwise unchanged conditions" means - for example - that the expression which is initiated by one of the expression constructs to be compared is not modified by combination with additional genetic control sequences, for example enhancer sequences and is done in the same environment (*e.g*., the same plant species) at the same developmental stage and under the same growing conditions.

Functional equivalent homologs with regard to the ptxA promoter means, in particular, natural or artificial mutations of the ptxA promoter sequence described in SEQ ID NO: 1 or of the deletion variants derived or its homologs from other plant genera and plant species which continue to exhibit essentially the same promoter activity.

Mutations encompass substitutions, additions, deletions, inversions or insertions of one or more nucleotide residues. Thus, those nucleotide sequences, which are obtained by modification of the ptxA promoter as shown in SEQ ID NO: 1 are also encompassed by the present invention. Aim of such a modification may be the further delimitation of the sequence comprised therein or else, for example, the introduction of further cleavage sites for restriction enzymes, the removal of excess DNA or the addition of further sequences, for example further regulatory sequences.

Where insertions, deletions or substitutions such as, for example, transitions and transversions are suitable, techniques known *per se* such as *in vitro* mutagenesis, primer repair, restriction or ligation may be used. In the case of suitable manipulations such as, for example, restriction, chewing back or filling in overhangs for blunt ends, complementary ends of the fragments may be provided for ligation. Analogous results may also be achieved using the polymerase chain reaction (PCR) using specific oligonucleotide primers.

Functional equivalent homologs of a promoter sequence as described by SEQ ID NO: 1 (for example by substitution, insertion or deletion of nucleotides; or representing a homologous promoter from another plant species) have at least 80% homology, by preference at least 90% homology, especially preferably at least 95% homology, very especially preferably at least 98% homology - but less then 100% homology - to the promoter sequence as described by SEQ ID NO: 1, wherein said homology is determined over a sequence of at least 700 consecutive base pairs, preferably at least 800 consecutive base pairs, more preferably at least 850 consecutive base pairs of the sequence as described by SEQ ID NO: 1, and are having essentially the same promoter activity characteristics as the ptxA promoter as shown in SEQ ID NO: 1.

In an preferred embodiment, functional equivalent homologs of a promoter sequence as described by SEQ ID NO: 1 (for example by substitution, insertion or deletion of nucleotides; or representing a homologous promoter from another plant species) have at least 90% homology, preferably at least 95% homology, by preference at least 97% homology, especially preferably at least 98% homology, very especially preferably at least 99% homology - but less then 100% homology - to the promoter sequence as described by SEQ ID NO: 1, wherein said homology is determined over a sequence of at least 700 consecutive base pairs of the sequence as described by SEQ ID NO: 1, and are having essentially the same promoter activity characteristics as the ptxA promoter as shown in SEQ ID NO: 1.

In an more preferred embodiment, functional equivalent homologs of a promoter sequence as described by SEQ ID NO: 1 (for example by substitution, insertion or deletion of nucleotides; or representing a homologous promoter from another plant species) have at least 95% homology, preferably at least 96% homology, by preference at least 97% homology, especially preferably at least 98% homology, very especially preferably at least 99% homology - but less then 100% homology - to the promoter sequence as described by SEQ ID NO: 1, wherein said homology is determined over a sequence of at least 700 consecutive base pairs of the sequence as described by SEQ ID NO: 1, and are having essentially the same promoter activity characteristics as the ptxA promoter as shown in SEQ ID NO: 1.

Functional equivalent homologs of a promoter sequence as described by SEQ ID NO: 1 are not to be understood to include the promoter of the MsPRP2 gene from alfalfa (Bastola 1998; WO 99/53016).

Further examples of promoter sequences employed in the expression constructs or expression vectors according to the invention can be found readily in different organisms whose genomic sequence is known such as, for example, *Arabidopsis thaliana, Brassica napus, Nicotiana tabacum, Solanum tuberosum, Helianthus anuus, Linum sativum* from databases by homology alignment.

Methods for the generation of artificial functional equivalent homologs according to the invention preferably comprise the introduction of mutations into the ptxA promoter as shown in SEQ ID NO: 1 Mutagenesis can be random, the mutagenized sequences subsequently being screened in a "trial-and-error" procedure for their characteristics. Methods for the mutagenic treatment of nucleic acid sequences are known to the skilled worker and include, for example, the use of oligonucleotides with one or more mutations in comparison with the region to be mutated (for example in a "site-specific mutagenesis"). Typically, primers with approximately 15 to approximately 75 nucleotides or more are employed, with approximately 10 to approximately 25 or more nucleotide residues preferably being located on both sides of the sequence to be modified. Details and the procedure of said mutagenesis methods are known to the skilled worker (Kunkel 1987; Tomic 1990; Upender 1995; US 4,237,224). A mutagenesis can also be carried out by treating for example vectors comprising one of the nucleic acid sequences according to the invention with mutagens such as hydroxylamine.

Natural occurring functional equivalent homologs can be identified and isolated either starting from the promoter sequences as described by SEQ ID NO: 1 or - alternatively - by starting from the corresponding protein encoding sequences. The latter are normally demonstrating higher significant homologies and allow for easier identification of corresponding genes in other plant species.

Examples for functional equivalent promoter sequences which can be employed in the transgenic expression cassettes of the invention can be identified and/or isolated from organism which genomic sequence is known (*e.g*., *Arabidopsis thaliana, Brassica napus, Nicotiana tabacum, Solanum tuberosum, Helianthium annuus, Linum sativum*) by homology search in the corresponding databases. Preferably, the person skilled in the art will start such analysis based on the coding regions of the genes, which promoters are described by SEQ ID NO: 1.

Natural occurring functional equivalent promoter sequences can be identified and isolated by multiple methods known in the art. For example, probes or primers derived from either the promoter sequences as described by SEQ ID NO: 1 or the corresponding protein encoding sequences can be employed to screen libraries of genomic DNA clones.

As used herein, the term "probe" refers to an oligonucleotide, whether occurring naturally as in a purified restriction digest or produced synthetically, recombinantly or by PCR amplification, which is capable of hybridizing to a nucleotide sequence of interest. A probe may be single-stranded or double-stranded. It is contemplated that any probe used in the present invention will be labeled with any "reporter molecule," so that it is detectable in any detection system including, but not limited to enzyme (*e.g*., ELISA, as well as enzyme-based histochemical assays), fluorescent, radioactive, calorimetric, gravimetric, magnetic, and luminescent systems. It is not intended that the present invention be limited to any particular detection system or label.

The probes provided herein are useful in the detection, identification and isolation of, for example, sequences such as those listed as SEQ ID NOs:1 as well as of homologs thereof. Preferred probes are of sufficient length (*e.g.*, from about 9 nucleotides to about 20 nucleotides or more in length) such that high stringency hybridization may be employed. In one embodiment, probes from 20 to 50 nucleotide bases in length are employed.

Similar a portion of the nucleic acid sequences set forth as SEQ ID NOs:1 can be used as a primer for the amplification of nucleic acid sequences useful as function equivalent homologs by, for example, polymerase chain reactions (PCR) or reverse transcription-polymerase chain reactions (RT-PCR). The term "amplification" is defined as the production of additional copies of a nucleic acid sequence and is generally carried out using polymerase chain reaction technologies well known in the art (Dieffenbach 1995). With PCR, it is possible to amplify a single copy of a specific target sequence in genomic DNA to a level detectable by several different methodologies (*e.g*., hybridization with a labeled probe; incorporation of biotinylated primers followed by avidin-enzyme conjugate detection; and/or incorporation of ³²P-labeled deoxyribonucleotide triphosphates, such as dCTP or dATP, into the amplified segment).

As used herein, the term "primer" refers to an oligonucleotide, whether occurring naturally as in a purified restriction digest or produced synthetically, which is capable of acting as a point of initiation of synthesis when placed under conditions in which synthesis of a primer extension product which is complementary to a nucleic acid strand is induced, (*i.e*., in the presence of nucleotides and an inducing agent such as DNA polymerase and at a suitable temperature and pH). The primer is preferably single stranded for maximum efficiency in amplification, but may alternatively be double stranded. If double stranded, the primer is first treated to separate its strands before being used to prepare extension products. Preferably, the primer is an oligodeoxyribonucleotide. The primer must be sufficiently long (*e.g*., from about 9 nucleotides to about 20 nucleotides or more in length) to prime the synthesis of extension products in the presence of the inducing agent. Suitable lengths of the primers may be empirically determined and depend on factors such as temperature, source of primer and the use of the method. In one embodiment, the present invention employs probes from 20 to 50 nucleotide bases in length.

The invention also contemplates functional equivalent fragments of at least 700 consecutive base pairs of SEQ ID NOs:1 (and functional equivalent homologs thereof) having essentially the same promoter activity. Functional equivalent fragments of ptxA promoter can be produced preferably by eliminating (deleting) non-essential sequences and restricting the original sequence to those comprising promoter elements affecting promoter activity, but without adversely affecting the abovementioned characteristics to a significant extent. Such functional equivalent fragments are also termed "core promoter" or "core promoter region" herein.

Sequences within a promoter, which affect promoter activity, may be determined by using deletion constructs such as those described by Sherri et al. (US 5,593,874). Briefly, several expression plasmids are constructed to contain a reporter gene under the regulatory control of different candidate nucleotide sequences which are obtained either by restriction enzyme deletion of internal sequences in SEQ ID NOs:1, restriction enzyme truncation of sequences at the 5' and/or 3' end of SEQ ID NOs:1, or by the introduction of single nucleic acid base changes by PCR into SEQ ID NOs:1. Expression of the reporter gene by the deletion constructs is detected. Detection of expression of the reporter gene in a given deletion construct indicates that the candidate nucleotide sequence in that deletion construct has promoter activity.

Alternatively or in combination, restricting (cutting down) of the ptxA or SbHRGP3 promoter sequence to specific essential regulatory regions can also be achieved with the aid of search routine for the search of promoter elements. Specific promoter elements are frequently accumulated in the regions, which are relevant for promoter activity. This analysis can be carried out for example with computer programs such as the program PLACE ("Plant Cis-acting Regulatory DNA Elements") (Higo 1999).

The core region of the ptxA promoter described by SEQ ID NO: 1 was determined by promoter element analysis based on the PLACE algorithm (see Example 14). Based on the below given PLACE results a potential TATA box is localized at base pair 549 to base pair 554 of SEQ ID NO: 1. In consequence the 5' untranslated region starts at about base pair 584 and extends to base pair 863 of SEQ ID NO: 1. The sequence described by SEQ ID NO: 1 ends just before the ATG start codon. It is known for the person skilled in the art, that the 5' untranslated region is not part of the promoter. Therefore, this 5' untranslated region may be deleted to obtain a function equivalent fragment of the ptxA promoter as described by SEQ ID NO: 1. Based on the promoter element analysis there seem to be no clusters of promoter elements in the first 300 base pairs of the sequence described by SEQ ID NO: 1. Therefore, this region may be deleted to obtain a function equivalent fragment of the ptxA promoter as described by SEQ ID NO: 1. It is therefore very likely that the core region of the ptxA promoter extents from about base pair 300 to about base pair 583 of the sequence described by SEQ ID NO: 1.

Therefore, in a preferred embodiment of the invention a functional equivalent promoter fragment of the ptxA promoter as described by SEQ ID NO: 1 comprises a sequences described by the sequence of about base pair 300 to about 863 of SEQ ID NO: 1 or the sequence of about base pair 1 to about 583 of SEQ ID NO: 1, preferably the sequence of about base pair about 300 to about 863 of SEQ ID NO: 1.

The nucleotide sequence of SEQ ID NOs: 1 fragments, homologs and antisense sequences thereof may be synthesized by synthetic chemistry techniques which are commercially available and well known in the art [see Caruthers 1980; Horn 1980). Additionally, fragments of SEQ ID NOs:1 can be made by treatment of SEQ ID NOs:1 with restriction enzymes followed by purification of the fragments by gel electrophoresis. Alternatively, sequences may also produced using the polymerase chain reaction (PCR) as described by Mullis [US 4,683,195, 4,683,202 and 4,965,188, all of which are hereby incorporated by reference]. SEQ ID NOs:1 portions, homologs and antisense sequences thereof may be ligated to each other or to heterologous nucleic acid sequences using methods well known in the art.

The nucleotide sequence of synthesized sequences may be confirmed using commercially available kits as well as using methods well known in the art which utilize enzymes such as the Klenow fragment of DNA polymerase 1, Sequenase^{®}, Taq DNA polymerase, or thermostable T7 polymerase. Capillary electrophoresis may also be used to analyze the size and confirm the nucleotide sequence of the products of Nucleic acid synthesis, restriction enzyme digestion or PCR amplification.

### THE EXPRESSION CONSTRUCT OF THE INVENTION

In the transgenic expression construct of the invention one or more of the promoter sequences described above are operably linked (as defined above) to a nucleic acid of interest.

Beside the promoter sequence and the nucleic acid of interest operably linked thereto, the expression construct of the invention may comprise further genetic control sequences (as defined below in detail). For example, at the 3' end of the nucleic acid sequence of interest, other DNA sequences may also be included, *e.g*., a 3' untranslated region containing a polyadenylation site and transcription termination sites. Further sequences, which, for example, act as a linker with specific cleavage sites for restriction enzymes, or as a signal peptide, may be positioned between the promoter and the nucleic acid sequence of interest. For example, an expression construct according to the invention is generated by fusing the ptxA promoter (or a functional equivalent or functionally equivalent portion as shown in SEQ-ID NO: 1 or a functional equivalent) to a nucleic acid sequence to be expressed, and a terminator signal or polyadenylation signal.

A transgenic expression cassette of the invention (or a transgenic vector comprising said transgenic expression cassette) can be produced by means of customary recombination and cloning techniques as are described (for example, in Maniatis 1989; Silhavy 1984; and in Ausubel 1987).

However, a transgenic expression construct of the invention is also understood as meaning those constructs in which a promoter of the invention is introduced into a host genome without previously having been linked operably to a nucleic acid sequence of interest to be expressed (*e.g*., via directed homologous recombination or random insertion), and then, in this host genome, takes on regulatory control over the nucleic acid sequences to which it is now linked operably, and governs the transgenic expression of the latter. By inserting the promoter, for example by homologous recombination, upstream of an endogenous nucleic acid encoding a specific polypeptide, an expression construct according to the invention is obtained which governs the expression of the specific polypeptide in the vegetative plant tissues. Furthermore, insertion of the promoter may also be effected in such a manner that RNA, which is antisense to the nucleic acid encoding a specific polypeptide, is expressed. This selectively down-regulates or switches off expression of the specific polypeptide in the vegetative plant tissues.

Analogously, a nucleic acid sequence of interest to be expressed recombinantly may also be placed downstream of the endogenous natural ptxA promoter (or a function equivalent homolog thereof in another plant species), for example by homologous recombination, whereby an expression construct according to the invention is obtained which governs the expression, of the nucleic acid sequence to be expressed recombinantly, in the cotyledons of the plant embryo.

### FURTHER GENETIC CONTROL SEQUENCES

The transgenic expression construct of the invention may comprise further genetic control sequences in addition to the inventive promoter. The term "genetic control sequences" is to be understood in the broad sense and refers to all those sequences, which have an effect on the materialization, production, propagation, replication, or the function of the expression construct according to the invention. For example, genetic control sequences modify the transcription and translation in prokaryotic or eukaryotic organisms. Preferably, the expression constructs according to the invention encompass a promoter functional in plants 5'-upstream of the nucleic acid sequence in question to be expressed recombinantly, and 3'-downstream a terminator sequence as additional genetic control sequence and, if appropriate, further customary regulatory elements, in each case linked operably to the nucleic acid sequence to be expressed recombinantly.

Genetic control sequences furthermore also encompass the 5'-untranslated regions, introns or non-coding 3'-region of genes, such as, for example, the actin-1 intron, or the Adh1-S introns 1, 2 and 6 (general reference: The Maize Handbook, Chapter 116, Freeling and Walbot, Eds., Springer, New York (1994)). It has been demonstrated that they may play a significant role in the regulation of gene expression. Thus, it has been demonstrated that 5'-untranslated sequences can enhance the transient expression of heterologous genes. Examples of translation enhancers, which may be mentioned, are the tobacco mosaic virus 5' leader sequence (Gallie 1987) and the like. Furthermore, they may promote tissue specificity (Rouster 1998).

The expression construct may advantageously comprise one or more enhancer sequences, linked operably to the promoter, which make possible an increased recombinant expression of the nucleic acid sequence. Additional advantageous sequences, such as further regulatory elements or terminators, may also be inserted at the 3' end of the nucleic acid sequences to be expressed recombinantly. Polyadenylation signals, which are suitable as control sequences, are plant polyadenylation signals, preferably those which essentially correspond to T-DNA polyadenylation signals from *Agrobacterium tumefaciens*, in particular the OCS (octopin synthase) terminator and the NOS (nopalin synthase) terminator.

Control sequences are furthermore understood as meaning those sequences which make possible a homologous recombination or insertion into the genome of a host organism or which permit the removal from the genome. In the case of homologous recombination, for example, the ptxA promoter may be substituted for the natural promoter of an endogenous gene. Using homologous recombination, a promoter of the invention can be placed before the target gene to be transgenically expressed (e.g., an endogenous plant gene), by linking said promoter to DNA sequences which are homologous to, for example, endogenous sequences upstream of the reading frame of the target gene. Such sequences count as genetic control sequences. After a cell has been transformed with the DNA construct in question, the two homologous sequences can interact and thus place the promoter of the invention at the desired site before the target gene so that the promoter sequence of the invention becomes operably linked to the target gene and constitutes an expression construct of the invention. The choice of the homologous sequences determines the insertion type of the promoter. In this case the expression construct can be generated by homologous recombination by means of a singly- or doubly-reciprocal recombination. In the case of the singly-reciprocal recombination, only an individual recombination sequence is used, and all of the DNA introduced is inserted. In the case of the double-reciprocal recombination, the DNA to be introduced is flanked by two homologous sequences, and the flanking region is inserted. The latter method is suitable for substituting the ptxA promoter for the natural promoter of a specific gene, as described above, and thus modifying the natural expression profile of this gene. This operable linkage constitutes an expression construct according to the invention.

Homologous recombination is a relatively rare event in higher eukaryotes, especially in plants. Random integrations into the host genome predominate. A possibility of removing the randomly integrated sequences and thus accumulating cell clones with a correct homologous recombination is the use of a sequence- specific recombination system as described in US 6,110,736.

Control sequences are furthermore to be understood as those permitting removal of the inserted sequences from the genome. Methods based on the cre/lox (Sauer 1998; Odell 1990; Dale 1991), FLP/FRT (Lysnik 1993), or Ac/Ds system (Wader 1987; US 5,225,341; Baker 1987; Lawson 1994) permit a - if appropriate tissue-specific and/or inducible - removal of a specific DNA sequence from the genome of the host organism. Control sequences may in this context mean the specific flanking sequences (*e.g*., lox sequences), which later allow removal (*e.g*., by means of cre recombinase). In this case, specific flanking sequences (lox sequences), which later allow removal by means of cre recombinase, attach to the target gene.

Furthermore, other elements having influence on the performance of an expression construct or a vector are included under the term control sequences. Such control sequences may include
a) Origins of replication, which ensure amplification of the expression constructs or vectors according to the invention in, for example, *E*. *coli.* Examples which may be mentioned are ORI (origin of DNA replication), the pBR322 ori or the P15A ori (Maniatis 1989).
b) Elements, which are necessary for *Agrobacterium*-mediated plant transformation, such as, for example, the right or left border of the T-DNA or the *vir* region.
c) Multiple cloning regions (MCS) permit and facilitate the insertion of one or more nucleic acid sequences.

Control sequences further comprise sequences, which allow for transport of the expressed protein into specific cell compartment, such as, for example, the endomembrane system, the vacuole, or the plastids (*e.g*., the chloroplasts). Desired glycosylation reactions, specific folding and the like, are possible by exploiting the secretory pathway. Alternative possibilities are the secretion of the target-protein towards the cell surface or secretion into the culture medium, for example when using cells or protoplasts grown in suspension culture. The targeting sequences required for this purpose can be incorporated into the expression construct or vector of the invention in combination with the nucleic acid sequence of interest. Target sequences, which can be used, are homologous (with respect to the nucleic acid of interest - if present) or heterologous sequences. Targeting sequences are known for subcellular localization in apoplasts, vacuole, plastids, mitochondrion, endoplasmic reticulum (ER), nucleus, elaioplasts, and other compartments. The method for the targeted transport into plastids of proteins, which *per se* are not localized in the plastids, is described (Klosgen & Weil 1991; Van Breusegem 1998).

Genetic control sequences also encompass further promoters, promoter elements or minimal promoters, all of which can modify or enhance the expression-governing characteristics. Thus, for example, the tissue-specific expression may additionally depend on certain stresses, owing to genetic control sequences. Such elements have been described, for example, for water stress, abscisic acid (Lam & Chua 1991) and thermal stress (Schöffl 1989). For example, the expression of dicotyledonous promoter can be feasible in monocotyledonous plants in combination with an intron. Such intron is fused in 5' untranslated region, in general, can or cannot be spliced during transcription, which enhances the expression (Callis 1987; Clancy & Hannah 2002; Le 2003; Lorkovic 2000; Luehrsen & Walbot 1991; McEloy & Wu US 6,429,357).

Further promoters; which make possible an expression in further plant tissues or in other organisms such as, for example, *E.coli* bacteria, may furthermore be linked operably to the nucleic acid sequence to be expressed. Suitable plant promoters are, in principle, all of the above-described promoters. For example, it is feasible that a specific nucleic acid sequence is transcribed by a promoter (for example the ptxA or SbHRGP3 promoter) as sense RNA in a plant tissue and translated into the corresponding protein, while the same nucleic acid sequence is transcribed by another promoter with another specificity in another tissue into antisense RNA and the corresponding protein is down regulated. This can be effected by an expression construct according to the invention, by positioning the first promoter before the nucleic acid sequence to be expressed recombinantly, and the other promoter there behind.

### PREFERRED NUCLEIC ACID OF INTEREST

Preferably, the transgenic expression construct of the invention to be inserted into the genome of the target plant comprises at least one expression construct, which may - for example - facilitate expression of selection markers, trait genes, antisense RNA or double-stranded RNA. Preferably said expression constructs comprise a promoter sequence functional in plant cells (either - and preferably - a promoter of the invention or another suitable promoter as for example described in the BACKGROUND FOR THE INVENTION) operatively linked to a nucleic acid sequence which - upon expression - confers an advantageous phenotype to the so transformed plant. The person skilled in the art is aware of numerous sequences which may be utilized in this context, *e.g.* to increase quality of food and feed, to produce chemicals, fine chemicals or pharmaceuticals (*e.g*., vitamins, oils, carbohydrates; Dunwell 2000), conferring resistance to herbicides, or conferring male sterility. Furthermore, growth, yield, and resistance against abiotic and biotic stress factors (like *e.g.*, fungi, viruses, nematodes, or insects) may be enhanced. Advantageous properties may be conferred either by overexpressing proteins or by decreasing expression of endogenous proteins by *e.g*., expressing a corresponding antisense (Sheehy 1988; US 4,801,340; Mol 1990) or double-stranded RNA (Matzke 2000; Fire 1998; Waterhouse 1998; WO 99/32619; WO 99/53050; WO 00/68374; WO 00/44914; WO 00/44895; WO 00/49035; WO 00/63364). Nucleic acids of interest may encode for the following (but shall not be limited to):

### 1. Selection markers

Selection markers are useful to select and separate successfully transformed or homologous recombined cells.

### 1.1 Positive selection markers

Selection markers confer a resistance to a biocidal compound such as a metabolic inhibitor (*e.g*., 2-deoxyglucose-6-phosphate, WO 98/45456), antibiotics (*e.g*., kanamycin, G 418, bleomycin or hygromycin) or herbicides (*e.g*., phosphinothricin or glyphosate). Especially preferred selection markers are those, which confer resistance to herbicides. Examples, which may be mentioned are:
- Phosphinothricin acetyltransferases (PAT; also named Bialophos ^{®}resistance; bar; de Block 1987; EP 0 333 033; US 4,975,374)
- 5-enolpyruvylshikimate-3-phosphate synthase (EPSPS) conferring resistance to Glyphosate^{®} (N-(phosphonomethyl)glycine) (Shah 1986)
- Glyphosate^{®} degrading enzymes (Glyphosate^{®} oxidoreductase; gox),
- Dalapon^{®} inactivating dehalogenases (deh)
- sulfonylurea- and imidazolinone-inactivating acetolactate synthases (for example mutated ALS variants with, for example, the S4 and/or Hra mutation
- Bromoxynil^{®} degrading nitrilases (bxn)
- Kanamycin- or. G418- resistance genes (NPTII; NPTI) coding *e.g*., for neomycin phosphotransferases (Fraley 1983)
- 2-Desoxyglucose-6-phosphate phosphatase (DOG^{R}1-Gene product; WO 98/45456; EP 0 807 836) conferring resistance against 2-desoxyglucose (Randez-Gil 1995).
- hygromycin phosphotransferase (HPT), which mediates resistance to hygromycin (Vanden Elzen 1985).
- dihydrofolate reductase (Eichholtz 1987)

Additional positive selectable marker genes of bacterial origin that confer resistance to antibiotics include the aadA gene, which confers resistance to the antibiotic spectinomycin, gentamycin acetyl transferase, streptomycin phosphotransferase (SPT), aminoglycoside-3-adenyl transferase and the bleomycin resistance determinant (Hayford 1988; Jones 1987; Svab 1990; Hille 1986).

Genes like isopentenyltransferase from *Agrobacterium tumefaciens* (strain:PO22; Genbank Acc.-No.: AB025109) may - as a key enzyme of the cytokinin biosynthesis - facilitate regeneration of transformed plants (*e.g*., by selection on cytokinin-free medium). Corresponding selection methods are described (Ebinuma 2000a; Ebinuma 2000b). Additional positive selection markers, which confer a growth advantage to a transformed plant in comparison with a non-transformed one, are described *e.g*., in EP-A 0 601 092. Growth stimulation selection markers may include (but shall not be limited to) β-glucuronidase (in combination with *e.g.,* a cytokinin glucuronide), mannose-6-phosphate isomerase (in combination with mannose), UDP-galactose-4-epimerase (in combination with *e.g*., galactose), wherein mannose-6-phosphate isomerase in combination with mannose is especially preferred.

### 1.2) Negative selection markers

Negative selection markers are especially suitable to select organisms with defined deleted sequences comprising said marker (Koprek 1999). Examples for negative selection marker comprise thymidin kinases (TK), cytosine deaminases (Gleave 1999; Perera1993; Stougaard 1993), cytochrom P450 proteins (Koprek 1999), haloalkan dehalogenases (Naested 1999), iaaH gene products (Sundaresan 1995), cytosine deaminase codA (Schlaman & Hooykaas 1997), or tms2 gene products (Fedoroff & Smith 1993).

### 2) Reporter genes

Reporter genes encode readily quantifiable proteins and, via their color or enzyme activity, make possible an assessment of the transformation efficacy, the site of expression or the time of expression. Very especially preferred in this context are genes encoding reporter proteins (Schenbom 1999) such as the green fluorescent protein (GFP) (Sheen 1995; Haseloff 1997; Reichel 1996; Tian 1997; WO 97/41228; Chui 1996; Leffel 1997), chloramphenicol transferase, a luciferase (Ow 1986; Millar 1992), the aequorin gene (Prasher 1985), β-galactosidase, R locus gene (encoding a protein which regulates the production of anthocyanin pigments (red coloring) in plant tissue and thus makes possible the direct analysis of the promoter activity without addition of further auxiliary substances or chromogenic substrates (Dellaporta 1988; Ludwig 1990), with β-glucuronidase (GUS) being very especially preferred (Jefferson 1987a,b). β-glucuronidase (GUS) expression is detected by a blue color on incubation of the tissue with 5-bromo-4-chloro-3-indolyl-β-D-glucuronic acid, bacterial luciferase (LUX) expression is detected by light emission; firefly luciferase (LUC) expression is detected by light emission after incubation with luciferin; and galactosidase expression is detected by a bright blue color after the tissue is stained with 5-bromo-4-chloro-3-indolyl-β-D-galactopyranoside. Reporter genes may also be used as scorable markers as alternatives to antibiotic resistance markers. Such markers are used to detect the presence or to measure the level of expression of the transferred gene. The use of scorable markers in plants to identify or tag genetically modified cells works well only when efficiency of modification of the cell is high.

The skilled worker is familiar with a multiplicity of nucleic acids of interest (or proteins encoded thereby) whose transgenic expression is advantageous. The skilled worker is furthermore familiar with a multiplicity of genes by whose repression or silencing by means of expression of a corresponding antisense or double stranded RNA advantageous effects may also be achieved. The following may be mentioned by way of example, but not by way of limitation, as advantageous effects:
- Obtaining a resistance to abiotic stresses (high and low temperatures, drought, increased humidity, environmental toxins, UV radiation)
- Obtaining a resistance to biotic stresses (pathogens, viruses, insects and diseases)
- Obtaining resistance against phytotoxic substances or herbicides
- Improving the growth rate or the yield.

The following may be mentioned by way of example but not by way of limitation as nucleic acid sequences or polypeptides which can be used for these applications:
1. Improved protection of the plant embryo against abiotic stresses such as drought, high or low temperatures, for example by overexpressing the antifreeze polypeptides from *Myoxocephalus scorpius* (WO 00/00512), *Myoxocephalus octodecemspinosus,* the *Arabidopsis thaliana* transcription activator CBF1, glutamate dehydrogenases (WO 97/12983, WO 98/11240), a late embryogenesis gene (LEA), for example from barley (WO 97/13843), calcium-dependent protein kinase genes (WO 98/26045), calcineurins (WO 99/05902), farnesyl transferases (WO 99/06580, Pei 1998), ferritin (Deak 1999), oxalate oxidase (WO 99/04013; Dunwell 1998), DREB1A factor (dehydration response element B 1A; Kasuga 1999), mannitol or trehalose synthesis genes, such as trehalose-phosphate synthase or trehalose-phosphate phosphatase (WO 97/42326), or by inhibiting genes such as the trehalase gene (WO 97/50561). Especially preferred nucleic acids are those which encode the transcriptional activator CBF1 from *Arabidopsis thaliana* (GenBank Acc. No.: U77378) or the *Myoxocephalus octodecemspinosus* antifreeze protein (GenBank Acc. No.: AF306348), or functional equivalents of these.
2. Obtaining resistance for example against fungi, insects, nematodes and diseases by the targeted secretion or concentration of specific metabolites or proteins in the embryol epidermis. Examples which may be mentioned are glucosinolates (defence against herbivores), chitinases or glucanases and other enzymes which destroy the cell wall of parasites, ribosome-inactivating proteins (RIPs) and other proteins of the plant's resistance and stress response as are induced upon wounding or microbial attack of plants or chemically by, for example, salicylic acid, jasmonic acid or ethylene; lysozymes from nonplant sources such as, for example, T4 lysozyme or lysozyme from a variety of mammals, insecticidal proteins such as *Bacillus thuringiensis* endotoxin, α-amylase inhibitor or protease inhibitors (cowpea trypsin inhibitor), glucanases, lectins such as phytohemagglutinin, snowdrops lectin, wheat-germ agglutinin, RNAses or ribozymes. Nucleic acids which are especially preferred are those which encode the *Trichoderma harzianum* chit42 endochitinase (GenBank Acc. No.: S78423) or the *Sorghum bicolor* N-hydroxylating multifunctional cytochrome P-450 (CYP79) proteins (GenBank Acc. No.: U32624), or functional equivalents of these.

The transgenic expression constructs of the invention can be employed for suppressing or reducing expression of endogenous target genes by "gene silencing". Preferred genes or proteins whose suppression brings about an advantageous phenotype are known to the skilled worker. Examples may include but are not limited to down-regulation of the β-subunit of *Arabidopsis* G protein for increasing root mass (Ullah *et al.* 2003), inactivating cyclic nucleotide-gated ion channel (CNGC) for improving disease resistance (WO 2001007596), and down-regulation of 4-coumarate-CoA ligase (4CL) gene for altering lignin and cellulose contents (US 2002138870).

Gene silencing can be realized by antisense or double-stranded RNA or by co-suppression (sense-suppression). An "antisense" nucleic acid is firstly understood as meaning a nucleic acid sequence, which is fully or partially complementary to at least part of the "sense" strand of said target protein. The skilled worker knows that he can use alternative cDNA or the corresponding gene as starting template for suitable antisense constructs. The "antisense" nucleic acid is preferably complementary to the coding region of the target protein or part thereof. However, the "antisense" nucleic acid may also be complementary to the non-coding region or part thereof. Starting from the sequence information on a target protein, an antisense nucleic acid can be designed in the manner with which the skilled worker is familiar, taking into consideration Watson's and Crick's rules of base pairing. An antisense nucleic acid can be complementary to the entire or part of the nucleic acid sequence of a target protein.

Likewise encompassed is the use of the above-described sequences in sense orientation, which, as is known to the skilled worker, can lead to co-suppression (sense-suppression). It has been demonstrated that expression of sense can reduce or switch off expression of same, analogously to what has been described for antisense approaches (Goring 1991; Smith 1990; Napoli 1990;Van der Krol1990). In this context, the construct introduced may represent the gene to be reduced fully or only in part. The possibility of translation is not necessary.

Especially preferred is the use of gene regulation methods by means of double-stranded RNAi ("double-stranded RNA interference"). Such methods are known to the person skilled in the art (*e.g*., Matzke 2000; Fire 1998; WO 99/32619; WO 99/53050; WO 00/68374; WO 00/44914; WO 00/44895; WO 00/49035; WO 00/63364). The processes and methods described in the references stated are expressly referred to.

Furthermore, artificial transcription factors (*e.g*. of the zinc finger protein type; Beerli 2000) can be expressed under control of a promoter of the invention to modulate expression of specific endogenous genes. These factors attach to the regulatory regions of the endogenous genes to be expressed or to be repressed and, depending on the design of the factor, bring about expression or repression of the endogenous gene.

### TARGET ORGANISM

Another subject matter of the invention relates to transgenic organisms transformed with at least one transgenic expression construct or vector of the invention, and to cells, cell cultures, tissues, organs (*e.g*., leaves, roots and the like in the case of plant organisms), or propagation material derived from such organisms.

The terms "organism", "target organism" or "host organism" are preferably understood as meaning prokaryotic or eukaryotic organisms, such as, for example, microorganisms or plant organisms. Preferred microorganisms are bacteria, yeasts, algae or fungi.

Preferred bacteria are bacteria of the genus *Escherichia, Erwinia*, *Agrobacterium*, *Flavobacterium*, *Alcaligenes or cyanobacteria*, for example of the genus *Synechocystis.* Especially preferred are microorganisms which are capable of infecting plants and thus of transferring the constructs according to the invention. Preferred microorganisms are those from the genus *Agrobacterium* and, in particular, the species *Agrobacterium tumefaciens*.

Preferred yeasts are *Candida, Saccharomyces, Hansenula or Pichia.* Preferred fungi are *Aspergillus*, *Trichoderma*, *Ashbya*, *Neurospora*, *Fusarium*, *Beauveria* or other fungi. Plant organisms are furthermore, for the purposes of the invention, other organisms which are capable of photosynthetic activity such as, for example, algae or cyanobacteria, and also mosses. Preferred algae are green algae such as, for example, algae of the genus *Haematococcus, Phaedactylum tricomatum, Volvox* or *Dunaliella.*

Host or target organisms, which are preferred as transgenic organisms, are especially plants. Included within the scope of the invention are all genera and species of higher and lower plants of the plant kingdom. Included are furthermore the mature plants, seeds, shoots and seedlings and parts, propagation material and cultures derived therefrom, for example cell cultures. The term "mature plants" is understood as meaning plants at any developmental stage beyond the seedling. The term "seedling" is understood as meaning a young, immature plant in an early developmental stage.

Annual, biennial, monocotyledonous and dicotyledonous plants are preferred host organisms for the generation of transgenic plants. The expression of genes is furthermore advantageous in all ornamental plants, useful or ornamental trees, flowers, cut flowers, shrubs or lawns. Plants which may be mentioned by way of example but not by limitation are angiosperms, bryophytes such as, for example, *Hepaticae* (liverworts) and *Musci* (mosses); *Pteridophytes* such as ferns, horsetail and club mosses; gymno-sperms such as conifers, cycads, ginkgo and *Gnetatae*; algae such as *Chlorophyceae*, *Phaeophpyceae, Rhodophyceae, Myxophyceae, Xanthophyceae, Bacillariophyceae* (diatoms) and *Euglenophyceae*.

Preferred are plants which are used for food or feed purpose such as the families of the *Leguminosae* such as pea, alfalfa and soya; *Gramineae* such as rice, maize, wheat, barley, sorghum, millet, rye, triticale, or oats; the family of the *Umbelliferae*, especially the genus *Daucus*, very especially the species *carota* (carrot) and *Apium,* very especially the species *Graveolens dulce* (celery) and many others; the family of the *Solana*ceae, especially the genus *Lycopersicon,* very especially the species *esculentum* (tomato) and the genus *Solanum,* very especially the species *tuberosum* (potato) and *melongena* (egg plant), and many others (such as tobacco); and the genus *Capsicum,* very especially the species *annuum* (peppers) and many others; the family of the *Leguminosae*, especially the genus *Glycine*, very especially the species *max* (soybean), alfalfa, pea, lucerne, beans or peanut and many others; and the family of the *Cruciferae* (*Brassicacae*), especially the genus *Brassica,* very especially the species *napus* (oil seed rape), *campestris* (beet), *oleracea* cv Tastie (cabbage), *oleracea* cv Snowball Y (cauliflower) and *oleracea* cv Emperor (broccoli); and of the genus *Arabidopsis,* very especially the species *thaliana* and many others; the family of the *Compositae*, especially the genus *Lactuca,* very especially the species *sativa* (lettuce) and many others; the family of the *Asteraceae* such as sunflower, Tagetes, lettuce or Calendula and many other, the family of the *Cucurbitaceae* such as melon, pumpkin/squash or zucchini, and linseed. Further preferred are cotton, sugar cane, hemp, flax, chillies, and the various tree, nut and wine species.

Very especially preferred are *Arabidopsis thaliana*, *Nicotiana tabacum*, *Tagetes erecta*, *Calendula officinalis*, *Gycine max*, *Zea mays, Oryza sativa, Triticum aestivum, Pisum sativum, Phaseolus vulgaris, Hordeum vulgare, Brassica napus.*

### TRANSGENIC EXPRESSION VECTORS

An expression construct according to the invention can advantageously be introduced into cells, preferably into plant cells, using vectors. In an advantageous embodiment, the expression construct is introduced by means of plasmid vectors. In one embodiment, the methods of the invention involve transformation of organism or cells (*e.g*. plants or plant cells) with a transgenic expression vector comprising at least a transgenic expression cassette of the invention (as described above). As used herein, the terms "vector" and "vehicle" are used interchangeably in reference to nucleic acid molecules that transfer DNA segment(s) from one cell to another. The term "expression vector" as used herein refers to a recombinant DNA molecule containing a desired coding sequence and appropriate nucleic acid sequences necessary for the expression of the operably linked coding sequence in a particular host organism.

The methods of the invention are not limited to the expression vectors disclosed herein. Any expression vector, which is capable of introducing a nucleic acid sequence of interest into a plant cell, is contemplated to be within the scope of this invention. Typically, expression vectors comprise the transgenic expression cassette of the invention in combination with elements, which allow cloning of the vector into a bacterial or phage host. The vector preferably, though not necessarily, contains an origin of replication, which is functional in a broad range of prokaryotic hosts. A selectable marker is generally, but not necessarily, included to allow selection of cells bearing the desired vector.

Examples of vectors may be plasmids, cosmids, phages, viruses or *Agrobacteria.* More specific examples are given below for the individual transformation technologies.

Preferred are those vectors, which make possible a stable integration of the expression construct into the host genome. In the case of injection or electroporation of DNA into plant cells, the plasmid used need not meet any particular requirements. Simple plasmids such as those of the pUC series can be used. If intact plants are to be regenerated from the transformed cells, it is necessary for an additional selectable marker gene to be present on the plasmid. A variety of possible plasmid vectors are available for the introduction of foreign genes into plants, and these plasmid vectors contain, as a rule, a replication origin for multiplication in *E.coli* and a marker gene for the selection of transformed bacteria. Examples are pBR322, pUC series, M13mp series, pA-CYC184 and the like.

The expression construct can be introduced into the vector via a suitable restriction cleavage site. The plasmid formed is first introduced into *E*.*coli*. Correctly transformed *E.coli* are selected and grown, and the recombinant plasmid is obtained by methods known to the skilled worker. Restriction analysis and sequencing can be used for verifying the cloning step.

Depending on the method by which DNA is introduced, further genes may be necessary on the vector plasmid.

*Agrobacterium tumefaciens* and *A. rhizogenes* are plant-pathogenic soil bacteria, which genetically transform plant cells. The Ti and Ri plasmids of *A. tumefaciens* and *A*. *rhizogenes,* respectively, carry genes responsible for genetic transformation of the plant (Kado 1991). Vectors of the invention may be based on the *Agrobacterium* Ti- or Ri-plasmid and may thereby utilize a natural system of DNA transfer into the plant genome.

As part of this highly developed parasitism *Agrobacterium* transfers a defined part of its genomic information (the T-DNA; flanked by about 25 bp repeats, named left and right border) into the chromosomal DNA of the plant cell (Zupan 2000). By combined action of the so-called *vir* genes (part of the original Ti-plasmids) said DNA-transfer is mediated. For utilization of this natural system, Ti-plasmids were developed which lack the original tumor inducing genes ("disarmed vectors"). In a further improvement, the so called "binary vector systems", the T-DNA was physically separated from the other functional elements of the Ti-plasmid (*e.g*., the *vir* genes), by being incorporated into a shuttle vector, which allowed easier handling (EP-A 120 516; US 4.940.838). These binary vectors comprise (beside the disarmed T-DNA with its border sequences), prokaryotic sequences for replication both in *Agrobacterium* and *E*. *coli.* It is an advantage of *Agrobacterium-mediated* transformation that in general only the DNA flanked by the borders is transferred into the genome and that preferentially only one copy is inserted. Descriptions of *Agrobacterium* vector systems and methods for *Agrobacterium-*mediated gene transfer are known in the art (Miki 1993; Gruber 1993; Moloney 1989). The use of T-DNA for the transformation of plant cells has been studied and described intensively (EP 120516; Hoekema 1985; Fraley 1985; and An 1985). Various binary vectors are known, some of which are commercially available such as, for example, pBIN19 (Clontech Laboratories, Inc. U.S.A.).

Hence, for *Agrobacteria*-mediated transformation the transgenic expression construct of the invention is integrated into specific plasmids, either into a shuttle or intermediate vector, or into a binary vector. If a Ti or Ri plasmid is to be used for the transformation, at least the right border, but in most cases the right and left border, of the Ti or Ri plasmid T-DNA is linked to the transgenic expression construct to be introduced in the form of a flanking region. Binary vectors are preferably used. Binary vectors are capable of replication both in *E.coli* and in *Agrobacterium.* They may comprise a selection marker gene and a linker or polylinker (for insertion of e.g. the expression construct to be transferred) flanked by the right and left T-DNA border sequence. They can be transferred directly into *Agrobacterium* (Holsters 1978). The selection marker gene permits the selection of transformed *Agrobacteria* and is, for example, the *npt*II gene, which confers resistance to kanamycin. The *Agrobacterium,* which acts as host organism in this case, should already contain a plasmid with the *vir* region. The latter is required for transferring the T-DNA to the plant cell. An *Agrobacterium* transformed in this way can be used for transforming plant cells. The use of T-DNA for transforming plant cells has been studied and described intensively (EP 120 516; Hoekema 1985; An 1985; see also below).

Common binary vectors are based on "broad host range"-plasmids like pRK252 (Bevan 1984) or pTJS75 (Watson 1985) derived from the P-type plasmid RK2. Most of these vectors are derivatives of pBIN19 (Bevan 1984). Various binary vectors are known, some of which are commercially available such as, for example, pBI101.2 or pBIN19 (Clontech Laboratories, Inc. USA). Additional vectors were improved with regard to size and handling (*e.g*. pPZP; Hajdukiewicz 1994). Improved vector systems are described also in WO 02/00900.

In a preferred embodiment, *Agrobacterium* strains for use in the practice of the invention include octopine strains, *e.g*., LBA4404 or agropine strains, *e.g*., EHA101 or EHA105. Suitable strains of A. tumefaciens for DNA transfer are for example EHA101pEHA101 (Hood1986), EHA105[pEHA105] (Li 1992), LBA4404[pAL4404] (Hoekema 1983), C58C1[pMP90] (Koncz 1986), and C58C1 [pGV2260] (Deblaere 1985). Other suitable strains are *Agrobacterium tumefaciens* C58, a nopaline strain. Other suitable strains are *A. tumefaciens* C58C1 (Van Larebeke 1974), A136 (Watson 1975) or LBA4011 (Klapwijk 1980). In a preferred embodiment, the *Agrobacterium* strain used to transform the plant tissue pre-cultured with the plant phenolic compound contains a L,L-succinamopine type Ti-plasmid, preferably disarmed, such as pEHA101. In another preferred embodiment, the *Agrobacterium* strain used to transform the plant tissue pre-cultured with the plant phenolic compound contains an octopine-type Ti-plasmid, preferably disarmed, such as pAL4404. Generally, when using octopine-type Ti-plasmids or helper plasmids, it is preferred that the *virF* gene be deleted or inactivated (Jarschow 1991). In a preferred embodiment, the *Agrobacterium* strain used to transform the plant tissue pre-cultured with the plant phenolic compound such as acetosyringone. The method of the invention can also be used in combination with particular *Agrobacterium* strains, to further increase the transformation efficiency, such as *Agrobacterium* strains wherein the *vir* gene expression and/or induction thereof is altered due to the presence of mutant or chimeric *virA* or *virG* genes (*e.g*. Hansen 1994; Chen 1991; Scheeren-Groot 1994).

A binary vector or any other vector can be modified by common DNA recombination techniques, multiplied in *E*. *coli*, and introduced into *Agrobacterium* by *e.g*., electroporation or other transformation techniques (Mozo 1991). *Agrobacterium* is grown and used as described in the art. The vector comprising *Agrobacterium* strain may, for example, be grown for 3 days on YP medium (5 g/L yeast extract, 10 g/L peptone, 5 g/L Nail, 15 g/L agar, pH 6.8) supplemented with the appropriate antibiotic (*e.g*., 50 mg/L spectinomycin). Bacteria are collected with a loop from the solid medium and resuspended.

### TRANFORMATION TECHNIQUES

The generation of a transformed organism or a transformed cell requires introducing the DNA in question into the host cell in question. A multiplicity of methods is available for this procedure, which is termed transformation (see also Keown (1990) Methods in Enzymology 185:527-537). For example, the DNA can be introduced directly by microinjection or by bombardment via DNA-coated microparticles. Also, the cell can be permeabilized chemically, for example using polyethylene glycol, so that the DNA can enter the cell by diffusion. The DNA can also be introduced by protoplast fusion with other DNA-containing units such as minicells, cells, lysosomes or liposomes. Another suitable method of introducing DNA is electroporation, where the cells are permeabilized reversibly by an electrical pulse.

Methods for introduction of a transgenic expression construct or vector into plant tissue may include but are not limited to, *e.g*., electroinjection (Nan 1995; Griesbach 1992); fusion with liposomes, lysosomes, cells, minicells or other fusible lipid-surfaced bodies (Fraley 1982); polyethylene glycol (Krens 1982); chemicals that increase free DNA uptake; transformation using virus, and the like. Furthermore, the biolistic method with the gene gun, electroporation, incubation of dry embryos in DNA-containing solution, and microinjection may be employed.

Protoplast based methods can be employed (*e.g*., for rice), where DNA is delivered to the protoplasts through liposomes, PEG, or electroporation (Shimamoto 1989; Datta 1990b). Transformation by electroporation involves the application of short, high-voltage electric fields to create "pores" in the cell membrane through which DNA is taken-up. These methods are - for example - used to produce stably transformed monocotyledonous plants (Paszkowski 1984; Shillito 1985; Fromm 1986) especially from rice (Shimamoto 1989; Datta 1990b; Hayakawa 1992).

Particle bombardment or "biolistics" is a widely used method for the transformation of plants, especially monocotyledonous plants. In the "biolistics" (microprojectile-mediated DNA delivery) method microprojectile particles are coated with DNA and accelerated by a mechanical device to a speed high enough to penetrate the plant cell wall and nucleus (WO 91/02071). The foreign DNA gets incorporated into the host DNA and results in a transformed cell. There are many variations on the "biolistics" method (Sanford 1990; Fromm 1990; Christou 1988; Sautter 1991). The method has been used to produce stably transformed monocotyledonous plants including rice, maize, wheat, barley, and oats (Christou 1991; Gordon-Kamm 1990; Vasil 1992, 1993; Wan 1994; Sommers 1992).

In addition to these "direct" transformation techniques, transformation can also be effected by bacterial infection by means of *Agrobacterium tumefaciens* or *Agrobacterium rhizogenes.* These strains contain a plasmid (Ti or Ri plasmid) which is transferred to the plant following *Agrobacterium* infection. Part of this plasmid, termed T-DNA (transferred DNA), is integrated into the genome of the plant cell (see above for description of vectors). To transfer the DNA to the plant cell, plant explants are cocultured with a transgenic *Agrobacterium tumefaciens* or *Agrobacterium rhizogenes.* Starting from infected plant material (for example leaf, root or stem sections, but also protoplasts or suspensions of plant cells), intact plants can be generated using a suitable medium which may contain, for example, antibiotics or biocides for selecting transformed cells. The plants obtained can then be screened for the presence of the DNA introduced, in this case the expression construct according to the invention. As soon as the DNA has integrated into the host genome, the genotype in question is, as a rule, stable and the insertion in question is also found in the subsequent generations. As a rule, the expression construct integrated contains a selection marker which imparts a resistance to a biocide (for example a herbicide) or an antibiotic such as kanamycin, G 418, bleomycin, hygromycin or phosphinotricin and the like to the transformed plant. The selection marker permits the selection of transformed cells from untransformed cells (McCormick 1986). The plants obtained can be cultured and hybridized in the customary fashion. Two or more generations should be grown in order to ensure that the genomic integration is stable and hereditary. The abovementioned methods are described (for example, in Jenes 1983; and in Potrykus 1991).

One of skill in the art knows that the efficiency of transformation by *Agrobacterium* may be enhanced by using a number of methods known in the art. For example, the inclusion of a natural wound response molecule such as acetosyringone (AS) to the *Agrobacterium* culture has been shown to enhance transformation efficiency with *Agrobacterium tumefaciens* (Shahla 1987). Alternatively, transformation efficiency may be enhanced by wounding the target tissue to be transformed. Wounding of plant tissue may be achieved, for example, by punching, maceration, bombardment with microprojectiles, *etc.* (see, *e.g.,* Bidney 1992).

A number of other methods have been reported for the transformation of plants (especially monocotyledonous plants) including, for example, the "pollen tube method" (WO 93/18168; Luo 1988), macro-injection of DNA into floral tillers (Du 1989; De la Pena 1987), injection of *Agrobacterium* into developing caryopses (WO 00/63398), and tissue incubation of seeds in DNA solutions (Töpfer 1989). Direct injection of exogenous DNA into the fertilized plant ovule at the onset of embryogenesis was disclosed in WO 94/00583. WO 97/48814 disclosed a process for producing stably transformed fertile wheat and a system of transforming wheat via *Agrobacterium* based on freshly isolated or pre-cultured immature embryos, embryogenic callus and suspension cells.

It may be desirable to target the nucleic acid sequence of interest to a particular locus on the plant genome. Site-directed integration of the nucleic acid sequence of interest into the plant cell genome may be achieved by, for example, homologous recombination using *Agrobacterium*-derived sequences. Generally, plant cells are incubated with a strain of *Agrobacterium* which contains a targeting vector in which sequences that are homologous to a DNA sequence inside the target locus are flanked by *Agrobacterium* transfer-DNA (T-DNA) sequences, as previously described (US 5,501,967, the entire contents of which are herein incorporated by reference). One of skill in the art knows that homologous recombination may be achieved using targeting vectors, which contain sequences that are homologous to any part of the targeted plant gene, whether belonging to the regulatory elements of the gene, or the coding regions of the gene. Homologous recombination may be achieved at any region of a plant gene so long as the nucleic acid sequence of regions flanking the site to be targeted is known.

Where homologous recombination is desired, the targeting vector used may be of the replacement- or insertion-type (US 5,501,967; supra). Replacement-type vectors generally contain two regions which are homologous with the targeted genomic sequence and which flank a heterologous nucleic acid sequence, *e.g.*, a selectable marker gene sequence. Replacement-type vectors result in the insertion of the selectable marker gene which thereby disrupts the targeted gene. Insertion-type vectors contain a single region of homology with the targeted gene and result in the insertion of the entire targeting vector into the targeted gene.

### SELECTION OF TRANSGENIC CELLS

Transformed cells, *i.e.* those which contain the introduced DNA integrated into the DNA of the host cell, can be selected from untransformed cells if a selectable marker is part of the introduced DNA. A selection marker gene may confer positive or negative selection.

A positive selection marker gene may be used in constructs for random integration and site-directed integration. Positive selection marker genes include antibiotic resistance genes, and herbicide resistance genes and the like. Transformed cells, which express such a marker gene, are capable of surviving in the presence of concentrations of the antibiotic or herbicide in question which kill an untransformed wild type. Examples are the bar gene, which imparts resistance to the herbicide phosphinotricin (bialaphos; Vasil1992; Weeks1993; Rathore 1993), the *npt*II gene, which imparts resistance to kanamycin, the *hpt* gene, which imparts resistance to hygromycin, or the EPSP gene, which imparts resistance to the herbicide glyphosate, geneticin (G-418) (aminoglycoside) (Nehra1994), glyphosate (Della-Cioppa1987) and the ALS gene (chlorsulphuron resistance). Further preferred selectable and screenable marker genes are disclosed above.

A negative selection marker gene may also be included in the constructs. The use of one or more negative selection marker genes in combination with a positive selection marker gene is preferred in constructs used for homologous recombination. Negative selection marker genes are generally placed outside the regions involved in the homologous recombination event. The negative selection marker gene serves to provide a disadvantage (preferably lethality) to cells that have integrated these genes into their genome in an expressible manner. Cells in which the targeting vectors for homologous recombination are randomly integrated in the genome will be harmed or killed due to the presence of the negative selection marker gene. Where a positive selection marker gene is included in the construct, only those cells having the positive selection marker gene integrated in their genome will survive. The choice of the negative selection marker gene is not critical to the invention as long as it encodes a functional polypeptide in the transformed plant cell. The negative selection gene may for instance be chosen from the aux-2 gene from the Ti-plasmid of *Agrobacterium,* the tk-gene from SV40, cytochrome P450 from *Streptomyces griseolus*, the *Adh* gene from Maize or *Arabidopsis*, *etc*. Any gene encoding an enzyme capable of converting a substance, which is otherwise harmless to plant cells, into a substance, which is harmful to plant cells, may be used. Further preferred negative selection markers are disclosed above.

However, insertion of an expression cassette or a vector into the chromosomal DNA can also be demonstrated and analyzed by various other methods (not based on selection marker) known in the art like including, but not limited to, restriction mapping of the genomic DNA, PCR-analysis, DNA-DNA hybridization, DNA-RNA hybridization, DNA sequence analysis and the like. More specifically such methods may include *e.g*., PCR analysis, Southern blot analysis, fluorescence *in situ* hybridization (FISH), and *in situ* PCR.

### REGENERATION OF TRANSGENIC ORGANISM

As soon as a transformed plant cell has been generated, an intact plant can be obtained using methods known to the skilled worker. Accordingly, the present invention provides transgenic plants. The transgenic plants of the invention are not limited to plants in which each and every cell expresses the nucleic acid sequence of interest under the control of the promoter sequences provided herein. Included within the scope of this invention is any plant, which contains at least one cell, which expresses the nucleic acid sequence of interest (*e.g*., chimeric plants). It is preferred, though not necessary, that the transgenic plant comprises the nucleic acid sequence of interest in more than one cell, and more preferably in one or more tissue.

Once transgenic plant tissue, which contains an expression vector, has been obtained, transgenic plants may be regenerated from this transgenic plant tissue using methods known in the art. The term "regeneration" as used herein, means growing a whole plant from a plant cell, a group of plant cells, a plant part or a plant piece (e.g., from a protoplast, callus, protocorm-like body, or tissue part).

Species from the following examples of genera of plants may be regenerated from transformed protoplasts: *Fragaria*, *Lotus*, *Medicago*, *Onobrychis*, *Trifolium*, *Trigonella*, *Vigna, Citrus, Linum, Geranium, Manihot, Daucus, Arabidopsis, Brassica, Raphanus, Sinapis, Atropa, Capsicum, Hyoscyamus, Lycopersicon, Nicotiana, Solanum, Petunia, Digitalis, Majorana, Ciohorium, Helianthus, Lactuca, Bromus, Asparagus, Antirrhinum*, *Hererocallis, Nemesia, Pelargonium, Panicum, Pennisetum, Ranunculus, Senecio, Salpiglossis*, *Cucumis*, *Browaalia*, *Glycine*, *Pisum*, *Lolium*, *Zea*, *Triticum*, *Sorghum*, and *Datura.*

For regeneration of transgenic plants from transgenic protoplasts, a suspension of transformed protoplasts or a Petri plate containing transformed explants is first provided. Callus tissue is formed and shoots may be induced from callus and subsequently rooted. Alternatively, somatic embryo formation can be induced in the callus tissue. These somatic embryos germinate as natural embryos to form plants. The culture media will generally contain various amino acids and plant hormones, such as auxin and cytokinins. It is also advantageous to add glutamic acid and proline to the medium, especially for such species as corn and alfalfa. Efficient regeneration will depend on the medium, on the genotype, and on the history of the culture. These three variables may be empirically controlled to result in reproducible regeneration.

Plants may also be regenerated from cultured cells or tissues. Dicotyledonous plants which have been shown capable of regeneration from transformed individual cells to obtain transgenic whole plants include, for example, apple (*Malus pumila*), blackberry (*Rubus*), Blackberry/raspberry hybrid (*Rubus*), red raspberry (*Rubus*), carrot (*Daucus carota*), cauliflower (*Brassica oleracea*), celery (*Apium graveolens*), cucumber (*Cucumis sativus*), eggplant (*Solanum melongena*), lettuce (*Lactuca sativa*), potato (*Solanum tuberosum*), rape (*Brassica napus*), wild soybean (*Glycine canescens*), strawberry (*Fragaria ananassa*), tomato (*Lycopersicon esculentum*), walnut (*Juglans regia*), melon (*Cucumis melo*), grape (*Vitis vinifera*), and mango (*Mangifera indica*). Monocotyledonous plants, which have been shown capable of regeneration from transformed individual cells to obtain transgenic whole plants, include, for example, rice (*Oryza sativa*), rye (*Secale cereale*), and maize (*Zea mays*).

In addition, regeneration of whole plants from cells (not necessarily transformed) has also been observed in: apricot (*Prunus armeniaca*), asparagus (*Asparagus officinalis*), banana (hybrid *Musa*), bean (*Phaseolus vulgaris*), cherry (hybrid *Prunus*), grape (*Vitis vinifera*), mango (*Mangifera indica*), melon (*Cucumis melo*), ochra (*Abelmoschus esculentus*), onion (hybrid *Allium*), orange (*Citrus sinensis*), papaya (*Carica papaya*), peach (*Prunus persica*), plum (*Prunus domestica*), pear (*Pyrus communis*), pineapple (*Ananas comosus*), watermelon (*Citrullus vulgaris*), and wheat (*Triticum aestivum*).

The regenerated plants are transferred to standard soil conditions and cultivated in a conventional manner. After the expression vector is stably incorporated into regenerated transgenic plants, it can be transferred to other plants by vegetative propagation or by sexual crossing. For example, in vegetatively propagated crops, the mature transgenic plants are propagated by the taking of cuttings or by tissue culture techniques to produce multiple identical plants. In seed propagated crops, the mature transgenic plants are self crossed to produce a homozygous inbred plant which is capable of passing the transgene to its progeny by Mendelian inheritance. The inbred plant produces seed containing the nucleic acid sequence of interest. These seeds can be grown to produce plants that would produce the selected phenotype. The inbred plants can also be used to develop new hybrids by crossing the inbred plant with another inbred plant to produce a hybrid.

Confirmation of the transgenic nature of the cells, tissues, and plants may be performed by PCR analysis, antibiotic or herbicide resistance, enzymatic analysis and/or Southern blots to verify transformation. Progeny of the regenerated plants may be obtained and analyzed to verify whether the transgenes are heritable. Heritability of the transgene is further confirmation of the stable transformation of the transgene in the plant. The resulting plants can be bred in the customary fashion. Two or more generations should be grown in order to ensure that the genomic integration is stable and hereditary. Corresponding methods are described (Jenes 1993; Potrykus 1991).

Also in accordance with the invention are cells, cell cultures, tissues, parts, organs,- such as, for example, roots, leaves and the like in the case of transgenic plant organisms - derived from the above-described transgenic organisms, and transgenic propagation material such as seeds or fruits.

Genetically modified plants according to the invention, which can be consumed by humans or animals, can also be used as food or feedstuffs, for example directly or following processes known *per se.*

A further subject matter of the invention relates to the use of the above-described transgenic organisms according to the invention and the cells, cell cultures, parts, tissues, organs- such as, for example, roots, leaves and the like in the case of transgenic plant organisms - derived from them, and transgenic propagation material such as seeds or fruits, for the production of foods or feedstuffs, pharmaceuticals or fine chemicals.

Preferred is furthermore a method for the recombinant production of pharmaceuticals or fine chemicals in host organisms, where a host organism is transformed with one of the above-described expression constructs, and this expression construct contains one or more structural genes which encode the desired fine chemical or catalyze the biosynthesis of the desired fine chemical, the transformed host organism is cultured, and the desired fine chemical is isolated from the culture medium. This process can be used widely for fine chemicals such as enzymes, vitamins, amino acids, sugars, fatty acids, natural and synthetic flavorings, aroma substances and colorants. Especially preferred is the production of tocopherols and tocotrienols, carotenoids, oils, polyunsaturated fatty acids etc. Culturing the transformed host organisms, and isolation from the host organisms or the culture medium, is performed by methods known to the skilled worker. The production of pharmaceuticals such as, for example, antibodies, vaccines, enzymes or pharmaceutically active proteins is described (Hood 1999;Ma 1999; Russel 1999; Cramer 1999; Gavilondo 2000; Holliger 1999).

### Sequences

1. SEQ ID NO: 1 Nucleic acid sequence encoding the ptxA promoter (including the 5' untranslated region of the ptxA gene)
2. SEQ ID NO: 2 Nucleic acid sequence encoding the SbHRGP3 promoter (including the 5' untranslated region of the SbHRGP3 gene)
3. SEQ ID NO: 3 Forward primer ptxA5' 5'-GGCGCGCCCGCAATTTTTTGTGAAGC-3'
4. SEQ ID NO: 4 Reverse primer ptxA3' 5'-TCTAGATAAGTTTCGAAGATTTTAG-3'
5. SEQ ID NO: 5 Forward Primer SbHRGP3
   5'-TCTAGATAGAAGCTTTTCAACAATCATGC-3'
6. SEQ ID NO: 6 Reverse primer SbHRGP3 5'-AGATCTTACTGCCATTAGGAGAGG-3'
7. SEQ ID NO: 7 Nucleic acid sequence encoding functional equivalent homolog of the SbHRGP3 promoter (including the 5' untranslated region of the SbHRGP3 gene)
8. SEQ ID NO: 8 Nucleic acid sequence encoding functional equivalent homolog of the SbHRGP3 promoter (including the 5' untranslated region of the SbHRGP3 gene)
9. SEQ ID NO: 9 Nucleic acid sequence encoding functional equivalent homolog of the SbHRGP3 promoter (including the 5' untranslated region of the SbHRGP3 gene)
10. SEQ ID NO: 10 Nucleic acid sequence encoding chimeric ptxA promoter - ubiquitin intron construct.
11. SEQ ID NO: 11 Reverse primer-2 ptxA3'-2
   5'-TCTAGATAAACTATGAAGCTTTG-3'
12. SEQ ID NO: 12 Oligonucleotide primer GUS forward
   5'-tggtcgtcatgaagatgcggactt-3'
13. SEQ ID NO: 13 Oligonucleotide primer GUS reverse 5'-ccgcttcgaaaccaatgcctaa-3'
14. SEQ ID NO: 14 Oligonucleotide primer: Forward primer for ptxA gene (ptxA-F1) 5'-gggccaaggacatagtagaa-3'
15. SEQ ID NO: 15 Oligonucleotide primer: Reverse primer for 1 ptxA gene (ptxA-R1) 5'-tgaagttacaaacgctgaca-3'
16. SEQ ID NO: 16 Oligonucleotide primer: Reverse primer 2 for ptxA gene (ptxA-R2) 5'-agagcatcacacgcaatcaa-3'
17. SEQ ID NO: 17 Oligonucleotide primer: Forward primer 1 for SbHRGP3 gene (SbHRGP3-F1) 5'-catgtgcgcgtacttttgta-3'
18. SEQ ID NO: 18 Oligonucleotide primer: Forward primer 2 for SbHRGP3 gene (SbHRGP3-F2) 5'-atgaagaatataagccaata-3'
19. SEQ ID NO: 19 Oligonucleotide primer: Reverse primer for SbHRGP3 gene (SbHRGP3-R1) 5'-agtgccatacaactgtctaa-3'

### Examples

### Chemicals

Unless indicated otherwise, chemicals and reagents in the Examples were obtained from Sigma Chemical Company (St. Louis, MO), restriction endonucleases were from New England Biolabs (Beverly, MA) or Roche (Indianapolis, IN), oligonucleotides were synthesized by MWG Biotech Inc. (High Point, NC), and other modifying enzymes or kits regarding biochemicals and molecular biological assays were from Clontech (Palo Alto, CA), Pharmacia Biotech (Piscataway, NJ), Promega Corporation (Madison, WI), or Stratagene (La Jolla, CA). Materials for cell culture media were obtained from Gibco/BRL (Gaithersburg, MD) or DIFCO (Detroit, MI). The cloning steps carried out for the purposes of the present invention, such as, for example, restriction cleavages, agarose gel electrophoresis, purification of DNA fragments, transfer of nucleic acids to nitrocellulose and nylon membranes, linking DNA fragments, transformation of E. *coli* cells, growing bacteria, multiplying phages and sequence analysis of recombinant DNA, are carried out as described by Sambrook (1989). The sequencing of recombinant DNA molecules is carried out using ABI laser fluorescence DNA sequencer following the method of Sanger (Sanger 1977).

### Example 1: Growth conditions of the plants for tissue-specific RT-PCR analysis or Northern analysis

In order to obtain 6-day old seedlings, in each case approximately 500 seeds (*Arabidopsis thaliana* ecotype Columbia) are surface-sterilized for 2 minutes with a 70% strength ethanol solution, treated for 2 minutes with a sodium hypochlorite solution (5% v/v), washed five times with distilled water and incubated for 1 day at 4°C in order to ensure uniform germination. The seeds are subsequently sown in sterilized containers (9.7 cm x 9.6 cm x 9 cm) on filter paper soaked in Hoagland's nutrient solution (modified for *Arabidopsis thaliana*). Hoagland's solution is prepared with three different 200x stock solutions. Stock solution I comprises 0.5 M Ca(NO₃)₂, stock solution II comprises 0.1 M MgSO₄, and stock solution III comprises 0.5 M KNO₃ and 0.1 M KH₂PO₄. Before use, all stock solutions were diluted 1:200 and then mixed 1:1:1. Trace elements were added by means of a 2,000x trace element stock solution (5 x 10⁻² M H₃BO₃, 4.5 x 10⁻³ M MNCl₂, 3.8 x 10⁻³ M ZnSO₄, 3 x 10⁻⁴ M CuSO₄, 1 x 10⁻⁴ M (NH₄)₆MO₇O₂₄) and 250x Fe-EDTA stock solution (10 mM FeCl₃, 10 mM Na-EDTA). The pH of the stock solution was then brought to 6.0 using 5 N KOH, and the Hoagland solution was then autoclaved. The seedlings are grown in the dark at 22°C and harvested 6 days after the germination phase has begun.

To obtain roots, 100 seeds are sterilized as described above, incubated for 4 days at 4°C and then grown in 250 mL flasks with MS medium (Sigma M5519) with addition of a further 3% sucrose and 0.5 g/LMES (Sigma M8652), pH 5.7. The seedlings are grown in a 16/8 hour photoperiod (Philips 58W/33 white-light lamp) at 22°C and 120 rpm and harvested after 3 weeks. For all the other plant organs used, the seeds are grown in standard soil, incubated for 4 days at 4°C to ensure uniform germination and then grown first under short-day conditions at 22°C, 9 h light (150 µE/m²S) and 60 to 65% relative atmospheric humidity, the temperature being lowered to 18°C during the night. In order to stimulate the development of shoot and flower, the plants were transferred into long-day conditions under a 16/8 hour photoperiod (OSRAM Lumilux Daylight 36W/12 fluorescent tubes) at 22°C. Young rosette leaves are harvested in the 8-leaf stage (after 3 weeks), and stems and opened flowers are harvested at development stage 14 (Smyth 1990) immediately after the stamens have developed. The green pods, which were used, were 10 to 13 mm in length.

### Example 2: RNA extraction and RT-PCR analysis

Total RNA is isolated from the plant organs described in Example 1 at various points in time of the development, following the RNA isolation protocol (Sambrook 1989) as modified for *Arabidopsis thaliana.* The samples were comminuted finely in a pestle and mortar with liquid N₂, 1 mL of homogenization buffer was added (4 M guanidinium thiocyanate, 0.1 M Tris HCl pH 7.0, 10 mM EDTA, 0.5% sodium laurylsarcosine, 1% (v/v) of β-mercaptoethanol), carefully disrupted further while defrosting and transferred into a 2 mL reaction vessel filled with 800 µL of phenol/chloroform/isoamyl alcohol (P/C/I) (25:24:1 v/v, covered with a layer of DEPC (diethylpyrocarbonate) treated water. The mixture was vortexed for 1 minute, centrifuged for 15 minutes at 4°C and 17,500 x g, the aqueous phase was removed and re-extracted by shaking with 800 µL of P/C/I and centrifuged (for 15 minutes at 4°C and 17,500 x g). To remove the phenol, the mixture was extracted with 800 µL of chloroform/isoamyl alcohol (24:1 v/v). Better phase separation was achieved by recentrifugation (see above). The supernatant was removed, and the nucleic acids were precipitated for 1 hour at -20°C with the same volume of isopropanol. The precipitate was sedimented at 4°C for 15 minutes at 17,500 x g, washed with 3 M sodium acetate (pH 5.4), recentrifuged (for 10 minutes at 17,500 x g and 4°C), and then washed 2 more times with ice-cold 70% ethanol. The pellet was resuspended in 750 µL of TENS buffer (50 mM Tris HCl pH 8.0, 10 mM EDTA, 100 mM NaCl, 2% SDS (w/v), 3 mg/mL diethyl thiocyanate), extracted with 800 µL of P/C/I and extracted by shaking with 800 µL of chloroform/isoamyl alcohol (see above). 5 M LiCl was added to the aqueous phase in a ratio of 1 : 1, the RNA was precipitated overnight at 4°C and then removed by centrifugation for 30 minutes at 4°C and 17,500 x g. Thereupon, the pellet was washed twice with 70% strength ethanol, dried at 50°C in a heating block and resuspended in 40 µL of H₂O. All of the solutions were made with triple-distilled H₂O, which had previously been treated with diethyl pyrocarbonate (DEPC) and subsequently autoclaved.

The reverse transcriptase polymerase chain reaction (RT-PCR) is used to detect the ptxA or SbHRGP3 gene transcript The first-strand cDNA synthesis is carried out starting with 6 µg of total RNA with an oligo (dT) primer and RT Superscript™II enzyme (200 units) following the manufacturer's instructions in a total volume of 20 µL (Life Technologies, Gaithersburg, MD; Cat. No. 18064-022). For the RNA, 500 ng of oligo (dT) primer is added in a final volume of 12 µL. The mixture is heated for 10 minutes at 70°C and subsequently immediately cooled on ice. Then, 4 µL of the 5x first-strand buffer [250 mM Tris-HCl (pH 8.3 at room temperature), 375 mM KCI, 15 mM MgCl₂], 2 µL of 0.1 M DTT and 1 mL of 10 mM dNTP mix (in each case 10 mM dATP, dCTP, dGTP and dTTP at neutral pH) are added. The mixture is heated for 2 minutes at 42°, RT Superscript™II enzyme (1 µL (200 units), Life Technologies) is added, and the mixture is incubated for 50 minutes at 42°C. The oligo (dT) primer used is an oligonucleotide with 17 dT residues.

Approximately 2 µL of the first-strand cDNA synthesis are employed for the PCR reaction. The followings are combined in a total volume of 50 µL, following the manufacturer's instructions (Life Technologies):
5 µL of 10x PCR buffer [200 mM Tris-HCl (pH 8.4), 500 mM KCl]
1.5 µL of 50 mM MgCl₂
1 µL 10 mM dNTP mix (in each case 10 mM dATP, dCTP, dGTP and dTTP)
1 µL amplification primer 1 (10 µM)
1 µL amplification primer 2 (10 µM)
0.4 µL Taq DNA polymerase (5 U/µl)
2 µL cDNA (from the first-strand cDNA synthesis)
38.1. µL of autoclaved distilled water

The following amplification primers were employed as RT-PCR primers:
Forward primer for ptxA gene ptxA-F1 (SEQ ID NO: 14):
   5'-gggccaaggacatagtagaa-3'
Reverse primer 1 for ptxA gene ptxA-R1 (SEQ ID NO: 15):
   5'-tgaagttacaaacgctgaca-3'
Reverse primer 2 for ptxA gene ptxA-R2 (SEQ ID NO:16):
   5'-agagcatcacacgcaatcaa-3'
Forward primer 1 for SbHRGP3 gene SbHRGP3-F1 (SEQ ID NO: 17):
   5'-catgtgcgcgtacttttgta-3'
Forward primer 2 for SbHRGP3 gene SbHRGP3-F2 (SEQ ID NO: 18):
   5'-atgaagaatataagccaata-3'
Reverse primer for SbHRGP3 gene SbHRGP3-R1 (SEQ ID NO: 19):
   5'-agtgccatacaactgtctaa-3'

The alternative primer for the ptxA promoter reverse primer or the SbHRGP3 forward primer can be used alternatively in combination with its counter part, but can also be used to verify product identity in a "nested" PCR reaction.

The reaction mixture is covered with a layer of approximately 50 µL of silicone oil and subjected to the following temperature program (Thermocycler: MWG Biotech Primus HT; MWG Biotech, Germany):

| | |
|---|---|
| 1 | cycle of 180 sec at 95°C |
| 30 | cycles of 40 sec at 95°C, 60 sec at 53°C and 2 min at 72°C. |
| 1 | cycle of 5 minutes at 72°C. |

The presence of the ptxA or SbHRGP3 mRNA in a sample is then detected electrophoretically by staining, for example with ethidium bromide, by separating the reaction mixture on a 1% agarose gel.

### Example 3: Cloning of the ptxA or SbHRGP3 promoter

Genomic DNA from pea and soybean is extracted using the Qiagen DNAeasy Plant Mini Kit (Qiagen). The ptxA promoter region including the 5'-untranslated region (882 bp) and the SbHRGP3 promoter region including the 5'-untranslated region (1380 bp), respectively, were isolated from genomic DNA of pea (*Pisum sativum*) or soybean (*Glycine max*), respectively, using conventional PCR. Approximately 0.1 µg of digested genomic DNA was used for the regular PCR reaction (see below). The primers were designed based on the pea ptxA sequence disclosed by Bown (GenBank accession number X67427.1) and the SbHRGP3 sequence disclosed by Ahn (GenBank Acc.-No.: U44838), respectively. One µL of the diluted digested genomic DNA was used as the DNA template in the primary PCR reaction. The reaction comprised primers primer 1 (SEQ ID NO:3) and primer 2 (SEQ ID NO:4 or 11) for amplification of the ptxA promoter, or primers primer 1 (SEQ ID NO: 5) and primer 2 (SEQ ID NO: 6 or 11) for amplification of the SbHRGP3 promoter, respectively, in a mixture containing Buffer 3 following the protocol outlined by an Expand Long PCR kit (Cat #1681-842, Roche-Boehringer Mannheim). The isolated DNA is employed as template DNA in a PCR amplification reaction using the following primers:

The amplifications primers employed ("forward" and "reverse" primers) are the following oligonucleotides:
Forward primer (ptxA5'): 5'-GGCGCGCCCGCAATTTTTTGTGAAGC-3' (SEQ ID NO:3)
Reverse primer-1 (ptxA3'): 5'-TCTAGATAAGTTTCGAAGATTTTAG -3' (SEQ ID NO: 4)

For amplification of the ptxA promoter described by base 1 to 828 of SEQ ID NO: 1 the following reverse primer is used instead of primer ptxA3':
Reverse primer-2 (ptxA3'-2): 5'-TCTAGATAAACTATGAAGCTTTG-3' (SEQ ID NO: 11).
Forward Primer (SbHRGP3 5') 5'-TCTAGATAGAAGCTTTTCAACAATCATGC-3' (SEQ ID NO: 5)
Reverse primer (SbHRGP3 3') 5'-AGATCTTACTGCCATTAGGAGAGG-3' (SEQ ID NO: 6)

Amplification is carried out as follows:

| | |
|---|---|
| 1 x PCR | reaction buffer (Roche Diagnostics) |
| 5 µL | genomic DNA (corresponds to approximately 80 ng) |
| 2 µL | 10 mM of each dATP, dCTP, dGTP and dTTP (Invitrogen: dNTP mix) |
| 1 µL | primer ptxA5' (SEQ ID NO: 3) or SbHRGP3 5' (SEQ ID NO: 5), respectively (100 µM) |
| 1 µL | primer ptxA3' (SEQ ID NO: 4), ptxA3'-2 (SEQ ID NO: 11) or SbHRGP3 3' (SEQ ID NO: 6), respectively (100µM) |
| 1 µL | Taq DNA polymerase 5 U/µL (Roche Diagnostics). |

in a final volume of 100 µL.

The following temperature program is used (Thermocycler:T3 Thermocycler Biometra ):

| | |
|---|---|
| 1 | cycle with 180 sec at 95°C |
| 30 | cycles with 40 sec at 95°C, 60 sec at 53°C and 2 min at 72°C |
| 1 | cycle with 5 min at 72°C |

The PCR product is applied to a 1% (w/v) agarose gel and separated at 80V. Fragments of approximately 882 base pairs in length are excised from the gel and purified with the aid of the Qiagen Gel Extraction Kit (Qiagen, Hilden, Germany). If appropriate, the eluate of 50 µL can be evaporated. The purified DNA is digested as follows for 2 hours at 37°C:
19 µL purified PCR-DNA
1 µL *Asc*I restriction enzyme (10 U, Roche Diagnostics)
1 µL *Xba*I restriction enzyme (10 U, Roche Diagnostics)
10 µL buffer B (Roche Diagnostics)
69 µL distilled water

This is followed by purification via the PCR Purification Kit (Roche Diagnostics). The cut and purified DNA fragment is inserted into the Bluescript plasmid (Stratagene) into the *Asc*I and *Xba*I cleavage sites. Ligation of the vectors, transformation into *E.coli* cells and analysis of the plasmids is carried out by standard methods (Sambrook 1989). The identity can be verified by sequencing the plasmid and comparison with the genomic DNA sequence (Genbank Number X67427.1). The resulting construct is pBPS-ptxA or pBPS-SbHRGP3 (Fig. 2, construct I).

As an alternative, the PCR product can be cloned directly into vector pCR4-TOPO (Invitrogen) following the manufacturer's instructions, i.e. the PCR product obtained is inserted into a vector having T overhangs with its A overhangs and a topoisomerase.

### EXAMPLE 4: Construction of ptxA or SbHRGP3 promoter containing transformation vectors

PtxA promoter fragment in the Topo vector (Invitrogen) is digested with *Asc*I and *Xba*I at 37°C for 2h or 4°C overnight. The promoter fragment was purified from the gel (Qiagen kit) after electrophoresis and cloned into upstream of GUS reporter gene in pUC using Rapid Ligation kit (Roche). The ligation solution is transformed into *E.coli* DH5α cells (Stratagene). The GUS chimeric constructs in pUC are digested with *Asc*I and *Pme*I for and cloned into a binary vector. SbHRGP3 is cloned into *Xba*I and *Bg*III sites in a binary vector to generate the GUS chimeric construct.

GUS chimeric constructs for monocotyledonous plant transformation is made by adding intron of interest in the 5' untranslated region, which is located between downstream of the promoter and upstream of the reporter gene. Intron of interest is amplified with the primers containing *Pac*I for 5' terminus and *Sbf*I or *Xma*I for the 3' terminus overhang. The PCR fragment is digested with *Pac*I and *Sbf*I *or Xma*I and cloned in the 5' untranslated region of a binary vector.

### EXAMPLE 5: Agrobacterium-mediated transformation in dicotyledonous and monocotyledonous plants

### 5.1: Transformation and regeneration of transgenic Arabidopsis thaliana (Columbia) plants

To generate transgenic *Arabidopsis* plants, *Agrobacterium tumefaciens* (strain C58C1 pGV2260) is transformed with various ptxA or SbHRGP3 promoter/GUS vector constructs. The agrobacterial strains are subsequently used to generate transgenic plants. To this end, a single transformed *Agrobacterium* colony is incubated overnight at 28°C in a 4 mL culture (medium: YEB medium with 50 µg/mL kanamycin and 25 µg/mL rifampicin). This culture is subsequently used to inoculate a 400 mL culture in the same medium, and this is incubated overnight (28°C, 220 rpm) and spun down (GSA rotor, 8,000 rpm, 20 min). The pellet is resuspended in infiltration medium (1/2 MS medium; 0.5 g/L MES, pH 5.8; 50 g/L sucrose). The suspension is introduced into a plant box (Duchefa), and 100 mL of SILWET L-77 (heptamethyltrisiloxan modified with polyalkylene oxide; Osi Specialties Inc., Cat. P030196) was added to a final concentration of 0.02%. In a desiccator, the plant box with 8 to 12 plants is exposed to a vacuum for 10 to 15 minutes, followed by spontaneous aeration. This is repeated twice or 3 times. Thereupon, all plants are planted into flowerpots with moist soil and grown under long-day conditions (daytime temperature 22 to 24°C, nighttime temperature 19°C; relative atmospheric humidity 65%). The seeds are harvested after 6 weeks.

As an alternative, transgenic *Arabidopsis* plants can be obtained by root transformation. White root shoots of plants with a maximum age of 8 weeks are used. To this end, plants, which are kept under sterile conditions in 1 MS medium (1% sucrose; 10mg/L inositol; 1.0 mg/L thiamine; 0.5 mg/L pyridoxine; 0.5 mg/L nicotinic acid; 0.5 g MES, pH 5.7; 0.8 % agar) are used. Roots are grown on callus-inducing medium for 3 days (1x Gamborg's B5 medium; 2% glucose; 0.5 g/L mercaptoethanol; 0.8% agar; 0.5 mg/L 2,4-D (2,4-dichlorophenoxyacetic acid); 0.05 mg/L kinetin). Root sections 0.5 cm in length are transferred into 10 to 20 mL of liquid callus-inducing medium (composition as described above, but without agar supplementation), inoculated with 1 mL of the above-described overnight agrobacterial culture (grown at 28°C, 200 rpm in LB) and shaken for 2 minutes. After excess medium has been allowed to run off, the root explants are transferred to callus-inducing medium with agar, subsequently to callus-inducing liquid medium without agar (with 500 mg/L betabactyl, SmithKline Beecham Pharma GmbH, Munich), incubated with shaking and finally transferred to shoot-inducing medium (5 mg/L 2-isopentenyladenine phosphate; 0.15 mg/L indole-3-acetic acid; 50 mg/L kanamycin; 500 mg/L betabactyl). After 5 weeks, and after 1 or 2 medium changes, the small green shoots are transferred to germination medium (1 MS medium; 1% sucrose; 100 mg/L inositol; 1.0 mg/L thiamine; 0.5 mg/L pyridoxine; 0.5 mg/L nicotinic acid; 0.5 g MES, pH 5.7; 0.8% agar) and regenerated into plants.

### 5.2: Transformation and regeneration of crop plants

The *Agrobacterium*-mediated plant transformation using standard transformation and regeneration techniques may also be carried out for the purposes of transforming crop plants (Gelvin 1995; Glick 1993).

For example, oilseed rape can be transformed by cotyledon or hypocotyl transformation (Moloney 1989; De Block 1989). The use of antibiotics for the selection of *Agrobacteria* and plants depends on the binary vector and the *Agrobacterium* strain used for the transformation. The selection of oilseed rape is generally carried out using kanamycin as selectable plant marker.

The *Agrobacterium*-mediated gene transfer in linseed (*Linum usitatissimum*) can be carried out using for example a technique described by Mlynarova (1994).

The transformation of soya can be carried out using, for example, a technique described in EP-A1 0424 047 or in EP-A1 0397 687, US 5,376,543, US 5,169,770.

The transformation of maize or other monocotyledonous plants can be carried out using, for example, a technique described in US 5,591,616.

The transformation of plants using particle bombardment, polyethylene glycol-mediated DNA uptake or via the silicon carbonate fiber technique is described, for example, by Freeling & Walbot (1993) "The maize handbook" ISBN 3-540-97826-7, Springer Verlag New York).

### Example 6: Detection of the tissue-specific expression

To identify the characteristics of the promoter and the essential elements of the latter, which bring about its tissue specificity, it is necessary to place the promoter itself and various fragments thereof before what is known as a reporter gene, which allows the determination of the expression activity. An example, which may be mentioned, is the bacterial β-glucuronidase (Jefferson 1987a). The β-glucuronidase activity can be detected *in-planta* by means of a chromogenic substrate such as 5-bromo-4-chloro-3-indolyl-β-D-glucuronic acid in an activity staining (Jefferson 1987b). To study the tissue specificity, the plant tissue is cut, embedded, stained and analyzed as described (for example Bäumlein 1991 b).

A second assay permits the quantitative determination of the GUS activity in the tissue studied. For the quantitative activity determination, MUG (4-methylumbelliferyl-β-D-glucuronide) is used as substrate for β-glucuronidase, and the MUG is cleaved into MU (methylumbelliferone) and glucuronic acid.

To do this, a protein extract of the desired tissue is first prepared and the substrate of GUS is then added to the extract. The substrate can be measured fluorimetrically only after the GUS has been reacted. Samples, which are subsequently measured in a fluorimeter, are taken at various points in time. This assay may be carried out for example with linseed embryos at various developmental stages (21, 24 or 30 days after flowering). To this end, in each case one embryo is ground into a powder in a 2 mL reaction vessel in liquid nitrogen with the aid of a vibration grinding mill (Type: Retsch MM 2,000). After addition of 100 µL of EGL buffer, the mixture is centrifuged for 10 minutes at 25°C and 14,000 x g. The supernatant is removed and recentrifuged. Again, the supernatant is transferred to a new reaction vessel and kept on ice until further use. 25 µl of this protein extract are treated with 65 µL of EGL buffer (without DTT) and employed in the GUS assay. 10 µL of the substrate MUG (10 mM 4-methylumbelliferyl-β-D-glucuronide) are now added, the mixture is vortexed, and 30 µL are removed immediately as zero value and treated with 470 µL of Stop buffer (0.2 M Na₂CO₃). This procedure is repeated for all of the samples at an interval of 30 seconds. The samples taken were stored in the refrigerator until measured. Further readings were taken after 1 h and after 2 h. A calibration series, which contained concentrations from 0.1 mM to 10 mM MU (4-methylumbelliferone), was established for the fluorimetric measurement. If the sample values were outside these concentrations, less protein extract was employed (10 µL, 1 µL, 1 µL from a 1:10 dilution), and shorter intervals were measured (0 h, 30 min, 1 h). The measurement was carried out at an excitation of 365 nm and an emission of 445 nm in a Fluoroscan II apparatus (Labsystem). As an alternative, the substrate cleavage can be monitored fluorimetrically under alkaline conditions (excitation at 365 nm, measurement of the emission at 455 nm; Spectro Fluorimeter BMG Polarstar+) as described in Bustos (1989). All the samples were subjected to a protein concentration determination by the method of Bradford (1976), thus allowing an identification of the promoter activity and promoter strength in various tissues and plants.

EGL buffer: 0.1 M KPO₄, pH 7.8; 1 mM EDTA; 5% glycerol; 1 M DTT.

### EXAMPLE 7: Analysis of ptxA and SbHRGP3 promoter expression in Arabidopsis and canola

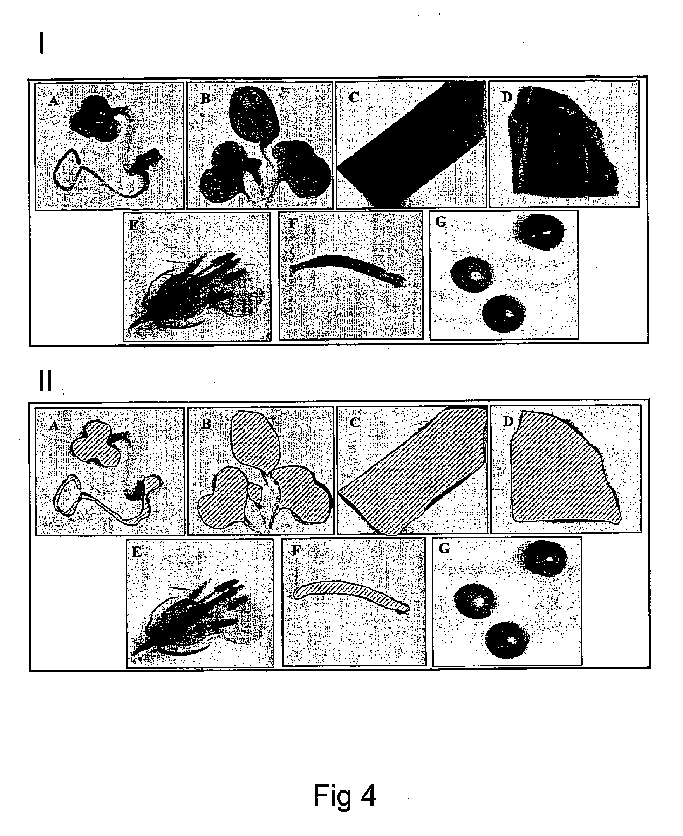

In *Arabidopsis,* ptxA promoter shows strong constitutive and ubiquitous expression in most tissues and organs at different developmental stages and very low levels or no (by GUS staining) in seeds (Figure 3A-3G). Strong ubiquitous expression can be detected in young seedlings. The GUS expression levels are low in the organs at the reproductive stages (siliques and flowers). No GUS histochemical stain is detected in seeds (Table 1). In canola, the expression patterns are very similar to those in *Arabidopsis* (Figure 4A-4G, Table 1).

**Table 1. GUS expression controlled by ptxA promoter in Arabidopsis and canola**

| Plant species | Seedlings | Leaves at early reproductive stages | Roots at early reproductive stages | Flowers | Siliques or seedpods | Seeds |
|---|---|---|---|---|---|---|
| *Arabidopsis* | +++++ | ++ | ++ | ++ | ++ | - |
| Canola | +++++ | ++++ | N/A | ++* | -/+ | - |

| | | | | | | |
|---|---|---|---|---|---|---|
| *no expression in petals, medium levels of expression in sepals; a range of GUS expression levels measured by histochemical assay (- to +++++) | | | | | | |

Expression profiles of ptxA homologue in soybean showed no or very low expression in abiotic stressed roots, leaves, shoots, and rosettes under normal conditions, high expression in stems and roots (normal and infected) and flowers, and strong expression in calli.

These expression patterns found in *Arabidopsis* and canola are entirely different from those which would be expected from the expression patterns controlled by MsPRP2 promoter, since nucleotide sequence of ptxA promoter is highly homologous to MsPRP2 promoter. MsPRP2 promoter is reported as a salt-inducible and highly root-specific promoter (Bastola 1998; WO 99/53016). These results indicate that sequence similarity and expression patterns are not correlated.

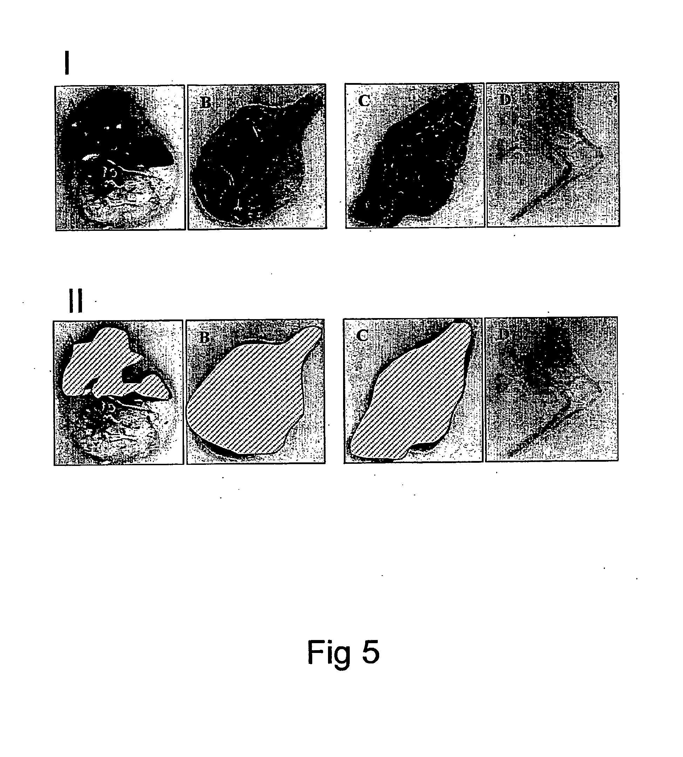

In *Arabidopsis,* SbHRGP3 promoter shows almost identical expression patterns but lower expression levels in general compared to ptxA promoter (Figure 5A-5D).

### EXAMPLE 8: Assessment of expression patterns by real time RT PCR analysis

Total RNA is extracted from plant tissues using Qiagen RNeasy Plant Mini Kit (Cat. No 74904). Quality and quantity of the RNA are determined using Molecular Probes RiboGreen Kit (Cat. No. R-11490) on the Spectra MAX Gemini. One µg of RNA is used for RT-PCR (Roche RT-PCR AMV kit, Cat. No. 1483188) in the reaction solution I under the optimized PCR program described below.

### Reaction solution I:

1 µg RNA
2 µL 10x Buffer
4 µL 25 mM MgCl₂
2 µL 1 mM dNTPs
2 µL 3.2 µg Random Primers
1 µL 50 units RNase Inhibitor
0.8 µL 20 units AMV-RT polymerase
   Fill to 20 µL with sterile water

### PCR Program

1) 25°C 10minutes
2) 42°C 1 hour
3) 99°C 5minutes
4) 4°C Stop reaction

RT-PCR sample is used for the LightCycler reaction (Roche: LightCycler FastStart DNA Master SYBR Green I, Cat. No. 3003230).

### LightCycler reaction

11.6µL sterile water
2.4µL 25mM MgCl₂
2µL SYBER Green Polymerase mix
2µL 10 µM Specific Primer Mix (GUS forward and GUS reverse)
2µL RT-PCR reaction product

GUS forward: 5'-tggtcgtcatgaagatgcggactt-3' (SEQ ID NO: 12)
GUS reverse: 5'-ccgcttcgaaaccaatgcctaa-3' (SEQ ID NO: 13)

### LightCycler Program

1) 95°C 5minutes
2) 95°C 30seconds
3) 61°C 40seconds
4) 72°C 40seconds - Repeat steps 2-4 for 30 cycles
5) 72°C 10minutes
6) 4°C Stop reaction

Standardizing the concentration of RNA (1 µg) in each of the RT-PCR reactions is sufficient to directly compare samples if the same primers are used for each LightCycler reaction. The output results are a number that corresponds to the cycle of PCR at which the sample reaches the inflection point in the log curve generated. The lower the cycle number, the higher the concentration of target RNA present in the sample. Each sample is repeated in triplicate and an average is generated to produce the sample "crosspoint" value. The lower the crosspoint, the stronger the target gene is expressed in that sample. (For detailed procedure see *Roche Molecular Biochemicals* LightCycler System: Reference Guide May 1999 version)

### EXAMPLE 9: Utilization of transgenic crops

PtxA or SbHRGP3 promoter may be employed to either express transgenes in a target plant or to suppress expression of endogenous genes (*e.g*., by antisense or double-stranded RNA; see above), thereby improving - for example - biomass and/or yield, or tolerant to biotic and abiotic environmental stresses. The chimeric constructs are transformed into dicotyledonous and monocotyledonous plants. Standard methods for transformation in the art can be used if required. Transformed plants are regenerated using known methods. Various phenotypes are measured to determine improvement of biomass, yield, fatty acid composition, high oil, disease tolerance, or any other phenotypes that link yield enhancement or stability. Gene expression levels are determined at different stages of development and at different generations (To to T₂ plants or further generations). Results of the evaluation in plants lead to determine appropriate genes in combination with this promoter to increase yield.

### EXAMPLE 10: Expression of selectable marker gene in dicotyledonous plants

A chimeric construct composed of ptxA or SbHRGP3 promoter and selectable marker gene can be transformed into dicotyledonous plants such as *Arabidopsis,* soybean, or canola, but is not restricted to these plant species. Standard methods for transformation in the art can be used if required. Transformed plants are selected under the selection agent of interest and regenerated using known methods. Selection scheme is examined at early developmental stages of tissues or tissue culture cells. Gene expression levels can be determined at different stages of development and at different generations (To to T₂ plants or further generations). Results of the evaluation in plants lead to determine appropriate genes in combination with this promoter.

### EXAMPLE 11: Expression of selectable marker gene in monocotyledonous plants

A chimeric construct composed of ptxA or SbHRGP3 promoter and selectable marker gene can be transformed into monocotyledonous plants such as rice, barley, maize, wheat, or ryegrass but is not restricted to these plant.species. Any methods for improving expression in monocotyledonous plants are applicable such as addition of intron or exon with intron in 5'UTR either non-spliced or spliced. Standard methods for transformation in the art can be used if required. Transformed plants are selected under the selection agent of interest and regenerated using known methods. Selection scheme is examined at early developmental stages of tissues or tissue culture cells. Gene expression levels can be determined at different stages of development and at different generations (To to T₂ plants or further generations). Results of the evaluation in plants lead to determine appropriate genes in combination with this promoter.

### Example 12: Deletion analysis

The cloning method is described by Rouster (1997) and S ambrook (1989). Detailed mapping of the ptxA or SbHRGP3 promoter (*i.e*., narrowing down of the nucleic acid segments relevant for its specificity) is performed by generating various reporter gene expression vectors which firstly contain the entire promoter region and secondly various fragments thereof. Firstly, the entire promoter region or fragments thereof are cloned into a binary vector containing GUS or other reporter gene. To this end, fragments are employed firstly, which are obtained by using restriction enzymes for the internal restriction cleavage sites in the full-length promoter sequence. Secondly, PCR fragments are employed which are provided with cleavage sites introduced by primers. The chimeric GUS constructs containing various deleted promoters are transformed into *Arabidopsis* and other plant species using transformation methods in the current art. Promoter activity is analyzed by using GUS histochemical assays or other appropriate methods in various tissues and organs at the different developmental stages.

### Example 13: In vivo mutagenesis

The skilled worker is familiar with a variety of methods for the modification of the promoter activity or identification of important promoter elements. One of these methods is based on random mutation followed by testing with reporter genes as described above. The *in vivo* mutagenesis of microorganisms can be achieved by passage of the plasmid (or of another vector) DNA through *E*. *coli* or other mi croorganisms (for example *Bacillus* spp. or yeasts such as *Saccharomyces cerevisiae*) in which the ability of maintaining the integrity of the genetic information is disrupted. Conventional mutator strains have mutations in the genes for the DNA repair system (for example mutHLS, mutD, mutT and the like; for reference, see Rupp 1996). The skilled worker is familiar with these strains. The use of these strains is illustrated for example by Greener (1994). The transfer of mutated DNA molecules into plants is preferably effected after selection and testing of the microoganisms. Transgenic plants are generated and analyzed as described above.

### Example 14: PLACE Analysis for ptxA Promoter (SEQ ID NO: 1)

Based on the below given PLACE results a potential TATA box is localized at base pair 549 to base pair 554 of SEQ ID NO: 1. In consequence the 5' untranslated region starts at about base pair 584 and extends to base pair 863 of SEQ ID NO: 1. The sequence described by SEQ ID NO: 1 end just before the ATG start codon. Based on the promoter element analysis there seem to be no clusters of promoter elements in the first 300 base pairs of the sequence described by SEQ ID NO: 1. It is therefore very likely that the core region of the ptxA promoter extends from about base pair 300 to about base pair 583 of the sequence described by SEQ ID NO: 1.

The following clusters of promoter elements were identified in the ptxA promoter as described by SEQ ID NO: 1:

| Motif Name | Location (Strand) | Motif Sequence |
|---|---|---|
| AMYBOX2 | 537 (+) | TATCCAT |
| C8GCARGAT | 571 (+/-) | CWWWWWWWWG |
| CAATBOX1 | 368(+); 439, 525 (-) | CAAT |
| CARGCW8GAT | 571 (+/-) | CWWWWWWWWG |
| CCAATBOX1 | 367 (+) | CCAAT |
| DOFCOREZM | 334, 357, 382, 389, 400, 429 (+); 446, 517, 591 (-) | AAAG |
| EBOXBNNAPA | 407, 409 (+); 407, 409 (-) | CANNTG |
| GATABOX | 337 (+), 537 (-) | GATA |
| GT1CONSENSUS | 424, 544 (+); 363, 518, 593 (-) | GRWAAW |
| GTGANTG10 | 406, 452 (-) | GTGA |
| GTGANTG10 | 479 (-) | GTGA |
| IBOX | 535 (-) | GATAAG |
| IBOXCORE | 536 (-) | GATAA |
| IBOXCORENT | 534 (-) | GATAAGR |
| MYBST1 | 537 (-) | GGATA |
| MYCATERD1 | 409 (+); 407 (-) | CATGTG |
| MYCATRD22 | 407 (+); 409 (-) | CACATG |
| MYCCONSENSUSAT | 407 (+) | CANNTG |
| MYCCONSENSUSAT | 409 (+); 407, 409 (-) | CANNTG |
| POLASIG1 | 550 (+) | AATAAA |
| POLASIG2 | 396 (+) | AATTAAA |
| POLASIG3 | 462 (+) | AATAAT |
| POLLEN1LELAT52 | 359 (+); 595 (-) | AGAAA |
| PYRIMIDINEBOXOSRAMY1A | 590 (+) | CCTTTT |
| SEBFCONSSTPR10A | 476 (+) | YTGTCWC |
| SEF4MOTIFGM7S | 301 (+) | RTTTTTR |
| TAAAGSTKST1 | 388, 399 (+) | TAAAG |
| TATABOX5 | 549 (-) | TTATTT |
| TATCCAOSAMY | 537 (+) . | TATCCA |
| TATCCAYMOTIFOSRAMY3D | 537 (+) | TATCCAY |

### Example 15: PLACE Analysis for SbHRGP3 Promoter (SEQ ID NO: 2)

Based on the below given PLACE results a potential TATA box is localized at base pair 1147 to base pair 1152 of SEQ ID NO: 2. In consequence the 5' untranslated region starts at about base pair 1179 and extends to base pair 1380 of SEQ ID NO: 2. The sequence described by SEQ ID NO: 2 ends 12 base pairs before the ATG start codon. Based on the promoter element analysis there seem to be no clusters of promoter elements in the first 800 base pairs of the sequence described by SEQ ID NO: 2. It is therefore very likely that the core region of the SbHRGP3 promoter extends from about base pair 800 to about base pair 1179 of the sequence described by SEQ ID NO: 2. The following clusters of promoter elements were identified in the SbHRGP3 promoter as described by SEQ ID NO: 2:

| Motif Name | Location (Strand) | Motif Sequence |
|---|---|---|
| -300ELEMENT | 856 (+) | TGHAAARK |
| AMYBOX1 | 841 (-) | TAACARA |
| ARFAT | 1166 (+) | TGTCTC |
| BOXIINTPATPB | 966 (+) | ATAGAA |
| CBGCARGAT | 1014 (+/-) | CWWWWWWWWG |
| CAATBOX1 | 801, 1014, 1228, 1234 (+); 996, 1212, 1258, 1274 (-) | CAAT |
| CARGCW8GAT | 1014 (+/-) | CWWWWWWWWG |
| CCAATBOX1 | 1212 (-) | CCAAT |
| DOFCOREZM | 852, 859, 931, 1026, 1080, 1339, 1349 (+) | AAAG |
| DOFCOREZM | 825, 951, 1189 (-) | AAAG |
| GARE1OSEP1 | 841 (-) | TAACAGA |
| GATABOX | 868, 915, 1283, 1311, 1324 (+) | GATA |
| GATABOX | 1172, 1231 (-) | GATA |
| GT1CONSENSUS | 1083, 1283, 1311, 1324, 1332 (+) | GRWAAW |
| GT1CONSENSUS | 1104, 1131, 1149, 1238 (-) | GRWAAW |
| GTGANTG10 | 855, 989 (+) ; 936 (-) | GTGA |
| IBOXCORE | 1283, 1311, 1324 (+) | GATAA |
| INRNTPSADB | 852, 976 (-) | YTCANTYY |
| MARTBOX | 1124 (+) | TTWTWTTWTT |
| MYB1LEPR | 1119 (+) | GTTAGTT |
| MYBCORE | 842 (+) | CNGTTR |
| MYBPLANT | 1301 (+) | MACCWAMC |
| MYBPZM | 1303 (+) | CCWACC |
| MYBST1 | 1323 (+) | GGATA |
| PALBOXPPC | 1190 (+) | YTYYMMCMAMCMMC |
| POLASIG1 | 1049, 1128 (-) | AATAAA |
| POLASIG2 | 1054 (-) | AATTAAA |
| POLASIG3 | 1015 (+); 1146 (-) | AATAAT |
| POLLEN1LELAT52 | 1082 (+); 1133 (-) | AGAAA |
| PYRIMIDINEBOXOSRAMY1A | 930 (-) | CCTTTT |
| QELEMENTZMZM13 | 933 (+) | AGGTCA |
| RAV1AAT | 1100, 1355 (+) | CAACA |
| RBCSCONSENSUS | 1177 (+) | AATCCAA |
| REALPHALGLHCB21 | 1197 (+) | AACCAA |
| ROOTMOTIFTAPOX1540, | 811, 1046, 1236(+); 802, 1229, 12135(-) | ATATT |
| RYREPEATBNNAPA | 940 (+) | CATGCA |
| RYREPEATGMGY2 | 940 (+) | CATGCAT |
| RYREPEATLEGUMINBOX | 940 (+) | CATGCAY |
| SEBFCONSSTPR10A | 1165 (+); 989 (-) | YTGTCWC |
| SEF1MOTIF | 1046 (+) | ATATTTAWW |
| SV40COREENHAN | 1189 (-) | GTGGWWHG |
| TAAAGSTKST1 | 1079, 1348 (+); 951 (-) | TAAAG |
| TATABOX4 | 1042 (-) | TATATAA |
| TATABOX5 | 1050, 1124, 1129, 1147 (+); 1085 (-) | TTATTT |
| TATAPVTRNALEU | 1041 (+) | TTTATATA |

| | | |
|---|---|---|
| TATCCAOSAMY | 1322 (-) | TATCCA |
| TGTCACACMCUCUMISIN | 988 (-) | TGTCACA |
| TRANSINITDICOTS | 889 (-) | AMNAUGGC |
| TRANSINITMONOCOTS | 889 (-) | RMNAUGGC |
| WBOXATNPR1 | 1021 (+); 1098 (-) | TTGAC |
| WUSATAg | 845 (+) | TTAATGG |

### EXAMPLE 16: Analysis of ptxA in T₃ Arabidopsis

Based on GUS histochemical assays, T₃ *Arabidopsis* lines containing a ptxA::GUS chimeric construct show strong expression in vegetative tissues and organs, sporadic and low expression in flowers, low to medium expression in siliques and funiculus (the stalk of a seed), and no expression in seeds (4, 8, and 14 Days After Flowering; DAF). These expression patterns are very similar to those in T₂ generation. T₂ lines show low to no expression or low expression in restricted regions of the flowers. In T₃, young flowers (4 DAF) show more expression than older flowers (8 and 14 DAF). In addition, the high copy lines (*e.g*. D54) show more expression in flowers than si ngle copy lines. In T₂ and T₃, however, no GUS stain is detected in seeds at various developmental stages (4, 8, and 14 DAF).

For the tissues in the vegetative stages, GUS expression is measured at the mRNA levels using real time RT-PCR (Table 2). The real time RT-PCR results indicate that ptxA promoter controls medium to strong expression in most tissues in the vegetative stages. The high copy lines (*e.g*. D54) show stronger expression than low copy lines (Table 2), which is not easily distinguished by the GUS histochemical assays, since the expression levels in the vegetative tissues are already high. This data supports the GUS histochemical assays with respect to the effect of gene dosage found in flower. Quantification of GUS expression in only seeds is not feasible, since siliques and the region connected between seed and silique have medium level of expression, which can easily contaminate the expression in seed samples.

**Table 2: GUS expression controlled by ptxA promoter in vegetative tissues at various developmental stages of T₃ Arabidopsis**

| Developmental stages | Crosspoints | | | | |
|---|---|---|---|---|---|
| | D31 (1)* | D36 (1) | D52 (1) | D69 (1) | D54 (5) |
| Germination [4 DAG] | 25.45±0.049 | 23.42±0.30 | 21.62±0.303 | 20.88±0.116 | 20.30±0.112 |
| Leaves & stems [14 DAG*] | 25.633±0.071 | 23.79±0.123 | 21.23±0.102 | 21.4±0.095 | 21.33±0.107 |
| Roots [14 DAG] | 24.84±0.150 | 25.54±0.031 | 24.41±0.369 | 22.62±0.124 | N/A |
| Leaves & stems [21 DAG] | 24.84±0.128 | 26.49±0.039 | 24.2±0.110 | 23.87±0.965 | 21.77±0.327 |
| Roots [21 DAG] | 25.99±0.199 | 24.00±0.195 | 22.06±0.251 | 24.97±0.502 | 21.9±0.955 |
| Rosette leaves | 24.743±0.068 | 22.770±0.075 | 20.030±0.053 | 20.85±0.095 | 21.97±0.651 |
| Stem leaves | 24.16±0.105 | 23.045±0.186 | 21.17±0.443. | 21.40±0.199 | 19.92±0.251 |

Quantitative PCR (qPCR) experiments detected increased expression levels of the GUS gene from reverse-transcribed mRNA isolated from the tissues of the transgenic *Arabidopsis* (T₃). Expression levels are represented as the crosspoint observed during qPCR of each sample. The crosspoint represents the cycle at which PCR enters log linear amplification, which is directly proportional to the amount of starting template. Therefore, the lower the crosspoint is the higher the expression. Samples were qPCR-amplified in triplicate. The GUS expression is normalized by the internal control (mean±standard deviations). * Five independent events (copy number)

### EXAMPLE 17: Construction of ptxA promoter in combination with maize Ubiquitin intron for monocot transformation

The PtxA-GUS construct in pUC is digested with *Pac*I and *Xma*I. pBPSMM348 is digested with *Pac*I and *Xma*I to isolate maize Ubiquitin intron (ZmUbi intron) followed by electrophoresis and the QIAEX II Gel Extraction Kit (cat# 20021). The ZmUbi intron is ligated into the PtxA-GUS in pUC to generate pUC based PtxA-ZmUbi intron-GUS construct followed by restriction enzyme digestion with *Afe*I and *Pme*I. PtxA-ZmUbi intron GUS cassette is cut out of a Seaplaque low melting temperature agarose gel (SeaPlaque® GTG® Agarose catalog No. 50110) after electrophoresis. A monocotyledonous base vector containing a selectable marker cassette (Monocot base vector) is digested with *Pme*I. The GUS expression cassette containing ptxA promoter-ZmUbi intron is ligated into the Monocot base vector) to generated pBPSET004 (PtxA-ZmUbi intron-GUS) construct (Fig. 9).

The pBPSET004 (PtxA-ZmUbi intron-GUS) construct is transformed into a recombinant LBA4404 strain containing pSB1 (super *vir* plasmid) using electroporation following a general protocol in the art. *Agrobacterium*-mediated transformation in maize is performed using immature embryo following a protocol described in US 5,591,616. An imidazolinone-herbicide selection is applied to obtain transgenic maize lines. GUS histochemical assays are conducted with the following samples: immature embryos at 3 days after co-cultivation, *in vitro* roots and leaves, and young transgenic plantlets (Table 3). This chimeric GUS construct shows strong expression *in vitro* in tissues and young T0 plantlets. This result indicates that dicotyledonous promoter (such as the ptxA promoter) in combination with monocotyledonous intron can be functional in monocotyledonous plants.

**Table 3: GUS expression controlled by ptxA promoter::ZmUbi intron in maize**

| Plant species | Immature embryo [3 days after co-cultivation] | Embyogenic calli | *In vitro* roots | *In vitro* leaves | To plantlets |
|---|---|---|---|---|---|
| Maize | - | ++++ | ++++ | ++ | +++ |

| | | | | | |
|---|---|---|---|---|---|
| *no expression in petals, medium levels of expression in sepals; a range of GUS expression levels measured by histochemical assay (- to +++++) | | | | | |

### REFERENCES

The references listed below and all references cited herein are incorporated herein by reference to the extent that they supplement, explain, provide a background for, or teach methodology, techniques, and/or compositions employed herein.
1. Ahn et al. (1998) Plant Physiol. 116: 671-679
2. An et al. (1985) EMBO J. 4:277-287
3. Atanassova et al. (1992) Plant J 2(3): 291-300
4. Ausubel et al. (1987) Current Protocols in Molecular Biology, Greene Publishing Assoc. and Wiley Interscience
5. Baerson & Lamppa (1993) Plant Mol Biol 22(2):255-67
6. Baker et al. (1987) EMBO J 6:1547-1554
7. Bastola et al. (1998) Plant Molecular Biology 38:1123-1135
8. Bäumlein et al. (1991a) Mol Gen Genet 225(3):459-467
9. Bäumlein et a/. (1991 b) Mol Gen Genet 225:121-128
10. Beerli et al. (2000) Proc Natl Acad Sci USA 97(4):1495-500
11. Bevan et al. (1984) Nucl Acid Res 12:8711-8720
12. Bidney et al. (1992) Plant Molec. Biol. 18:301-313
13. Bogusz et al. (1990) Plant Cell 2:633-641
14. Bradford (1976) Anal. Biochem. 72:248-254
15. Broglie et al. (1984) Science 234:838-845
16. Bustos et al. (1989) Plant Cell 1(9):839-53
17. Callis et al. (1987) Genes Dev 1:1183-1200
18. Callis et al. (1990) J Biol Chem 265:12486-12493
19. Caruthers et al. (1980) Nuc. Acids Res. Symp. Ser. 215-223
20. Chen & Winans (1991) J. Bacteriol. 173:1139-1144
21. Christou et al. (1988) Plant Physiol 87:671-674
22. Christou et al. (1991) Bio/Technology 9:957-962
23. Chui et al. (1996) Curr Biol 6:325-330
24. Clancy & Hannah (2002) Plant Physiol 130:918-929
25. Colbere-Garapin et al. (1981) J. Mol. Biol. 150:1-14
26. Coombs (1994) Dictionary of Biotechnology, Stockton Press, New York N.Y.
27. Cramer et al. (1999) Current Topics in Microbiology and Immunology 240:95-118
28. Dale & Ow (1991) Proc Natl Acad Sci USA 88:10558-10562
29. Datta et al. (1990b) Bio/Technology 8:736-740
30.. de Block et al. (1987) EMBO J 6:2513-2518
31. de Block et al. (1989) Plant Physiol. 91:694-701
32. de la Pena et al. (1987) Nature 325:274-276
33. Deak et al. (1999) Nature Biotechnology 17:192-196
34. Deblaere et al. (1985) Nucl Acids Res 13:4777-4788
35. Della-Cioppa et al. (1987) Bio/Technology 5:579-584
36. Dellaporta et al. (1988) In: Chromosome Structure and Function: Impact of New Concepts, 18th Stadler Genetics Symposium, 11:263-282
37. Dieffenbach & Dveksler (1995) PCR Primer, a Laboratory Manual, Cold Spring Harbor Press, Plainview N.Y.
38. Du et al. (1989) Genet Manip Plants 5:8-12
39. Dunwell (1998) Biotechnology and Genetic Engeneering Reviews 15:1-32
40. Dunwell (2000) J Exp Bot 51 Spec No:487-96
41. Ebinuma et al. (2000a) Proc Natl Acad Sci USA 94:2117-2121
42. Ebinuma et al. (2000b) Selection of Marker-free transgenic plants using the oncog enes (ipt, rol A, B, C) of Agrobacterium as selectable markers, In Molecular Biology of Woody Plants. Kluwer Academic Publishers
43. Eichholtz et al. (1987) Somatic Cell and Molecular Genetics 13:67-76
44. EP-A1 0 397 687
45. EP-A1 0 424 047
46. EP-A1 0 601 092
47. EP-A1 0 120 516
48. EP-A1 0 333 033
49. EP-A1 0 807 836
50. EP-A1 0 409 625
51. Fedoroff & Smith (1993) Plant J 3:273- 289
52. Feltkamp et al. (1994) Plant Physiol. 105(1):259-269
53. Fire et al (1998) Nature 391:806-811
54. Fraley et al. (1982) Proc. Natl. Acad. Sci. USA 79:1859-1863
55. Fraley et al. (1983) Proc Natl Acad Sci USA 80:4803
56. Fraley et al. (1985) CRC Crit. Rev. Plant. Sci. 4:1-45
57. Freeling & Walbot (1994) The Maize Handbook, Chapter 116 Eds., Springer, New York
58. Fromm et al. (1986) Nature 319:791-793
59. Fromm et al. (1990) Bio/Technology 8:833-839
60. Gallie et al. (1987) Nucl Acids Res 15:8693-8711
61. Gavilondo & Larrick (2000) Biotechniques 29(1):128-138
62. Gelvin et al. (Eds) (1990) Plant Molecular Biology Manual; Kluwer Academic Publisher, Dordrecht, The Netherlands
63. Gelvin & Schilperoort (1995) Plant Molecular Biology Manual, 2nd Edition, Dordrecht: Kluwer Academic Publ. ISBN 0-7923-2731-4
64. GenBank Acc. No.: AB025109
65. GenBank Acc. No.: AF306348
66. GenBank Acc. No.: S78423
67. GenBank Acc. No.: U32624
68. GenBank Acc. No.: U44838
69. GenBank Acc. No.: U77378
70. GenBank Acc. No.: X67427
71. Gleave et al. (1999) Plant Mol Biol. 40(2):223-35
72. Glick & Thompson (1993) Methods in Plant Molecular Biology and Biotechnology, Boca Raton: CRC Press, ISBN 0-8493-5164-2)
73. Gordon-Kamm et al. (1990) Plant Cell 2:603-618
74. Goring et al. (1991) Proc. Natl Acad. Sci. USA 88:1770-1774
75. Greener & Callahan (1994) Strategies 7:32-34
76. Griesbach (1992) Hort Science 27:620
77. Gruber et al. (1993) "Vectors for Plant Transformation," in METHODS IN PLANT MOLECULAR BIOLOGY AND BIOTECHNOLOGY; pp.89-119
78. Guerrero et al. (1990) Mol Gen Genet 224:161-168
79. Hajdukiewicz et al. (1994) Plant Mol Biol 25:989-994
80. Hansen et al. (1994) Proc. Natl. Acad. Sci. USA 91:7603-7607
81. Haseloff et al. (1997) Proc Natl Acad Sci USA 94(6):2122-2127
82. Hayakawa et al. (1992) Proc Natl Acad Sci USA 89:9865-9869
83. Hayford et al. (1988) Plant Physiol. 86:1216
84. -Higo et al. (1999) Nucleic Acids Research 27 (1):297-300
85. Hille et al. (1986) Plant Mol. Biol. 7:171
86. Hoekema (1985) In: The Binary Plant Vector System, Offsetdrukkerij Kanters B.V., Alblasserdam, Chapter V
87. Hoekema et al. (1983) Nature 303:179-181
88. Holliger & Bohlen (1999) Cancer & Metastasis Reviews 18(4):411-419
89. Holsters et al. (1978) Mol Gen Genet 163:181-187
90. Hood & Jilka (1999) Curr Opin Biotechnol. 10(4):382-6
91. Hood et al. (1986) J Bacteriol 168:1291-1301
92. Horn et al. (1980) Nuc. Acids Res. Symp. Ser. 225-232
93. Jarchow et al. (1991), Proc. Natl. Acad. Sci. USA 88:10426-10430
94. Jefferson (1987b) Plant Mol. Bio. Rep. 5:387-405
95. Jefferson et al. (1987a) EMBO J. 6:3901-3907
96. Jenes et al. (1983) Techniques for Gene Transfer, in: Transgenic Plants, Vol. 1, Engineering and Utilization, edited by Kung & Wu, Academic Press 128-143
97. Jones et al. (1987) Mol Gen Genet 210:86
98. Joseffson et al. (1987) J Biol Chem 262:12196-12201
99. Kado (1991) Crit Rev Plant Sci 10:1
100. Kasuga et al. (1999) Nature Biotechnology 17:276-286
101. Klapwijk et al. (1980) J. Bacteriol. 141:128-136
102. Klosgen & Weil (1991) Mol Gen Genet 225(2):297-304
103. Koncz & Schell (1986) Mol Gen Genet 204:383-396
104. Koprek et al. (1999) Plant J 19(6): 719-726
105. Krens et al. (1982) Nature 296:72-74
106. Kunkel et al. (1987) Methods Enzymol, 154:367-382
107. Lam & Chua (1991) J Biol Chem 266(26):17131-17135
108. Lawson et al. (1994) Mol Gen Genet 245:608-615
109. Le et al. (2003) Trends in Biochemical Sciences 28(4):215-220
110. Leffel et al. (1997) Biotechniques. 23(5):912-8
111. Li et al. (1992) Plant Mol Biol 20:1037-1048
112. Lorkovic et al. (2000) Trends in Plant Sciences 5(4):160-167
113. Ludwig et al. (1990) Science 247:449
114. Luehrsen & Walbot (1991) Mol Gen Genetics 225:81-93
115. Luo & Wu (1988) Plant Mol. Biol. Rep. 6:165-174
116. Lysnik et al. (1993) NAR 21:969-975
117. Ma & Vine (1999) Curr Top Microbiol. Immunol. 236:275-92
118. Maniatis et al. (1989) Molecular Cloning: A Laboratory Manual, 2nd Ed., Cold Spring Harbor Laboratory, Cold Spring Harbor (NY)
119. Matzke et al. (2000) Plant Mol Biol 43:401-415
120. McCormick et al. (1986) Plant Cell Reports 5:81-84
121. Miki et al. (1993) "Procedures for Introducing Foreign DNA into Plants" in METHODS IN PLANT MOLECULAR BIOLOGY AND BIOTECHNOLOGY; pp.67-88
122. Millar et al. (1992) Plant Mol Biol Rep 10:324-414
123. Mlynarova et al. (1994) Plant Cell Report 13:282-285
124. Mol et al. (1990) FEBS Lett 268(2):427-430
125. Moloney et al. (1989) Plant Cell Reports 8: 238-242
126. Mozo & Hooykaas (1991) Plant Mol. Biol. 16: 917-918
127. Naested (1999) Plant J 18:571-576
128. Nan et al. (1995) In "Biotechnology in Agriculture and Forestry," Ed. Y. P. S. Bajaj, Springer-Verlag Berlin Heidelberg, Vol 34:145-155
129. Napoli et al. (1990) Plant Cell 2:279-289
130. Nehra et al. (1994) Plant J. 5:285-297
131. Odell et al. (1985) Nature 313:810-812
132. Odell et al. (1990) Mol Gen Genet 223:369-378
133. Ow et al. (1986) Science 234:856-859
134. Paszkowski et al. (1984) EMBO J 3:2717-2722
135. Pei et al. (1998) Science 282:287-290
136. Perera et al. (1993) Plant Mol. Biol 23(4): 793-799
137. Potrykus (1991) Ann Rev Plant Physiol Plant Mol Biol 42:205-225
138. Prasher et al. (1985) Biochem Biophys Res Commun 126(3):1259-1268
139. Randez-Gil et al. (1995) Yeast 11:1233-1240
140. Rathore et al. (1993) Plant Mol Biol 21(5):871-884
141. Reichel et al. (1996) Proc Natl Acad Sci USA 93(12):5888-5893
142. Rhodes et al. (1995) Methods Mol Biol 55:121-131
143. Rhodes et al. (1988) Science 240, 204-207
144. Rouster et al. (1998) Plant J 15:435-440
145. Rouster et al. (1997) Plant J 11 (3):513-523
146. Rupp (1996) DNA repair mechanisms, in: Escherichia coli and Salmonella, pp. 2277-2294, ASM: Washington
147. Russel (1999) Current Topics in Microbiology and Immunology 240:119-138
148. Sadanandom et al. (1996) Plant J. 10(2):235-242
149. Sambrook et al. (1989) Cold Spring Harbor Laboratory Press; ISBN 0-87969-309-6
150. Sanford (1990) Physiologia Plantarium 79:206-209
151. Sanger et al. (1977) Proc Natl Acad Sci USA 74:5463-5467
152. Sauer (1998) Methods 14(4):381-92
153. Sautter et al. (1991) Bio/Technology, 9:1080-1085
154. Scheeren-Groot et al. (1994) J. Bacteriol 176: 6418-6426
155. Schenbom & Groskreutz (1999) Mol Biotechnol 13(1):29-44
156. Schlaman & Hooykaas (1997) Plant J 11:1377-1385
157. Schmulling et al. (1989) Plant Cell 1(7):665-670
158. Schöffl et al. (1989) Molecular & General Genetics 217(2-3):246-53
159. Shah et al. (1986) Science 233:478
160. Shahla et al. (1987) Plant Mole. Biol. 8:291-298
161. Sheehy et al. (1988) Proc Natl Acad Sci USA 85: 8805-8809
162. Sheen (1995) Plant J 8(5):777-784
163. Shewmaker et al. (1985) Virology 140:281-288
164. Shillito et al. (1985) Bio/Technology, 3:1099-1103
165. Shimamoto et al. (1989) Nature 338:274-276
166. Shirsat et al. (1989) Mol Gen Genet 215(2):326-331
167. Silhavy et al. (1984) Experiments with Gene Fusions, Cold Spring Harbor Laboratory, Cold Spring Harbor, NY
168. Smith et al. (1990) Mol. Gen. Genet. 224:447-481
169. Smyth et al. (1990) Plant Cell 2: 755-767
170. Somers et al. (1992) Bio/Technology 10:1589-1594
171. Stalberg et al. (1993) Plant Mol Biol 23:671-683
172. Stalberg et al. (1996) Planta 199:515-519
173. Stockhaus et al. (1989) EMBO J 8:2445-2245
174. Stougaard (1993) Plant J 3:755-761
175. Sundaresan et al. (1995) Gene Develop 9: 1797-1810 176. Suzuki et al. (1993) Plant Mol Biol 21:109-119
177. Svab et al. (1990) Plant Mol. Biol. 14:197
178. The Maize Handbook, Chapter 116, Freeling and Walbot, Eds., Springer, New York (1994). 179. Tian et al. (1997) Plant Cell Rep 16:267-271
180. Tomic et al. (1990) Nucl Acids Res 12:1656
181. Topfer et al. (1989) Plant Cell 1:133-139
182. Ullah et al. (2003) Plant Cell 15:393-409
183. Upender et al. (1995) Biotechniques 18(1):29-30
184. US 4,237,224
185. US 4,683,195
186. US 4,683,202
187. US 4,801,340
188. US 4,965,188
189. US 4,975,374
190. US 4,940,838
191. US 5,169,770
192. US 5,225,341
193. US 5,376,543
194. US 5,501,967
195. US 5,504,200
196. US 5,565,350
197. US 5,584,807
198. US 5,591,616
199. US 5,593,874
200. US 6,110,736
201. US 6,429,357
202. US 20020138870 (Methods for simultaneous control of lignin content and composition, and cellulose content in plants
203. Van Breusegem et al. (1998) Plant Mol Biol. 38(3):491-496
204. Van der Krol et al.(1990) Plant Cell 2:291-99
205. Van Laerebeke et al. (1974) Nature 252:169-170
206. Vanden Elzen et al. (1985) Plant Mol Biol. 5:299
207. Vasil et al. (1992) Bio/Technology 10:667-674
208. Vasil et al.. (1993) Bio/Technology 11:1153-1158
209. Villela (1998) Aci. Agric., Piracicaba 55:98-101
210. Wader et al. (1987) TOMATO TECHNOLOGY 189-198 (Alan R. Liss, Inc.)
211. Wan & Lemaux (1994) Plant Physiol. 104:3748
212. Waterhouse et al. (1998) Proc Natl Acad Sci USA 95:13959-64
213. Watson et al. (1975) J. Bacteriol 123:255-264
214. Watson et al. (1985) EMBO J 4(2):277-284
215. Weeks et al. (1993) Plant Physiol 102:1077-1084
216. Wigler et al. (1980) Proc Natl Acad Sci 77:3567-70
217. WO 00/00512
218. WO 00/15815
219. WO 00/26388
220. WO 00/44895
221. WO 00/44914
222. WO 00/49035
223. WO 00/63364
224. WO 00/68374
225. WO 02/00900
226. WO 91/02071
227. WO 91/13991
228. WO 92/16635
229. WO 93/18168
230. WO 94/00583
231. WO 94/21794
232. WO 94/48814
233. WO 97/12983
234. WO 97/13843
235. WO 97/41228
236. WO 97/42326
237. WO 97/48814
238. WO 97/50561
239. WO 98/11240
240. WO 98/22593
241. WO 98/26045
242. WO 98/45456
243. WO 99/04013
244. WO 99/05902
245. WO 99/06580
246. WO 99/32619
247. WO 99/53016
248. WO 99/53050
249. WO 01/07596 *ARABIDOPSIS THALIANA* CYCLIC NUCLEOTIDE-GATED ION CHANNEL/*DND* GENES: REGULATORS OF PLANT DISEASE RESISTANCE AND CELL DEATH
250. Zupan et al. (2000) Plant J 23(1):11-28

## Claims

1. A transgenic expression construct for predominant expression of a nucleic acid sequence of interest in substantially all vegetative plant tissues and/or organs but where expression is comparatively low or non existing in seeds and flowers comprising a promoter sequence selected from the group consisting of
a) the promoter of the *Pisum sativum* ptxA gene as described by SEQ ID NO: 1,
b) a nucleotide sequence which has essentially the same promoter activity as the promoter sequence described by SEQ ID NO: 1, and has a identity of at least 80% over a sequence of at least 700 consecutive base pairs to the sequence as described by SEQ ID NO: 1.

2. The transgenic expression construct of Claim 1, wherein the nucleotide sequence comprises a sequence from base pair 300 to base pair 583 of the sequence described by SEQ ID NO: 1 and has essentially the same promoter activity as the promoter sequence described by SEQ ID NO: 1.

3. The transgenic expression construct of any of Claim 1 to 2, wherein the expression rate realized by the transgenic expression construct measured by an quantitative β-glucuronidase assay and normalized to units of β-glucuronidase per gram of biomass in seed and flower tissue is less than 10% of the corresponding value in total vegetative plant tissue.

4. The transgenic expression construct of Claim 1 to 3, wherein
a) the nucleic acid sequence of interest to be expressed is linked operably to further genetic control sequences, or
b) the expression construct comprises additional functional elements, or
c) both a) and b) apply.

5. The transgenic expression construct of Claim 4, wherein the further genetic control sequence is the maize Ubiquitin intron.

6. The transgenic expression construct of Claim 1 to 5, wherein the nucleic acid sequence to be expressed transgenically results in,
a) expression of a protein encoded by said nucleic acid sequence, and/or
b) expression of sense, antisense, or double-stranded RNA encoded by said nucleic acid sequence.

7. The transgenic expression construct of Claim 1 to 6, wherein expression occurs in leafs, stems and roots but is not detectable in seeds.

8. A transgenic expression vector comprising a transgenic expression construct of any of Claim 1 to 7.

9. A transgenic organism transformed with an expression construct as claimed in any of claims 1 to 7 or a vector as claimed in Claim 8, wherein said organism is selected from the group consisting of bacteria of the genus *Escherichia, Erwinia, Agrobacterium, Flavobacterium, Alcaligenes, Cyanobacteria* and plant organisms.

10. The transgenic organism of Claim 9 selected from the group consisting of sugarcane, maize, sorghum, pineapple, rice, barley, oat, wheat, rye, yam, onion, banana, coconut, date, hop, rapeseed, tobacco, tomato, tagetes (marigold), soybean, pea, common bean, and papaya.

11. A cell culture, part or transgenic propagation material derived from a transgenic organism of Claim 9 or 10 and containing an expression construct as claimed in any of Claims 1 to 7 or a vector as claimed in Claim 8.

12. A method for transgenic predominant expression of a nucleic acid sequence of interest in substantially all vegetative plant tissues and/or organs, but where the expression is comparatively low or non existing in seeds and flowers comprising:
i. introduction of a transgenic expression construct into a plant cell or a plant, said transgenic expression construct comprising a promoter sequence selected from the group consisting of
a) a nucleotide sequences as claimed in any of Claims 1 or 2 and having essentially the same promoter activity as the promoter sequence described by SEQ ID NO: 1,
wherein said promoter sequence or a nucleotide sequences as claimed in any of Claims 1 or 2 and having essentially the same promoter activity as the promoter sequence described by SEQ ID NO: 1, is operably linked to a nucleic acid sequence of interest to be transgenically expressed, and wherein said promoter sequence or a nucleotide sequences as claimed in any of Claims 1 or 2 and having essentially the same promoter activity as the promoter sequence described by SEQ ID NO: 1, is heterologous with respect to said nucleic acid sequence of interest, under conditions such that said nucleic acid sequence of interest is expressed in said plant cell and/or predominantly expressed in the vegetative plant tissue and/or organs of said transgenic plant.

13. The method of Claim 12, wherein expression occurs in leafs, stems and roots but is not detectable in seeds.

14. The method of Claim 12 or 13, said method further comprising one or more of the following steps
ii) identifying or selecting the transgenic plant cell comprising said transgenic expression construct,
iii) regenerating transgenic plant tissue from the transgenic plant cell,
iv) regenerating a transgenic plant from the transgenic plant cell.

15. The method of any of Claim 12 to 14, wherein the transgenic expression construct is characterized as in Claim 1 to 7.

16. The use of a nucleotide sequence selected from the group of
a) a nucleotide sequences as claimed in any of Claims 1 or 2 and having essentially the same promoter activity as the promoter sequence described by SEQ ID NO: 1,
for transgenic expression of a nucleic acid sequence of interest in substantially all vegetative plant tissues, wherein said promoter sequence or a nucleotide sequences as claimed in any of Claims 1 or 2 and having essentially the same promoter activity as the promoter sequence described by SEQ ID NO: 1, is operably linked to a nucleic acid sequence of interest to be transgenically expressed, and wherein said promoter sequence or a nucleotide sequences as claimed in any of Claims 1 or 2 and having essentially the same promoter activity as the promoter sequence described by SEQ ID NO: 1, is heterologous with respect to said nucleic acid sequence of interest.

17. The use of a transgenic organism as claimed in claim 12 to 13 or of cell cultures, parts of transgenic propagation material derived therefrom as claimed in claim 14 for the production of foodstuffs, animal feeds, seeds, pharmaceuticals or fine chemicals.

18. A method for production of a foodstuff, animal feed, seed, pharmaceutical or fine chemical employing a transgenic organism as claimed in claim 12 to 13 or of cell cultures, parts of transgenic propagation material derived therefrom as claimed in claim 14.

## Patentansprüche

1. Transgenes Expressionskonstrukt zur vorherrschenden Expression einer interessierenden Nukleinsäuresequenz in weitgehend allen vegetativen Pflanzengeweben und/oder -organen, wobei jedoch die Expression in Samen und Blüten vergleichsweise gering ist oder nicht vorliegt, umfassend eine aus der aus
a) dem Promotor des wie durch SEQ ID NO: 1 beschriebenen ptxA-Gens aus Pisum sativum,
b) einer Nukleotidsequenz, die im Wesentlichen die gleiche Promotoraktivität wie die durch SEQ ID NO: 1 beschriebene Promotorsequenz und eine Identität von wenigstens 80% über eine Sequenz von wenigstens 700 aufeinander folgenden Basenpaaren zu der wie durch SEQ ID NO: 1 beschriebenen Sequenz aufweist,
bestehenden Gruppe ausgewählte Promotorsequenz.

2. Transgenes Expressionskonstrukt nach Anspruch 1, wobei die Nukleotidsequenz eine Sequenz von Basenpaar 300 bis Basenpaar 583 der durch SEQ ID NO: 1 beschriebenen Sequenz umfasst und im Wesentlichen die gleiche Promotoraktivität wie die durch SEQ ID NO: 1 beschriebene Promotorsequenz aufweist.

3. Transgenes Expressionskonstrukt nach einem der Ansprüche 1 bis 2, wobei die durch das transgene Expressionskonstrukt realisierte Expressionsrate, gemessen mit einem quantitativen β-Glucuronidase-Test und normiert auf Einheiten β-Glucuronidase pro Gramm Biomasse, in Samen- und Blütengewebe weniger als 10% des entsprechenden Werts im gesamten vegetativen Pflanzengewebe beträgt.

4. Transgenes Expressionskonstrukt nach Anspruch 1 bis 3, wobei
a) die zu exprimierende interessierende Nukleinsäuresequenz in operativer Verknüpfung mit weiteren genetischen Kontrollsequenzen steht oder
b) das Expressionskonstrukt zusätzliche funktionelle Elemente umfasst oder
c) sowohl a) als auch b) zutreffen.

5. Transgenes Expressionskonstrukt nach Anspruch 4, wobei es sich bei der weiteren genetischen Kontrollsequenz um das Mais-Ubiquitin-Intron handelt.

6. Transgenes Expressionskonstrukt nach Anspruch 1 bis 5, wobei die transgen zu exprimierende Nukleinsäuresequenz zur
a) Expression eines durch die Nukleinsäuresequenz codierten Proteins und/oder
b) Expression von durch die Nukleinsäuresequenz codierter Sense-, Antisense- oder Doppelstrang-RNA führt.

7. Transgenes Expressionskonstrukt nach Anspruch 1 bis 6, wobei die Expression in Blättern, Stängeln und Wurzeln auftritt, in Samen jedoch nicht nachweisbar ist.

8. Transgener Expressionsvektor, umfassend ein transgenes Expressionskonstrukt nach einem der Ansprüche 1 bis 7.

9. Transgener Organismus, transformiert mit einem Expressionskonstrukt gemäß einem der Ansprüche 1 bis 7 oder einem Vektor gemäß Anspruch 8, wobei der Organismus aus der aus Bakterien der Gattung Escherichia, Erwinia, Agrobacterium, Flavobacterium, Alcaligenes, Cyanobacteria und Pflanzenorganismen bestehenden Gruppe ausgewählt ist.

10. Transgener Organismus nach Anspruch 9, ausgewählt aus der aus Zuckerrohr, Mais, Sorghum, Ananas, Reis, Gerste, Hafer, Weizen, Roggen, Süßkartoffel, Zwiebel, Banane, Kokosnuss, Dattel, Hopfen, Raps, Tabak, Tomate, Tagetes, Sojabohne, Erbse, gemeiner Bohne und Papaya bestehenden Gruppe.

11. Zellkultur, Teil oder transgenes Propagierungsmaterial, gewonnen aus einem transgenen Organismus nach Anspruch 9 oder 10 und enthaltend ein Expressionskonstrukt gemäß einem der Ansprüche 1 bis 7 oder einen Vektor gemäß Anspruch 8.

12. Verfahren zur transgenen vorherrschenden Expression einer interessierenden Nukleinsäuresequenz in weitgehend allen vegetativen Pflanzengeweben und/oder -organen, wobei jedoch die Expression in Samen und Blüten vergleichsweise gering ist oder nicht vorliegt, umfassend:
i. Einführung eines transgenen Expressionskonstrukts in eine Pflanzenzelle oder eine Pflanze, wobei das transgene Expressionskonstrukt eine aus der aus
a) einer Nukleotidsequenz gemäß einem der Ansprüche 1 oder 2, die im Wesentlichen die gleiche Promotoraktivität wie die durch SEQ ID NO: 1 beschriebene Promotorsequenz aufweist,
bestehenden Gruppe ausgewählte Promotorsequenz umfasst, wobei die Promotorsequenz oder eine Nukleotidsequenz gemäß einem der Ansprüche 1 oder 2, die im Wesentlichen die gleiche Promotoraktivität wie die durch SEQ ID NO: 1 beschriebene Promotorsequenz aufweist, in operativer Verknüpfung mit einer transgen zu exprimierenden interessierenden Nukleinsäuresequenz steht und wobei die Promotorsequenz oder eine Nukleotidsequenz gemäß einem der Ansprüche 1 oder 2, die im Wesentlichen die gleiche Promotoraktivität wie die durch SEQ ID NO: 1 beschriebene Promotorsequenz aufweist, in Bezug auf die interessierende Nukleinsäuresequenz heterolog ist, unter solchen Bedingungen, dass die interessierende Nukleinsäuresequenz in der Pflanzenzelle exprimiert und/oder in dem bzw. den vegetativen Pflanzengewebe und/oder organen der transgenen Pflanze vorherrschend exprimiert wird.

13. Verfahren nach Anspruch 12, wobei die Expression in Blättern, Stängeln und Wurzeln auftritt, in Samen jedoch nicht nachweisbar ist.

14. Verfahren nach Anspruch 12 oder 13, bei dem man ferner einen oder mehrere der folgenden Schritte durchführt:
ii. Identifizieren oder Auswählen der das transgene Expressionskonstrukt umfassenden transgenen Pflanzenzelle,
iii. Regenerieren von transgenem Pflanzengewebe aus der transgenen Pflanzenzelle,
iv. Regenerieren einer transgenen Pflanze aus der transgenen Pflanzenzelle.

15. Verfahren nach einem der Ansprüche 12 bis 14, wobei das transgene Expressionskonstrukt wie in Anspruch 1 bis 7 gekennzeichnet ist.

16. Verwendung einer aus der Gruppe von
a) einer Nukleotidsequenz gemäß einem der Ansprüche 1 oder 2, die im Wesentlichen die gleiche Promotoraktivität wie die durch SEQ ID NO: 1 beschriebene Promotorsequenz aufweist,
ausgewählten Nukleotidsequenz zur transgenen Expression einer interessierenden Nukleinsäuresequenz in weitgehend allen vegetativen Pflanzengeweben, wobei die Promotorsequenz oder eine Nukleotidsequenz gemäß einem der Ansprüche 1 oder 2, die im Wesentlichen die gleiche Promotoraktivität wie die durch SEQ ID NO: 1 beschriebene Promotorsequenz aufweist, in operativer Verknüpfung mit einer transgen zu exprimierenden interessierenden Nukleinsäuresequenz steht und wobei die Promotorsequenz oder eine Nukleotidsequenz gemäß einem der Ansprüche 1 oder 2, die im Wesentlichen die gleiche Promotoraktivität wie die durch SEQ ID NO: 1 beschriebene Promotorsequenz aufweist, in Bezug auf die interessierende Nukleinsäuresequenz heterolog ist.

17. Verwendung eines transgenen Organismus nach Anspruch 12 bis 13 oder von daraus gewonnenen Zellkulturen, Teilen von transgenem Propagierungsmaterial gemäß Anspruch 14 zur Herstellung von Lebensmitteln, Tierfutter, Saatgut, Pharmazeutika oder Feinchemikalien.

18. Verfahren zur Herstellung eines Lebensmittels, Tierfutters, Saatguts, Pharmazeutikums oder einer Feinchemikalie unter Einsatz eines transgenen Organismus gemäß Anspruch 12 bis 13 oder daraus gewonnener Zellkulturen, Teile von transgenem Propagierungsmaterial gemäß Anspruch 14.

## Revendications

1. Construction d'expression transgénique pour l'expression prédominante d'une séquence d'acide nucléique d'intérêt dans pour ainsi dire la totalité des tissus et/ou organes végétaux végétatifs, mais où l'expression est comparativement faible, ou non existante, dans les graines et les fleurs, comprenant une séquence promotrice choisie dans le groupe consistant en
a) le promoteur du gène ptxA de *Pisum sativum* tel que décrit par SEQ ID NO: 1,
b) une séquence nucléotidique qui a sensiblement la même activité promotrice que la séquence promotrice décrite par SEQ ID NO: 1, et qui a une identité d'au moins 80 %, sur une séquence d'au moins 700 paires de bases consécutives, avec la séquence telle que décrite par SEQ ID NO: 1.

2. Construction d'expression transgénique de la revendication 1, dans laquelle la séquence nucléotidique comprend une séquence allant de la paire de bases 300 à la paire de bases 583 de la séquence décrite par SEQ ID NO: 1, et a sensiblement la même activité promotrice que la séquence promotrice décrite par SEQ ID NO: 1.

3. Construction d'expression transgénique de l'une quelconque des revendications 1 à 2, dans laquelle le taux d'expression réalisé par la construction d'expression transgénique, mesuré par un essai quantitatif de la β-glucuronidase, et normalisé à des unités de β-glucuronidase par gramme de biomasse dans le tissu de la graine et le tissu floral, est inférieur à 10 % de la valeur correspondante dans le tissu végétal végétatif total.

4. Construction d'expression transgénique des revendications 1 à 3, dans laquelle
a) la séquence d'acide nucléique d'intérêt à exprimer est liée d'une manière opérationnelle à d'autres séquences régulatrices génétiques, ou
b) la construction d'expression comprend des éléments fonctionnels additionnels, ou
c) a) et b) s'appliquent tous les deux.

5. Construction d'expression transgénique de la revendication 4, dans laquelle l'autre séquence régulatrice génétique est l'intron de l'ubiquitine du maïs.

6. Construction d'expression transgénique des revendications 1 à 5, dans laquelle la séquence d'acide nucléique devant subir une expression transgénique conduit
a) à l'expression d'une protéine codée par ladite séquence d'acide nucléique, et/ou
b) à l'expression d'un ARN sens, antisens ou double brin, codé par ladite séquence d'acide nucléique.

7. Construction d'expression transgénique des revendications 1 à 6, dans laquelle l'expression a lieu dans les feuilles, les tiges et les racines, mais n'est pas détectable dans les graines.

8. Vecteur d'expression transgénique comprenant une construction d'expression transgénique de l'une quelconque des revendications 1 à 7.

9. Organisme transgénique, transformé avec une construction d'expression telle que revendiquée dans l'une quelconque des revendications 1 à 7, ou vecteur tel que revendiqué dans la revendication 8, ledit organisme étant choisi dans le groupe consistant en les bactéries des genres *Escherichia, Erwinia, Agrobacterium, Flavobacterium, Alcaligenes, Cyanobacteria,* et les organismes végétaux.

10. Organisme transgénique de la revendication 9, choisi dans le groupe consistant en la canne à sucre, le maïs, le sorgho, l'ananas, le riz, l'orge, l'avoine, le blé, le seigle, l'igname, l'oignon, la banane, le cocotier, le dattier, le houblon, le colza, le tabac, la tomate, le tagète (souci), le soja, le pois, le haricot commun et la papaye.

11. Culture, partie ou matériel de propagation transgénique cellulaire, qui dérive d'un organisme transgénique de la revendication 9 ou 10, et contenant une construction d'expression telle que revendiquée dans l'une quelconque des revendications 1 à 7 ou un vecteur tel que revendiqué dans la revendication 8.

12. Procédé d'expression prédominante transgénique d'une séquence d'acide nucléique d'intérêt dans pour ainsi dire la totalité des tissus et/ou organes végétaux végétatifs, mais l'expression étant comparativement faible ou non existante dans les graines ou les fleurs, comprenant :
i. l'introduction d'une construction d'expression transgénique dans une cellule végétale ou dans une plante, ladite construction d'expression transgénique comprenant une séquence promotrice choisie dans le groupe consistant en
a) des séquences nucléotidiques telles que revendiquées dans l'une quelconque des revendications 1 ou 2 et ayant sensiblement la même activité promotrice que la séquence promotrice décrite par SEQ ID NO: 1, ladite séquence promotrice, ou une séquence nucléotidique telle que revendiquée dans l'une quelconque des revendications 1 ou 2, et ayant sensiblement la même activité promotrice que la séquence promotrice décrite par SEQ ID NO: 1, étant liée d'une manière opérationnelle à une séquence d'acide nucléique d'intérêt devant subir une expression transgénique, et ladite séquence promotrice, ou une séquence nucléotidique selon l'une quelconque des revendications 1 ou 2, et ayant sensiblement la même activité promotrice que la séquence promotrice décrite par SEQ ID NO: 1, étant hétérologue par rapport à ladite séquence d'acide nucléique d'intérêt, dans des conditions telles que ladite séquence d'acide nucléique d'intérêt soit exprimée dans ladite cellule végétale et/ou exprimée d'une manière prédominante dans le tissu et/ou les organes végétaux végétatifs de ladite plante transgénique.

13. Procédé de la revendication 12, dans lequel l'expression a lieu dans les feuilles, les tiges et les racines, mais n'est pas détectable dans les graines.

14. Procédé de la revendication 12 ou 13, ledit procédé comprenant en outre une ou plusieurs des étapes suivantes :
ii) identification ou sélection de la cellule végétale transgénique comprenant ladite construction d'expression transgénique,
iii) régénération du tissu végétal transgénique à partir de la cellule végétale transgénique,
iv) régénération d'une plante transgénique à partir de la cellule végétale transgénique.

15. Procédé de l'une quelconque des revendications 12 à 14, dans lequel la construction d'expression transgénique est caractérisée comme dans les revendications 1 à 7.

16. Utilisation d'une séquence nucléotidique choisie dans le groupe comprenant :
a) une séquence nucléotidique telle que revendiquée dans l'une quelconque des revendications 1 ou 2 et ayant sensiblement la même activité promotrice que la séquence promotrice décrite par SEQ ID NO: 1,
pour l'expression transgénique d'une séquence d'acide nucléique d'intérêt dans pour ainsi dire la totalité des tissus végétaux végétatifs, ladite séquence promotrice ou une séquence nucléotidique telle que revendiquée dans l'une quelconque des revendications 1 ou 2 et ayant sensiblement la même activité promotrice que la séquence promotrice décrite par SEQ ID NO: 1, étant liée d'une manière opérationnelle à une séquence d'acide nucléique d'intérêt devant subir une expression transgénique, et ladite séquence promotrice ou une séquence nucléotidique telle que revendiquée dans l'une quelconque des revendications 1 ou 2 et ayant sensiblement la même activité promotrice que la séquence promotrice décrite par SEQ ID NO: 1 étant hétérologue par rapport à ladite séquence d'acide nucléique d'intérêt.

17. Utilisation d'un organisme transgénique tel que revendiqué dans les revendications 12 à 13 ou de cultures, de parties ou d'un matériel de propagation transgénique cellulaires qui en dérivent, tel que revendiqué dans la revendication 14, pour la production de produits pour l'alimentation humaine ou animale, de graines, de produits pharmaceutiques ou de produits de la chimie fine.

18. Procédé de production d'un produit pour l'alimentation humaine ou animale, d'une graine, d'un produit pharmaceutique ou d'un produit de la chimie fine, utilisant un organisme transgénique tel que revendiqué dans les revendications 12 à 13, ou de cultures, parties ou matériel de propagation transgénique cellulaires qui en dérivent, tels que revendiqués dans la revendication 14.
